# EUROPEAN PATENT SPECIFICATION

(11) **EP 2 855 516 B1**
(45) Date of publication and mention of the grant of the patent: **17.07.2019**
(21) Application number: 13796588.5
(22) Date of filing: 27.05.2013
(51) Int. Cl.: C07K 14/52, A01K 67/027, A61K 31/7088, A61K 39/395, A61P 29/00, C07K 14/47, C12N 15/19, C12N 15/85, C12N 5/10, C12Q 1/00, C12Q 1/68, C40B 30/06, G01N 33/15, G01N 33/68

(54) **INFLAMMATION-ENABLING POLYPEPTIDES AND USES THEREOF**
ENTZÜNDUNGSHERBEIFÜHRENDES POLYPEPTID UND ANWENDUNGEN DAVON
POLYPEPTIDES ACTIVANT UNE INFLAMMATION ET LEURS UTILISATIONS

(30) Priority: 28.05.2012 US 201261652271 P
(43) Date of publication of application: 08.04.2015
(73) Proprietor: The Royal Institution for the Advancement of Learning / McGill University, Montréal, Québec H3A 0G4 (CA)
(72) Inventor: GROS, Philippe, Saint-Lambert, Québec J4R 1X4 (CA)
(74) Representative: Daub, Thomas
(86) International application number: PCT/CA2013/050403
(87) International publication number: WO 2013/177699

(56) References cited:
- WO-A2-2009/049228
- WO-A2-2011/011678
- WO-A2-2012/174282
- CA-A1- 2 812 940
- US-A1- 2012 121 697
- MATSUSHITA E. ET AL.: 'Protective role of Gipie, a Girdin family protein, in endoplasmic reticulum stress responses in endothelial cells.' MOLECULAR BIOLOGY OF THE CELL. vol. 22, 15 March 2011, pages 736 - 747, XP055179399
- BONGFEN SILAYUV E ET AL: "An N-Ethyl-N-Nitrosourea (ENU)-Induced Dominant Negative Mutation in the JAK3 Kinase Protects against Cerebral Malaria", February 2012 (2012-02), PLOS ONE, VOL. 7, NR. 2, PAGE(S) ARTICLE NO.: E31012 ISSN: 1932-6203(print)

## Description

### TECHNOLOGICAL FIELD

The present invention relates to the use of polypeptides (or their coding sequences) to screen for agents useful for the prevention, treatment and/or alleviations of symptoms associated with an inflammatory disorder, to identify individuals susceptible of developing an exacerbated inflammatory response as well as to determine if a therapeutic regimen is capable of preventing, treating or alleviating the symptoms associated to an inflammatory disorder in an individual. The present application also provides methods for preventing, treating and/or alleviating the symptoms associated to an inflammatory condition based on the inhibition of expression or activity of the inflammation-enabling targets.

### BACKGROUND

Embodiments and explanations with regard to an in vivo method merely serve for clarification and are not part of the invention. Inflammation is a normal physiological response to tissue injury caused by infections, burns, trauma and other insults. Tight regulation of this response is important for initial recognition of the associated danger signals, elimination of the causative lesion and restoration of homeostasis. This process involves a complex interplay between hematopoietic and stromal cells including the crosstalk between fibroblasts, endothelial and epithelial cells with cells of the innate and adaptive immune systems. The early production of pro-inflammatory soluble mediators and remodeling enzymes, and the timely synthesis of anti-inflammatory molecules that dampen and terminate the process are characteristic features of such a regulated inflammatory response. However, dysregulation of this process results in acute or chronic inflammatory conditions. Although many classes and types of anti-inflammatory drugs exist, their efficacy is limited and often transient, and their long-term use causes significant adverse side effects. Hence, inflammatory diseases represent an unmet pharmacological need in a large market.

The inflammatory response involves a complex cascade of events including the initial activation of pattern recognition receptors (PRRs) and inflammasomes by danger signals in epithelial, endothelial and tissue resident immune cells. This is rapidly followed by recruitment of immune cells such as neutrophils, basophils, monocytes, macrophages, CD4+ and CD8+ T lymphocytes from distant sites to the site of injury. These infiltrates release a number of soluble mediators (histamine, leukotrienes, nitric oxide), cytokines (TNFα, IFNγ, IL-1), chemokines (IL-8, MCP1, KC) and enzymes (lysosomal proteases), which along with certain plasma proteins (complement, bradykinin, plasmin) establish and amplify the inflammatory response. Timely production of anti-inflammatory molecules (PGE2, IL-10, TGFβ IL-1Ra) dampens and ultimately terminates this response. In the presence of persistent tissue injury or of an unusual infectious/environmental insult, overexpression of pro-inflammatory mediators or insufficient production of anti-inflammatory signals results in acute or chronic debilitating conditions. Acute inflammatory conditions, including sepsis and encephalitis, are difficult to manage clinically and have high mortality rates. Chronic inflammatory diseases show a high incidence in North America, and include rheumatoid arthritis (RA, incidence 75-1000/10⁵), inflammatory bowel disease (IBD, ∼0.5-25/10⁵), systemic lupus erythematosus (SLE, 40-200/10⁵), psoriasis (PA, 2/100), multiple sclerosis (MS, 18-350/10⁵), type 1 diabetes (T1D, 8-17/10⁵), and celiac disease (CeD, 1/100). It has also been proposed that chronic obstructive pulmonary disease, coronary atherosclerosis, diabetes, cancer and neurodegenerative disorders may have a contributing inflammatory component. These global "inflammatory" diseases are a significant burden on human health, and represent a large pharmaceutical market for anti-inflammatory drugs. Several classes of such drugs have been developed, including steroids (cortisone), nonsteroidal anti-inflammatory drugs including COX2 inhibitors (colecoxib), and derivatives of proprionic acid (ibuprofen), acetic acid (indomethacin), enolic and fenamic acid. However, the benefit of these drugs is limited, and they show significant person-to-person variability in efficacy and their long-term use has severe side effects, raising the need for specific targeted therapies. Recently, biologicals targeting the TNF (infliximab, etanercept, adalimumab), IL-12p40 (monoclonal antibodies) and IL-1 (anakinra) pathways, as well as B cells (rituximab) and T cells (abatacept) have shown clinical efficacy in inflammatory conditions, supporting the relevance of our host-based approach to identify novel anti-inflammatory targets.

Chronic inflammatory conditions share several clinical features, including persistent activation of the innate and acquired immune systems. Rupture of normal epithelial barriers, tissue damage, or persistent infections may lead to chronic exposure to inert environmental or host-derived products (food, monosodium urate crystals, asbestos, silica, etc.), microbial products (including commensal bacteria, viruses, parasites and fungi), and/or exposure to enticing self-antigens (nucleic acids, damaged proteins). These are recognized by intracellular or cell surface sensors of the innate immune system or by receptors of the acquired immune system. Engagement of these receptors and activation of associated signaling pathways in myeloid cells leads to the production of pro-inflammatory cytokines (IL-1, IL-18, IL-12, IL-23) and mediators (leukotrienes), and to the release of toxic species (reactive oxygen radicals) and proteases (lysosomal enzymes) *in situ* that exacerbate the local inflammatory environment by recruiting and activating other myeloid and lymphoid cells from systemic sites, including the engagement of CD8+ and CD4+ T lymphocytes (Th1, Th2 and Th17 cells). Persistence of pro-inflammatory T helper programs in these cells (Th1, Th2, Th17) and/or defects in suppressive T regulatory (Treg) responses lead to unrelenting tissue damage and is a major common feature of these inflammatory diseases.

Familial aggregation and twin studies have long established that chronic inflammatory conditions have a strong genetic component. Genome-wide association studies (GWAS) have shown that the genetic component of these diseases is complex with >250 loci detected, and notably several of these risk loci are shared in rheumatoid arthritis (RA), psoriasis (PA), systemic lupus erythematosus (SLE), multiple sclerosis (MS), type 1 diabetes (T1D), celiac disease (CeD), and inflammatory bowel disease (IBD), with additive small genetic effects combining to cause disease by a threshold mechanism. Genetic risks common to these conditions are found in immune pathways including: a) pattern recognition receptors of the innate immune system (NLRs, TLRs) and associated signaling cascades (inflammasomes); b) antigen processing and presentation, production of cytocidal species (ROS, iNOS), and secretion of pro-inflammatory mediators by myeloid cells (IL-18, IL-12); c) T and B lymphocyte maturation (e.g. by IL-2), including control of auto-reactive T and B cells; d) antigen receptors of T and B cells for recognition in the context of Class I or class II MHC (HLA); e) production of pro-inflammatory cytokines (IL-12, IL-18, IL-23), and associated regulation of Th1, Th17, Treg polarization of the immune response; f) activation of ubiquitous cellular responses such as autophagy, ER stress, and others. The proteins and pathways defined by these shared genetic variants may be excellent candidate targets for drug development and therapeutic intervention. However, the complexity of the genetic control renders the identification and prioritization of key non-redundant targets in these pathways difficult.

It would be highly desirable to be provided with additional polypeptide targets which enable the mounting and/or persistence of an inflammatory response. These targets should preferably be host proteins which are involved in inflammation, regardless of the etiology of the disease.

### BRIEF SUMMARY

The invention is set out in the appended set of claims.

One aim of the present invention is to provide host-derived inflammation-enabling polypeptides responsible for mounting and maintaining a pathological inflammatory response, independent of the etiology of the inflammatory disease.

In accordance with the present invention, there is provided a method for assessing the ability of an agent to prevent, treat and/or alleviate the symptoms associated with an inflammatory condition in an individual. Broadly, the method comprises (a) combining the agent with a reagent to obtain a parameter associated with the biological activity of at least one inflammation-enabling polypeptide (IEP); (b) measuring the parameter of the reagent of step (a) to obtain a test level; (c) comparing the test level to a control level, wherein the control level is associated with the biological activity of the at least one inflammation-enabling polypeptide observed during the onset or maintenance of the inflammatory condition; and (d) characterizing the agent based on the comparison. Since the biological activity IEP is increased during onset or maintenance of inflammation, the agent is considered useful for the prevention, treatment and/or alleviation of the symptoms associated with the inflammatory condition when it is capable to reduce the biological activity of the IEP. Alternatively, the agent is considered not to be useful for (e.g. lacking utility) for the prevention, treatment and/or alleviation of the symptoms associated with the inflammatory condition if it cannot reduce the biological activity of the IEP (e.g. if the biological activity in the presence of the agent is equal to or higher than the control biological activity). In an embodiment, the inflammation-enabling polypeptide is at least one of CCDC88B, USP15, IRF8, IRF1, IRGM1, THEMIS and/or FOXN1. In an embodiment, the reagent is a nucleic acid molecule. In another embodiment, the nucleic acid molecule comprises a promoter of a gene encoding the at least one inflammation-enabling polypeptide and the parameter is a measure of the expression driven by the promoter. Alternatively, the nucleic acid molecule can be a transcript encoding the at least one inflammation-enabling polypeptide and the parameter can be a measure of the amount of the transcript. In other embodiment, the nucleic acid molecule is a transcript of at least one gene whose expression can be modified by the inflammation-enabling polypeptide and the parameter is the amount of the transcript. For example, when the inflammation-enabling polypeptide is IRF8, the at least one gene whose expression can be modified by the inflammation-associated polypeptide can be selected from the group consisting of the genes presented in Table 1, 2 and 3. In yet another embodiment, the reagent can be a polypeptide. In still a further embodiment, the polypeptide is the at least one inflammation-enabling polypeptide. In such embodiment, the parameter is the amount of the at least one inflammation-enabling polypeptide. Alternatively, the parameter can be at least one biological activity of the at least one inflammation-enabling polypeptide. For example, when the at least one inflammation-enabling polypeptide is selected from the group consisting of FOXN1, IRF8 and IRF1, the at least one biological activity can be a transcription factor activity. As another example, when the at least one inflammation-enabling polypeptide is CCDC88B, the at least one biological activity can be protein folding. As yet another example, when the at least one inflammation-enabling polypeptide is USP15, the at least one biological activity can be a de-ubiquitinating activity. As still another example, when the at least one inflammation-enabling polypeptide is THEMIS, the at least one biological activity can be a protein signaling activity. In another embodiment, the polypeptide is a binding partner of the at least one inflammation-enabling polypeptide. For example, when the at least one inflammation-enabling polypeptide is USP15, the binding partner can be a polypeptide that can be de-ubiquinated by USP15. In another embodiment, the polypeptide is associated with the signaling pathway of the inflammation-enabling polypeptide. In an embodiment, step (i) is conducted *in vitro or in vivo.* In yet another embodiment, step (i) is conducted in a non-human animal, such as, for example, in an animal bearing at least one gene copy of a non-functional inflammation-enabling polypeptide. In still another embodiment, step (i) is conducted in a human.

In accordance with the present invention, there is provided a method for assessing the ability of an agent to treat and/or alleviate the symptoms associated with an inflammatory condition in an individual in need thereof. Broadly, the method comprises (a) administering a trigger for inducing an inflammatory response in an animal heterozygous for the gene encoding an inflammation-enabling polypeptide; (b) administering the agent to the animal experiencing the inflammatory response 9e.g. being in an inflammatory state); (c) measuring a parameter of the inflammatory response in the animal to provide a test level; (d) comparing the test level to a control level, wherein the control level is associated with the biological activity of the inflammation-enabling polypeptide observed during the onset or maintenance of the inflammatory condition; and (e) characterizing the agent based on the comparison. Since the biological activity IEP is increased during onset or maintenance of inflammation, the agent is considered useful for the treatment and/or alleviation of the symptoms associated with the inflammatory condition when it is shown to reduce the biological activity of the IEP in the treated animal. Alternatively, the agent is considered as lacking utility for the treatment and/or alleviation of the symptoms associated with the inflammatory condition when the biological activity of the IEP, in the presence of the agent, is equal to or higher than the control level in the treated animal. In an embodiment, the inflammation-enabling polypeptide is at least one of FOXN1, CCDC88B, IRF1, IRF8, THEMIS, IRGM1 or USP15. In still another embodiment, the trigger is an infectious agent. In yet another embodiment, the parameter is a survival rate. In yet another embodiment, the parameter is a neurological symptom selected from the group consisting to fever, tremors, lethargy, hind limb paralysis and coma. In still yet another embodiment, the parameter is an inflammation-associated parameters selected from the group consisting of immune cells number, immune cell type, cytokine profile, chemokine profile, immunoglobulin profile, edema and blood-brain-barrier permeability.

In accordance with the present invention, there is provided a method for assessing the ability of an agent to prevent an inflammatory condition in an individual. Broadly, the method comprises (a) administering an agent to an animal heterozygous for the gene encoding an inflammation-enabling polypeptide; followed by (b) administering a trigger capable of inducing an inflammatory response in the animal in the absence of the agent; (c) measuring a parameter of the inflammatory response in the animal to provide a test level; (d) comparing the test level to a control level, wherein the control level is associated with the biological activity of the at least one inflammation-enabling polypeptide observed during the onset or maintenance of the inflammatory condition; and (e) characterizing the utility of the agent in the individual based on the comparison. Since the biological activity IEP is increased during onset or maintenance of inflammation, the agent is considered useful for the prevention of the inflammatory condition when it is shown to reduce the biological activity of the IEP in the treated animal. Alternatively, the agent is considered as lacking utility for the prevention of the inflammatory condition in the individual when the biological activity of the IEP, in the presence of the agent, is equal to or higher than the control level in the treated animal. Embodiments described herein with respect to the inflammation-enabling polypeptide, the trigger or the parameter described herein can also be used in this method.

In accordance with the present invention, there is provided a method for determining if a therapeutic agent is useful for the prevention, treatment and/or alleviation of symptoms associated with an inflammatory condition in an individual. Broadly, the method comprises (a) providing a biological sample of the individual having received at least one dose of the therapeutic agent; (b) measuring a parameter of a reagent associated to at least one inflammation-enabling polypeptide to provide a test level; (c) comparing the test level to a control level, wherein the control level is associated with the biological activity of the at least one inflammation-enabling polypeptide observed during the onset or maintenance of the inflammatory condition; and (d) characterizing the usefulness of the therapeutic agent based on the comparison. Since the biological activity IEP is increased during onset or maintenance of inflammation, the agent is considered useful for the prevention, treatment and/or alleviation of the symptoms associated with the inflammatory condition in the individual when it is shown to reduce the biological activity of the IEP. Alternatively, the agent is considered as lacking utility for the prevention, treatment and/or alleviation of the symptoms associated with the inflammatory condition in the individual when the biological activity of the IEP, in the presence of the agent, is equal to or higher than the control level. Embodiments described herein with respect to the reagent, the parameter, the biological activity, the inflammation-enabling polypeptide, the polypeptide can also be used in this method. In yet a further embodiment, the characterization is performed in at least two biological samples (and in some embodiments, maybe more), preferably at two distinct time periods to evaluate the usefulness of the therapeutic agent in the individual in function of time.

In accordance with the present invention, there is provided a method for determining the predisposition to or presence of an inflammatory condition in an individual. Broadly, the method comprises (a) providing a biological sample from the individual; (b) measuring a parameter of a reagent associated to at least one inflammation-enabling polypeptide to provide a test level; (c) comparing the test level to a control level, wherein the control level is associated with the biological activity of the at least one inflammation-enabling polypeptide observed during the absence of the inflammatory condition; and (d) characterizing the individual based on the comparison. Embodiments described herein with respect to the reagent, the parameter, the biological activity, the inflammation-enabling polypeptide, the polypeptide can also be used in this method.

In accordance with the present invention, there is provided a method for preventing, treating and/or alleviating the symptoms associated to an inflammatory condition in an individual in need thereof. Broadly, the method comprises administering an agent capable of inhibiting at least one parameter of an inflammation-enabling polypeptide so as to prevent, treat and/or alleviation the symptoms associated to the inflammatory condition in the individual. Also provided herein, is the use of an agent capable of inhibiting at least one parameter of an inflammation-enabling polypeptide for the prevention, treatment and/or alleviation the symptoms associated to the inflammatory condition in the individual; the use of an agent capable of inhibiting at least one parameter of an inflammation-enabling polypeptide for the manufacture of a medicament for the prevention, treatment and/or alleviation the symptoms associated to the inflammatory condition in the individual; as well as an agent capable of inhibiting at least one parameter of an inflammation-enabling polypeptide for the prevention, treatment and/or alleviation the symptoms associated to the inflammatory condition in the individual. In an embodiment, the agent is a nucleic acid molecule capable of limiting the expression of the inflammation-enabling polypeptide. Embodiments with respect to the inflammation-enabling polypeptides described do apply to these therapeutic uses. In another embodiment, the agent is an antibody capable of limiting the biological activity of the inflammation-enabling polypeptide. In still another embodiment, the inflammatory condition is selected from the group consisting of neuroinflammation, rheumatoid arthritis, systemic lupus erythematosus, multiple sclerosis, type 1 diabetes and celiac disease.

In accordance with the present invention, there are provided various tools associated with inflammatory enabling polypeptides. In an embodiment, an isolated polypeptide is provided. The isolated polypeptide is a mutant of an inflammatory enabling polypeptide (IEP). The mutant IEP has, when expressed in a subject, the ability to prevent the onset and/or maintenance of an inflammatory condition in the subject. Embodiments associated with the different types of IEP described above can be applied herein. In an embodiment, the isolated polypeptide has the amino acid sequence of SEQ ID NO: 4. In yet another embodiment, a nucleic acid vector is provided and encodes the mutant IEP. In still another embodiment, a cell (or a cell line) is provided. The cell can be heterozygous for a gene encoding an inflammatory enabling polypeptide (IEP). In such embodiment, the heterozygous cell has a first gene copy encoding IEP and a second gene copy encoding a mutant of the IEP. Alternatively, the cell can be homozygous for a gene encoding for a mutant of an inflammatory enabling polypeptide (IEP). The cell can be, in an embodiment, a transgenic cell and can have, for example, the nucleic acid vector described herein. In yet another embodiment, an animal is provided. The animal can be heterozygous for a gene encoding an inflammatory enabling polypeptide (IEP). In such embodiment, the heterozygous animal has a first gene copy encoding IEP and a second gene copy encoding a mutant of the IEP. In another embodiment, the animal can be homozygous for a gene encoding for a mutant of an inflammatory enabling polypeptide (IEP). In some embodiments, the animal can be a transgenic animal, for example, those manipulated to have the nucleic acid vector described herein.

Throughout this text, various terms are used according to their plain definition in the art. However, for purposes of clarity, some specific terms are defined below.

*Biological sample.* A biological sample is a sample of an individual's bodily fluid, cells or tissues. In this present invention, the biological sample can be derived from the individual's blood. The biological sample can be used without prior modification in the various methods described herein. Optionally, the biological sample can be treated (mechanically, enzymatically, *etc*.) prior to the assays described herein.

*Heterozygote.* Zygosity refers to the similarities of alleles for a genetic trait in an individual organism. If both alleles are the same, the individual (an homozygote) is considered homozygous for the trait. If both alleles are different, the individual (an heterozygote) is heterozygous for that trait. If one allele is missing, the individual (an hemizygote) is considered hemizygous, and, if both alleles are missing, the individual (a nullizygote) is considered nullizygous. An heterozygote of an inflammation-enabling polypeptide bears a first allele coding for a functional inflammation-enabling polypeptide and a second allele coding for a non-functional inflammation-enabling polypeptide.

*Inflammation and inflammatory response.* Inflammation is a response of the body to harmful stimuli and is achieved by the increased movement of plasma and leukocytes (especially granulocytes) from the blood into the injured tissues. A cascade of biochemical events propagates and matures the inflammatory response, involving the local vascular system, the immune system, and various cells within the injured tissue. As used herein, the terms "inflammation" and "inflammatory response" refer to the non-pathological aspect of this response and may even be considered benefic to the individual experiencing it.

*Inflammatory condition, disease or disorder.* As used herein, these terms collectively refer to a dysregulated inflammatory response which causes a pathological cellular destruction of tissues in an afflicted individual. The inflammation can either be acute or chronic. Acute inflammatory conditions include, but are not limited to sepsis and encephalitis. Chronic inflammatory conditions share several clinical features, including persistent activation of the innate and acquired immune systems. The chronic inflammatory conditions can include the production of pro-inflammatory cytokines (IL-1, IL-18, IL-12, IL-23) and mediators (leukotrienes), the release of toxic species (reactive oxygen radicals) and proteases (lysosomal enzymes). In some embodiments, the chronic inflammatory condition also includes recruiting and activating other myeloid and lymphoid cells from systemic sites, such as, for example, CD8+ and CD4+ T lymphocytes (Th1, Th2 and Th17 cells). Persistence of pro-inflammatory T helper programs in these cells (Th1, Th2, Th17) and/or defects in suppressive T regulatory (Treg) responses can lead to unrelenting tissue damage. Chronic inflammatory conditions includes, but are not limited to, rheumatoid arthritis (RA), inflammatory bowel disease (IBD), systemic lupus erythematosus (SLE), psoriasis (PA), multiple sclerosis (MS), type 1 diabetes (T1D), and celiac disease (CeD). Other conditions associated with chronic inflammation include, but are not limited to chronic obstructive pulmonary disease, coronary atherosclerosis, diabetes, metabolic syndrome X, cancer and neurodegenerative disorders.

*Inflammation-enabling polypeptide (IEP).* As used herein, this term refers to polypeptides which, when their expression and/or is limited or inhibited, prevent the onset and/or maintenance of an inflammatory condition (either acute or chronic). These polypeptides are preferentially identified by the genetic screen described below and in Bongfen *et al.* (*P. berghei* challenge of ENU-mutated animals). These polypeptides are preferentially selected from the group consisting of IRF8, IRF1, IRGM1, CCDC88B, THEMIS, FOXN1 and USP15, more preferably from the group consisting of IRF8, CCDC88B, FOXN1 and USP15, even more preferably from the group consisting of CCDC88B, FOXN1 and USP15 and, in the most preferred embodiment, from the group consisting of CCDC88B and USP15. In an embodiment, the IEP do not include JAK-3. As it will be shown below, it may be necessary to provide heterozygous animals for these inflammatory-enabling polypeptides. Such heterozygous animals bear a first functional gene copy coding for a functional IEP and a second gene copy coding for a non-functional IEP. A "functional" IEP (also referred to as the wild-type IEP) refers to a polypeptide capable of being expressed and providing its biological activity for mounting and/or maintaining an inflammatory response. By contrast, a "non-functional" IEP (also referred to as a mutant IEP) refers to a polypeptide that is not being expressed from its corresponding gene copy, expressed at lower level (when compared to the functional IEP) and/or bearing a mutation in its coding sequence which ultimately lead to a decrease (and even in the absence) of the inflammation-associated biological activity of the IEP.

*Pharmaceutically effective amount or therapeutically effective amount.* These expressions refer to an amount (dose) effective in mediating a therapeutic benefit to a patient (for example prevention, treatment and/or alleviation of symptoms of an inflammatory condition). It is also to be understood herein that a "pharmaceutically effective amount" may be interpreted as an amount giving a desired therapeutic effect, either taken in one dose or in any dosage or route, taken alone or in combination with other therapeutic agents.

*Pharmaceutically acceptable salt.* This expression refers to conventional acid-addition salts or base-addition salts that retain the biological effectiveness and properties of the therapeutic agent described herein. They are formed from suitable non-toxic organic or inorganic acids or organic or inorganic bases. Sample acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, and the like. Sample base-addition salts include those derived from ammonium, potassium, sodium and, quaternary ammonium hydroxides, such as e.g., tetramethylammonium hydroxide. The chemical modification of an agent into a salt is a well known technique which is used in attempting to improve properties involving physical or chemical stability, e.g., hygroscopicity, flowability or solubility of compounds.

*Prevention, treatment and*/*or alleviation of symptoms.* These expressions refer to the ability of a method or an agent to limit the development, progression and/or symptomology of an inflammatory condition. The expressions include the prevent, treatment and/or alleviation of at least one symptoms associated to an inflammatory condition.

Acute inflammation can be observed in cerebral malaria, and/or encephalitis. Acute inflammation is usually characterized by the following symptoms: dolor (pain), calor (heat), rubor (redness), tumor (swelling) and/or *functio laesa* (loss of function). Redness and heat are considered to be due to increased blood flow at body core temperature to the inflamed site; swelling is considered to be caused by accumulation of fluid; pain is considered to be due to release of chemicals that stimulate nerve endings. Chronic inflammation can be characterized by the following symptoms: vasodilation; organ dysfunction; increased presence of acute-phase proteins (e.g. C-reactive protein, serum amyloid A, and serum amyloid P) that can cause fever, increased blood pressure, decreased sweating, malaise, loss of appetite and/or somnolence; modulation in leukocyte numbers (e.g. neutrophilia, eosinophiliam, leucopenia, etc.); modulation in interleukin, cytokine, hormone or growth factor concentration (e.g. IL-6, IL-8, IL-18, TNF-α, CRP, insulin, leptin); hyperglycemia; and/or heat.

Granulomatous inflammation is characterized by the formation of granulomas often observed in tuberculosis, leprosy, sarcoidosis, and syphilis. Fibrinous inflammation results in a large increase in vascular permeability and allows fibrin to pass through the blood vessels. If an appropriate procoagulative stimulus is present (e.g. cancer cells) a fibrinous exudate is deposited. This is commonly seen in serous cavities, where the conversion of fibrinous exudate into a scar can occur between serous membranes, limiting their function. Purulent inflammation results in large amount of pus (e.g. neutrophils, dead cells, and fluid). Serous inflammation is characterized by the copious effusion of non-viscous serous fluid, commonly produced by mesothelial cells of serous membranes, but may be derived from blood plasma. Ulcerative inflammation occurs near an epithelium and can result in the necrotic loss of tissue from the surface, exposing lower layers.

*Trigger.* As used herein, the term "trigger" (also referred to as an inflammatory response trigger) refers to agents capable of inducing and/or maintaining an inflammatory response in an individual. In some embodiment, the "trigger" can even lead to the onset of an inflammatory condition in the individual. Triggers includes, but are not limited to, bacterial infection, bacterial-derived components (such as bacteria or components derived therefrom), viral infection, viral-derived components, foreign antigens, and self antigens. In a preferred embodiment, the trigger is *P. berghei.*

### BRIEF DESCRIPTION OF THE DRAWINGS

Having thus generally described the nature of the invention, reference will now be made to the accompanying drawings, showing by way of illustration, a preferred embodiment thereof, and in which:

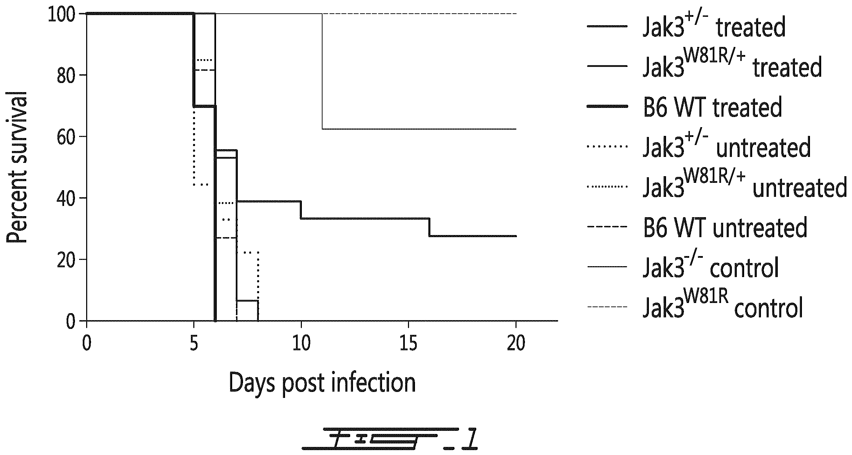

Figure 1 illustrates the percent survival rate of various mice upon challenge with *P. berghei.* Jak3 ^{+/-} mice are heterozygote for one normal copy of the *jak3* gene and one non functional copy of the gene that carries an internal deletion. Jak3^{W81R/+} mice are heterozygote for one normal copy of the *jak3* gene and one non functional copy of the gene that codes for a protein bearing the W81R variant. B6 mice are homozygote for the *jak3* gene and bear two functional copies of the gene. Jak^{-/-} mice are homozygote non functional copy of the gene that carries an internal deletion. Jak^{W81R} mice are homozygote for the *jak3* gene and both gene copies bear non-functional mutations. "Treated" refers to animal who have received a Jak3 kinase inhibitor (tasocitinib). "Untreated" refers to animal who have note received a Jak3 kinase inhibitor.

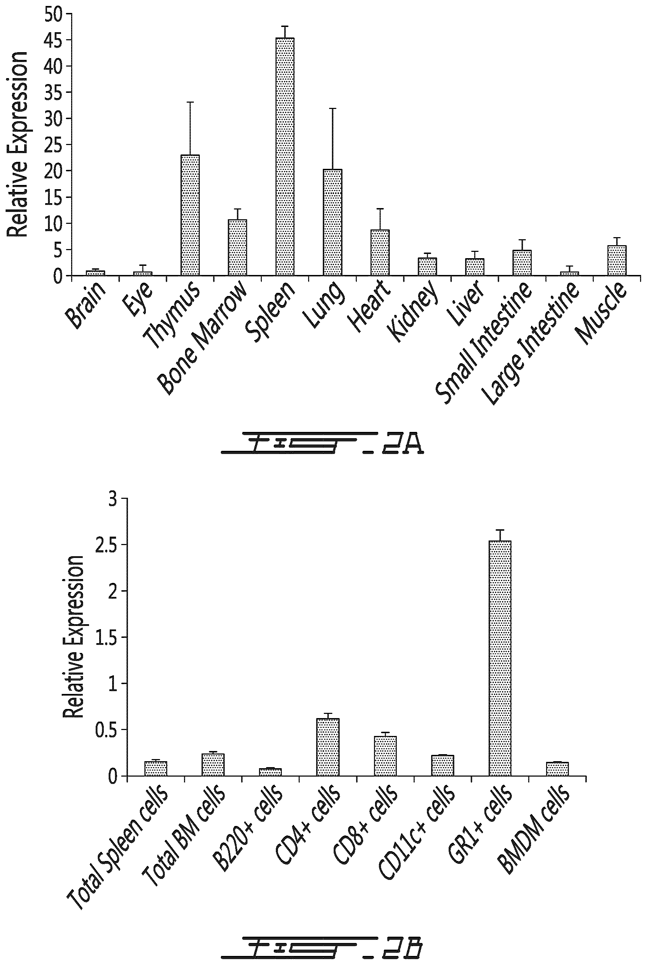

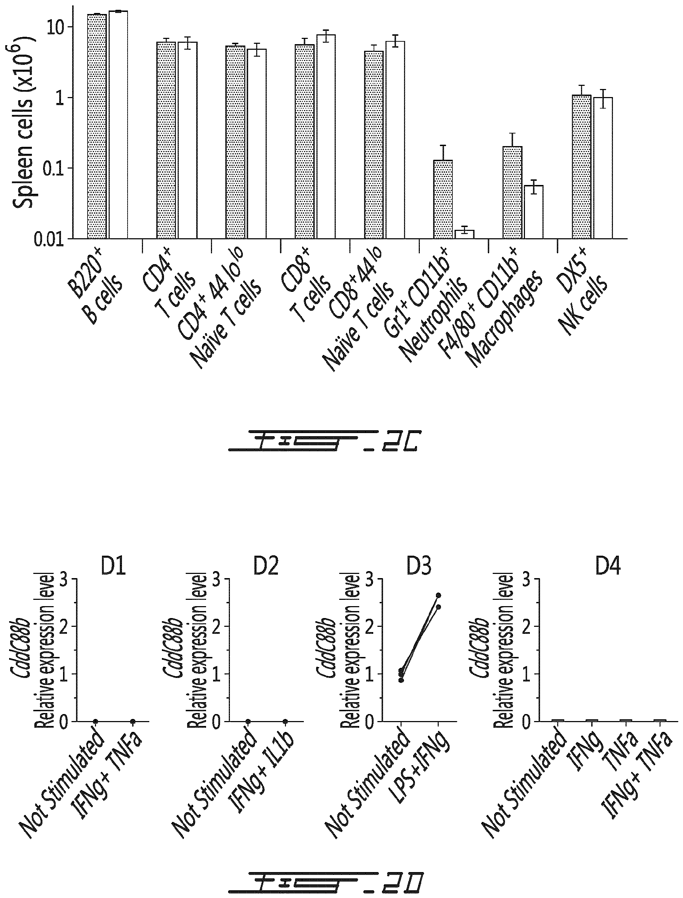

Figure 2 illustrates the expression of CCDC88B. (**A**) qPCR was performed to investigate Ccdc88b's mRNA expression across a panel of murine organs, in adult mice. Results are shown as relative expression (as a ratio to the house-keeping gene Hprt) in function of organ. (**B**) qPCR was performed to investigate Ccdc88b's mRNA expression across a panel of murine cells, in adult mice. Results are shown as relative expression expression (as a ratio to the house-keeping gene Hprt) in function of cell type. (**C**) Immunophenotyping of spleen cells of wild type animals (grey bars) and of hemyzygote animals (Ccdc88b^{+/-}, white bars). Results are shown as number of spleen cells (x10⁶) per cell type. (**D**) qPCR was performed to investigate Ccdc88b's mRNA expression in human astrocyte, microglia or blood-brain barrier endothelial cells. The relative Ccdc88b's mRNA expression level is provided for astrocytes either left unstimulated or stimulated with IFNγ and TNFα (D1) or with IFNγ and IL1b (D2); for microglia either left unstimulated or stimulated with IFNγ and LPS (D3); for BBB endothelial cells either left unstimulated or simulated with IFNγ, TNFα or with IFNγ and TNFα (D4).

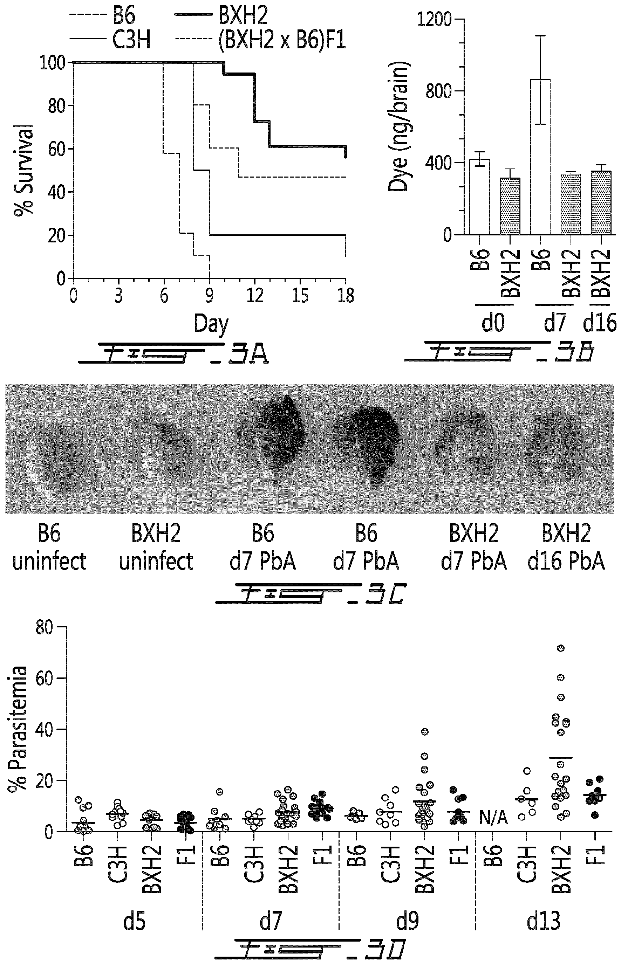

Figure 3 illustrates that BXH2 mice are resistant to cerebral malaria following *P. berghei* infection. (**A**) Survival curve of BXH2 mice, heterozygous (BXH2 x B6)F1 offspring, and susceptible B6 and C3H parental controls. All curves are statistically different from one another. (**B**) Quantification of Evans blue dye accumulation in perfused brains from uninfected and infected B6 (white bars) and BXH2 (grey bars) mice. Data are averaged from three mice per condition. (**C**) Qualitative comparison of representative Evans blue dyed brains from uninfected and infected B6 and BXH2 mice at different points in time (either 7 days post infection or d7; sixteeen days post infection or d16) which clearly indicate breakdown of the blood-brain barrier in infected B6 (d7 PbA), but not BXH2 (d7 PbA or d16 PbA) mice. (**D**) Blood parasitemia levels (percentage of parasitemia) following infection with *P. berghei* of susceptible B6 and C3H parental controls, BXH2 mice and heterozygous (BXH2 x B6)F1 offspring.

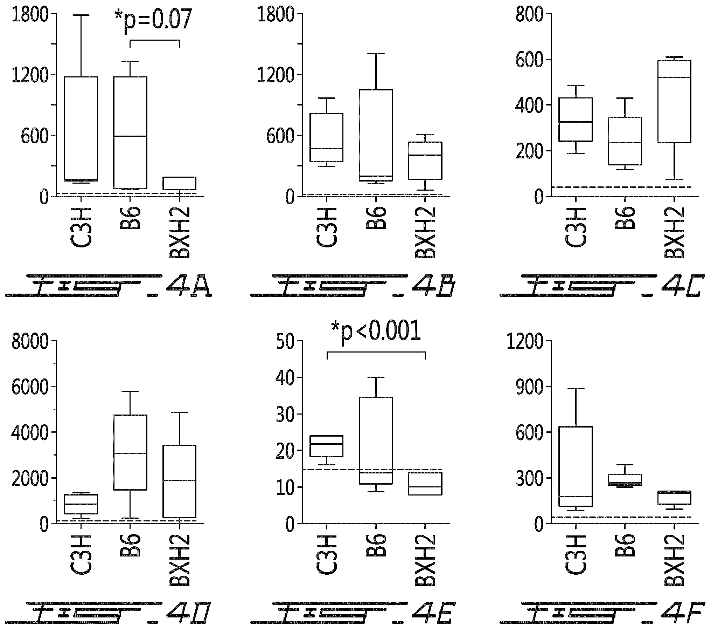

Figure 4 illustrates that BXH2 mice have a dampened serum cytokine and chemokine response compared to CM susceptible parental controls. Serum from *P. berghei* infected mice was assayed via multiplex ELISA (n=5 mice/strain) and average cytokine levels were determined. The levels of (**A**) IFN-γ (pg/mL; * = p = 0.07), (**B**) IL-10 (pg/mL), (**C**) MIP-1β (pg/mL), (**D**) CCL2 (pg/mL), (**E**) TNF-α (pg/mL; * = p < 0.001) and (F) RANTES (pg/mL) are provided in function of mouse type (susceptible B6 and C3H parental controls and BXH2 mice).

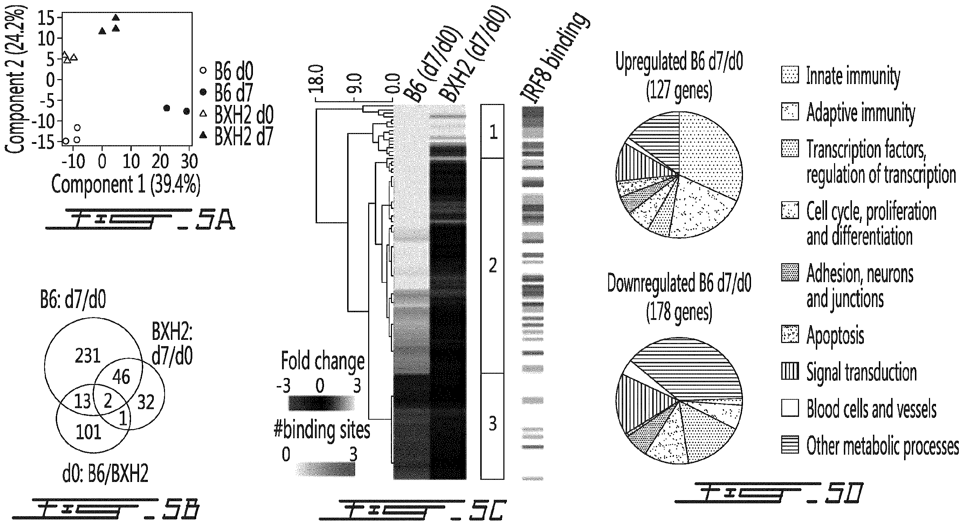

Figure 5 shows that transcript profiling of B6 and BXH2 brains reveals strain and infection specific differences in gene expression. (**A**) Unsupervised principal components analysis clusters samples according to strain and infection. Results are shown for component 2 (24.2%) in function of component 1 (39.4%) for B6 (d0 = ○; d7 = ●) and BXH2 mouse (d0 = Δ; d7 = ▲). (**B**) Intersection of gene lists generated by pairwise comparisons between infected and uninfected B6 and BXH2 transcript profiles. (**C**) Euclidean clustered heat map of transcripts regulated in both a strain and infection specific manner (two-factor ANOVA, padj-interaction<0.05) illustrated as infection-induced fold change in each strain (d7/d0). Each row represents a unique gene, and in cases where two or more transcript probes for a gene were significant, the average fold change was used. Differential expression patterns clustered into three groups with group 1 genes being upregulated by infection in both strains, group 2 genes upregulated by infection in B6 mice and unresponsive in BXH2 and group 3 genes downregulated by infection, typically more so in B6 than BXH2. See Table 1 for details. Shaded heat map indicates the presence of one or more direct IRF8 binding sites within 20kb of the gene transcription start site. (**D**) Gene ontology for transcripts differentially regulated by infection during CM pathology in B6 mice. Upregulated genes are predominantly involved in innate and adaptive immunity processes, while downregulated genes do not form a clear message, but include a variety of homeostatic biological and metabolic processes.

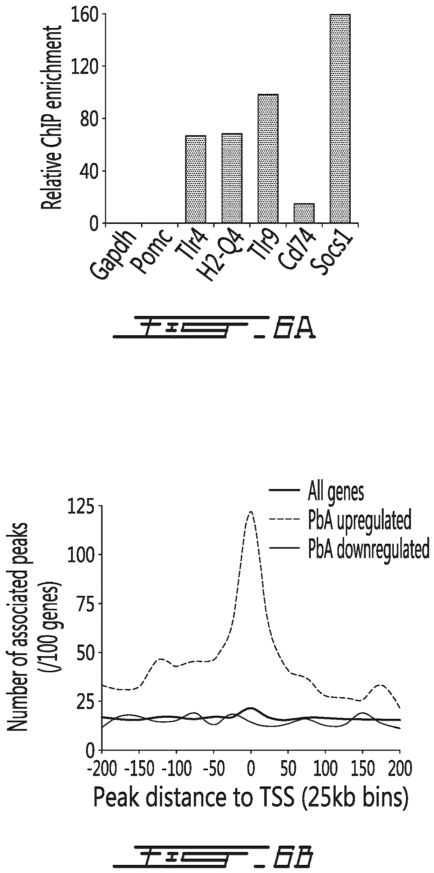

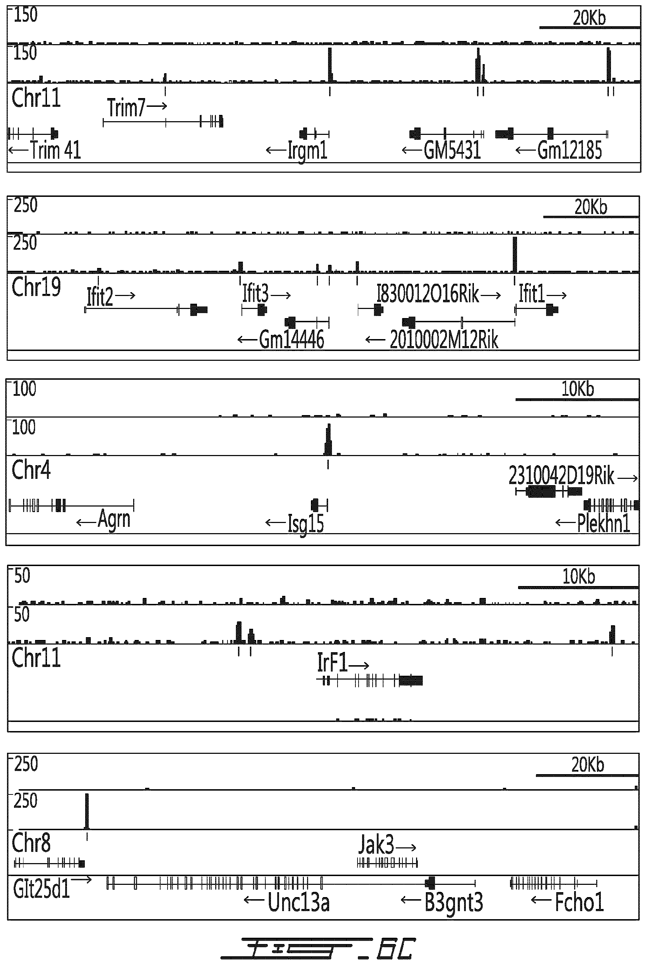

Figure 6 illustrates that genes upregulated during CM pathology in B6 mice are significantly enriched for IRF8 binding sites defined by chromatin immunoprecipitation and DNA sequencing (ChIP-Seq). (**A**) Known binding targets of IRF8 (e.g. Tlr4, H2-Q4, Tlr9, Cd74 and Socs1) were used to validate the success of the ChIP by quantitative RT--PCR. These targets were highly enriched in IRF8-immunoprecipitated DNA when compared to control IgG preparations. Representative data shown for n=5 experiments. (**B**) ChIP-Seq peaks were aligned along the mouse reference genome. Light blue (top) track indicates non-specific (IgG) binding sites and dark blue track (below) displays direct IRF8 binding sites. Genes were considered to have an IRF8 binding site if a peak was found within 20kb of the transcription start site. (**C**) The list of genes regulated by infection in B6 mice (d7/d0 pairwise) was interrogated for IRF8 binding sites within 20kb of the transcription start site. Transcripts which were upregulated in response to infection were significantly enriched for IRF8 binding sites, while downregulated transcripts showed no enrichment.

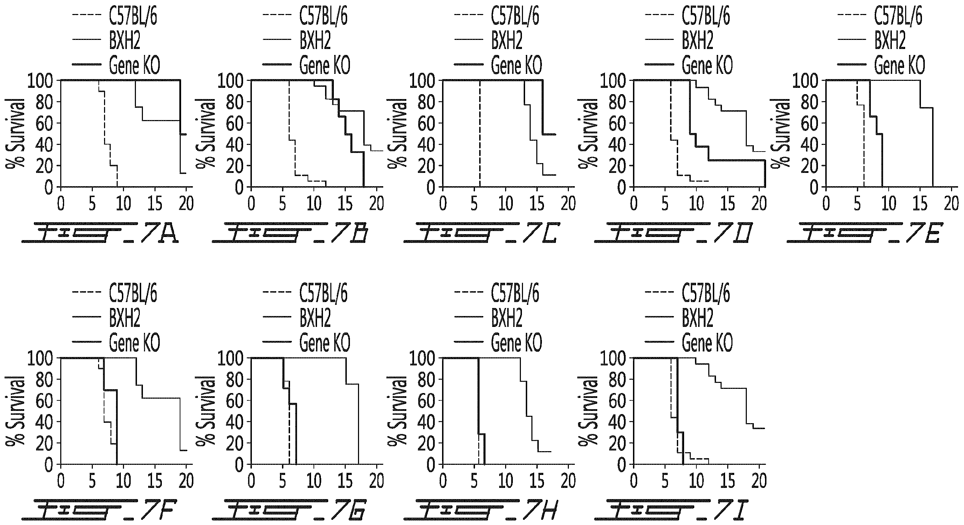

Figure 7 illustrates the survival curves for targeted gene knockout mice infected with 10⁶ *P. berghei* parasites *i.p.* Cerebral malaria susceptible mice succumbed with neurological symptoms between d5 and d10 post-infection, while mice who survived longer than 13 days never developed CM symptoms and were categorized as resistant. Infection specific B6 and BXH2 controls are plotted alongside each knockout strain. Results are shown as percentage of survivals in function of days post-infection for knock-outs of (**A**) IFN-γ, (**B**) STAT1, (**C**) JAK3, (**D**) IRF1, (**E**) IRGM1, (**F**) IL12p40, (**G**) IFIT1, (**H**) ISG15, and (**I**) NLRC4.

### DETAILED DESCRIPTION

In accordance with the present invention, there is provided host polypeptides (also refer to as targets) which are herein shown to enable the induction and/or persistence of a pathological inflammatory response. These polypeptides are considered to be involved in any pathological inflammation response, regardless of the etiology of the disease. As also shown herein, reduction in the biological activity of these polypeptides is shown useful for the treatment, prevention and/or alleviation of symptoms associated with an inflammatory condition.

Common inflammatory diseases such as psoriasis, rheumatoid arthritis, multiple sclerosis, lupus, celiac disease and IBD show deregulation of common pathways and associated signaling networks. Therefore, pharmacological modulators interfering with such shared pathways associated with pathogenesis may be of clinical benefit for several inflammatory diseases. Large-scale genetic studies show that the genetic component of these diseases is diverse and highly complex and, importantly, that they do share several of the same genetic risk alleles. This suggests that genes discovered as playing a role in one inflammatory condition may prove valuable to better understand the pathophysiology of related inflammatory conditions and may represent valuable targets for drug discovery in these disorders. Studies in experimental models have established that the principal cell types, physiological responses, and associated pathways and soluble mediators underlying normal and aberrant inflammatory responses are conserved in the mouse. In addition, germ-line modification in mouse provides an ideal model to study in isolation the contribution of individual genes and proteins to pathological inflammation, while carefully controlling triggering environmental stimuli.

Building on these findings, a random mutagenesis in mice is proposed followed by screening of the resultant mutants for resistance to acute neuroinflammation provides a systematic approach to identifying functionally validated factors that a) play crucial roles in a spectrum of inflammatory diseases, and b) constitute novel targets for drug discovery and pharmacological intervention in inflammatory conditions. As it will be provided below, such strategy has resulted in the identification of Jak3 (a known pharmacological target in RA) and Themis (a known genetic risk factor in SLE and celiac disease), providing convincing proof-of-principle of the robustness and power of this genetic approach. Thus, this approach provides the missing functional link absent in GWAS-derived information as well as an *in* vivo-validated pre-clinical system in which pharmacological inhibition would sufficiently blunt the inflammatory response to be clinically valuable.

IBD affect about 1.4 million individuals in North America and 2.2 million in Europe. Since the 1940s, the incidence of IBD has dramatically increased in countries with a more westernized lifestyle, suggesting the influence of environmental factors, including lifestyle, hygiene, diet and use of antibiotics, all of which may alter the microbiota in favor of disease onset and/or progression. A large body of evidence also points to genetic factors in the etiology of IBD, with more than 100 loci identified in GWAS. However, these loci account for only 20% of the estimated genetic risk for IBD. Furthermore, although the loci identified by GWAS point to specific pathways with implications in inflammation, epithelial barrier function, innate and adaptive immunity, autophagy, ER stress and others, it is currently impossible to rationally select a candidate druggable molecule/pathway from these studies due to lack of functional studies evaluating the non-redundant in vivo contribution of each of these loci to disease. The neuroinflammation model induced by *Plasmodium berghei* infection was chosen because it harbors several features common to inflammatory conditions, including recruitment of inflammatory myeloid and lymphoid cells to the site of infection, secretion of IL12, TNFα, IFNγ and subsequent disruption of endothelial cells integrity leading to a rapid and lethal encephalitis. Further, as it will be shown below, a number of genes which role in inflammation was previously unsuspected, establishing the proven potential of discovery of novel druggable targets.

### Genetic screening and characterization of putative inflammation-enabling targets

Mutagenesis with the alkylator N-ethyl N-nitrosourea is an efficient method to create random single point mutations in the mouse genome. A set of validated conditions was developed to successfully mutagenize the C57BL/6J (B6) mouse strain (Bongfen *et al*.). Briefly, founder B6 male mice (identified as G0) receive 1 intraperitoneal injection of 90 mg/Kg of ENU weekly for 3 weeks. Efficiently mutagenized males (G0) transiently lose fertility, which is regained after 11 weeks. Mutagenized G0 males are then crossed with wild-type B6 female mice to produce generation 1 (G1) offspring: these F1 hybrids carry one full set of mutagenized chromosomes and one full set of wild-type B6 chromosomes. Based on an average substitution rate of 1 nucleotide per 1 Mb, each G1 is expected to carry ∼3000 nucleotide variants in heterozygous state. Using an average frequency of reduction-of-function mutations of 1 per locus per 700 G1 mice, determined for visible loci, each G1 mouse is likely to be a heterozygous carrier for about 40 to 50 functional variants. Individual G1 males are bred as founders of separate B6 pedigrees aimed at bringing the ENU sequence variants (from the G0 male) to homozygosity, by first crossing the G1 male with a wild-type B6 female (to produce G2 offsprings) and then backcrossing two of the ensuing G2 daughters back with their G1 father. 50% of the sequence variants present in the G1 are inherited by each G2 daughter, and 25% percent of these (12.5% of the total) are expected to come to homozygosity in any one N3 offspring. Overall, each G3 offspring is thus expected to be homozygous for an estimated 375 sequence variants and 4 functional variants. During systematic phenotyping campaigns of these N3 pedigrees, the consistent appearance (at a frequency of about 25%) of individuals harboring a discordant or "phenodeviant" trait (e.g. resistance to lethal neuroinflammation) in successive G3 pedigrees from the same G1 grand-father, indicates segregation of a fully penetrant recessive mutation modifying response to a stimulus. Thus, by screening a minimum of 24-32 N3 offspring (six litters) per pedigree, one can expect to identify a cluster of 6-8 individuals with the same phenodeviant trait.

*Plasmodium berghei* ANKA (PbA) induces cerebral malaria, a condition characterized by acute neuroinflammation and rapidly fatal encephalitis. It is caused by trapping of PbA-parasitized erythrocytes at the blood-brain barrier (BBB), infiltration of inflammatory cells and production of IFNγ, TNFα, IL10, MCP-1, loss of endothelial integrity at the BBB, and precise onset of irreversible neurological symptoms that appear by day 5 post-infection and that are ultimately fatal by day 9-11. The infectious *P. berghei* ANKA (PbA) isolate was obtained from the Malaria Reference and Research Reagent Resource Centre (MR4), and stocks of parasitized erythrocytes (pRBCs) were prepared and stored frozen at -80°C. Prior to infection, the parasite is passaged intravenously, and the blood is diluted to prepare a large inoculum (titrated at 10⁷ pRBC/mL) to infect groups of 100-150 G3 mice (10⁶ pRBC, i.v). Starting at d3 post-infection, animals are monitored several times for the appearance of neurological symptoms which may range from shivering, tremors, hunched-back, lack of responsiveness to touch, and may progress to paralysis and coma. At first sight of such symptoms (usually by days 5 to 7), mice are euthanized and a tissue sample is collected by isolation of genomic DNA. Mice surviving d12 post-infection are flagged as resistant to neuroinflammation, and the corresponding G3 pedigree as phenodeviant. Additional G3 pedigrees from the same G1 founder are then phenotyped to ascertain stable segregation of the protective trait, and establish its frequency of transmission (expected about 25% G3 individuals in a positive pedigree) amongst a minimum of 24-32 G3 offspring (6 litters from the same G2 female). Resistant animals surviving the cerebral phase are tested for blood parasitemia (to ascertain productive infection), and are then drug-cured (Artemisin, s.c, 150mg/kg, 3 x per week for 3 weeks) to preserve the protective mutation in live animals for future propagation of the mutant line.

The sequence of all exons and exon-intron boundaries (exome sequencing) from 3 G3 mice from the same phenodeviant pedigree is determined using the Agilent SureSelectXT Mouse All Exon™ system. This system uses nested oligonucleotide primers to efficiently construct libraries that capture >221,784 exons within 24,306 annotated mouse genes (annotated using UCSC mm9/NCBI build 37 from reference C57BL/6J genome) for a total 49.6Mb captured genomic DNA. The captured exons are then subjected to deep sequencing using the Illumina HiSeq™ sequencing platform. These two components have been optimized for efficient and reliable whole genome coverage and for detection of ENU-mutations: 200-300 x 10⁶ sequence reads of an average read length of 180-200 nucleotides being sufficient to provide 20X coverage for >90% of the genome and 10X coverage for >95% . A read alignment and variant-calling pipeline based on the freely available tools BWA, SAM tools, Picard, and GATK have been implemented. This list is curated to eliminate duplicate reads and to flag sequencing errors (ratios of SNP to reference allele <50%, multiple allele systems, end sequences) using the IGV viewer™ software package (Broad Institute, MIT), and to identify other non-ENU irrelevant variants (previously seen in other exome files) or silent variants that do not affect the amino acid sequence. SNP lists from the 3 G3 mice are compared to identify variants common to the 3 mice (homozygote or heterozygote) states.

The causative nature of the ENU variant(s) are validated by genotyping 20 additional G3 mice from the same G1 grandfather, and by looking for co-segregation of homozygosity for the ENU-specific variant(s), and exclusion of homozygosity for wild type alleles in the "resistant" mice while ascertaining the reverse situation in "susceptible" mice. The cause for the loss-of-function in the protective variants are then examined. Although mis-sense variants causing either premature termination of the polypeptide, or intronic mutations in key donor or acceptor splice sites likely to affect RNA processing/splicing readily identify obvious loss-of-function, non-synonymous variants can be prioritized with respect to the type of substitution (non-conservative > conservative) and for residues showing cross-species conservation (BLAST or BLOSSOM searches). Genetic complementation in F1 mice double heterozygote for the ENU variant and for a null allele at the same gene (looking for recapitulation of protection against *P. berghei*) can be used as an ultimate validation test. Such null mutants may be obtained as live animals from independent laboratories or from public resources (Jackson Laboratories) or may be obtained as gene traps from the International Gene Trap Consortium (www.igtc.org.uk). To gain further insight into the functional role of poorly annotated proteins, the expression of their RNA by semiquantitative reverse transcriptase coupled amplification (RT-PCR) in organs (thymus, spleen, bone marrow, blood leukocytes) and cells (myeloid, lymphoid, epithelial) associated with inflammatory responses *in vivo* can be performed. The promoter region of these genes can be studied for the presence of sequence elements associated with regulation by transcription factors known to regulate "pro-inflammatory" pathways (such as STAT1, STAT3, IRF1, and IRF8). Finally, mutant mouse lines homozygote for prioritized ENU-variants are expanded to generate sufficient numbers of mice for downstream characterization.

Mutant phenodeviant pedigrees are subjected to a streamlined and stratified immunophenotyping analysis to determine the integrity, composition and activation status of their central and peripheral immune systems. Specifically, the different immune cell populations are enumerated (hematopoeitic stem cells [HSC], granulocytes, myeloid cells, NK cells, T and B lymphocytes) in central and peripheral immune organs (bone marrow, thymus, spleen, lymphnodes and peripheral blood) as well as in the gut, using population-specific cell surface markers and intracellular cytokine staining in a high throughput multivariate flow cytometry analysis. The following antibody cocktails can be used: for T cells (anti-CD3, CD4, CD8, CD44, CD62L, CD25, TCRVβ, γδTCR), B cells (anti-B220, CD22, CD138, IgM, IgD, CD24), HSC (anti-CD117, Sca-1), erythroid cells (anti-Ter119), granulocytes (anti-Gr-1), NK cells (anti-NK1.1), macrophages, DCs, inflammatory monocytes (anti-CD11b, CD11c, F4/80), and eosinophils, mast cells, basophils (anti-SiglecF, CD117, FcERI). Immune cell functions are interrogated by analyzing different immunological responses both ex *vivo* and *in vivo.*

The genetic screen provides decisive proof-of-principle and support these conclusions: a) the ENU screen is a robust and effective platform to identify mutations (and ultimately genes) that blunt acute neuroinflammation; b) some of the mutations detected so far are in genes are already known to be critical for the inflammatory response (*e.g.* JAK3) and/or c) other mutations detected so far are in genes are already known to be important in the genetic etiology of human chronic inflammatory diseases (*e.g.* THEMIS, IRF8) indicating that genes discovered in an acute inflammation screen in mice are relevant to other types of human inflammatory conditions; d) likewise, the neuroinflammation protective effect of a genetic mutation in the screen (*e.g.* JAK3) can be mimicked by pharmacological inhibition of the corresponding target with compounds in clinical use for chronic human inflammatory diseases (refer to Example I); e) the acute neuroinflammation model can be used as a primary screen *in vivo* for the rapid and effective pre-clinical evaluation of novel anti-inflammatory drug candidates; f) novel genes/proteins with unknown function are also identified and represent novel windows of opportunity for anti-inflammatory drug discovery; g) the screen is very sensitive and can detect gene-dosage dependent pathways since the mutations show partial resistance to neuroinflammation suggesting that the screen can detect targets of which partial inhibition *in vivo* may be of clinical value. The genetic screen herewith presented is also advantageous because it provide inflammation-enabling polypeptides derived from the host.

To further characterize the targets of the genetic screen, for unknown genes/proteins or proteins with unknown function in inflammation, different cell populations can be sorted using the AutoMACS™ system or more precise cell sorting strategies when necessary (e.g. BD FACSAria™) and can be challenged *in vitro* with appropriate triggers including PRR agonists (LPS, PGN, CpG, polyl:C, MDP, DAP, B-DNA), recombinant cytokines or mitogens (IL-2, IL-7, anti-CD3/CD28, PMA+ionomycin). Cell-based assays can be conducted to monitor cell survival (by Annexin V/propidium iodide staining and enumeration by FACS), proliferation (by following the dilution of the CFSE stain by FACS) and activation (intracellular staining for IFNγ [Th1], IL-17 [Th17], IL-4 [Th2], FoxP3 [Treg], and cell surface staining of MHC Class II and the co-stimulatory molecules CD80/CD86 [DCs]). These assays can be complemented with *in vivo* challenge and immunization experiments to determine the activation, survival and migratory capacities of the granulocytes, myeloid and lymphoid cells under investigation. Immunohistochemistry, bead-based immunoassays (BioPlex) and ELISA assays for different serum cytokines, chemokines and immunoglobulins can be used to assess the in vivo innate and adaptive (cellular and humoral) immune responses. Mice can be injected intraperitoneally (IP) with PAMPs/DAMPs (*e.g*. LPS, PGN, MSU, Alum) and flow cytometry is used to quantify the frequencies and numbers of infiltrating granulocytes and myeloid cells into the peritoneal cavity (Gr-1+, CD11b+, F4/80+, SiglecF+, FcERI+). Cytokines and chemokines in the peritoneal lavage and the serum can be quantified by BioPlex and ELISA assays. Classical immunization experiments can involve the immunization of animals by IP injection of DNP-Ficoll or DNP-KLH in complete or incomplete Freund's adjuvant followed by an antigenic rechallenge/boost on day 28. The production of different immunoglobulins (IgA, IgG1, IgG2a/2b, IgG3, IgM) and cytokines (IFNγ, IL-4, IL-5, IL-13, IL-17) can be quantified by BioPlex and ELISA. Acquired cellular immune response can be interrogated in delayed-type hypersensitivity assays (intraperitoneal immunization with TNBS and rechallenge in the foot pad and measurement of foot pad swelling as a readout).

A more in-depth analysis of the impact of the mutation on the inflammatory response can be undertaken by identifying transcriptional signatures (using RNAseq™) in the relevant immune cell type distinguishing inflammation-resistant versus inflammation-susceptible mice. This comprehensive analysis not only defines the immunological mechanism(s) by which the identified targets impact the inflammatory response but can also provide an integral view of the cell types and pathways involved in pathologic inflammation, and might provide additional drug targets that function either upstream or downstream of the identified genes/proteins with equivalent consequence on immune cell function and physiopathology of inflammatory disease.

The relevance of neuroinflammation-protective mutations previously identified can be assessed in other inflammatory conditions, such as an animal models of IBD. Experimental animal models of IBD individually recapitulate aspects of the problem, and have provided important insights into the role of the pathways, cells and molecules required for intestinal homeostasis. This is best illustrated by the remarkably convergent mouse and human studies on the role of the IL-23-Th17 cell pathway in IBD. IL-23p19 was first cloned in the mouse and has been shown to be required for colitis to develop in multiple models. Neutralization of IL-23 completely reversed active colitis in one model, which is consistent with data showing that the dominant IL23R SNP, which is protective in IBD, encodes for an alternative splicing of the gene resulting in a soluble receptor antagonist of IL-23. Similarly, the Nlrp3 inflammasome-IL-18 axis has been linked to protection from colitis, which is consistent with human genetic studies identifying SNPs in the NLRP3 and IL-18 receptor accessory protein (IL18RAP) as risk alleles for IBD. The response of the mutant mice identified in the primary screen can be examined in a number of complimentary models of acute and chronic inflammation of the bowel that each interrogates a specific aspect of the immune response. Such models include, but are not limited to, the acute and chronic dextran sulfate sodium (DSS)-induced injury/colitis models to probe for innate immunity mechanisms; the CD4+CD45RB^{Hi} T cell transfer colitis model to probe for Th1/Th17 mechanisms as well as deregulated Treg functions; the oxazolone colitis model to examine Th2 mechanisms and infection with *C. rodentium* to probe for epithelial and innate immunity anti-microbial defense mechanisms. Various parameters can be systemically measured to determine the severity of colitis, including body weight loss, colon histology to assess crypt architecture, loss of goblet cells, erosion of surface epithelia, infiltration of inflammatory cells and edema, quantitative real time PCR and BioPlex/ELISA assays to measure the expression of various antimicrobial peptides (including defensins), chemokines and cytokines in the colon tissue and in the serum, qPCR of 16S rRNA to determine extent of bacterial invasion in the colon, and to systemic sites (mesenteric lymphnodes and spleen), and flow cytometry analysis to enumerate the frequencies and absolute numbers of the different populations of immune cells in the colitic gut (neutrophils, macrophages, T and B cells).

It can also be investigated whether common polymorphic variants (SNPs) within or near the human counterparts of the targets have been associated as genetic risks of common inflammatory diseases in genome wide association studies (GWAS). Published GWAS studies have identified a very complex genetic component to common inflammatory conditions including rheumatoid arthritis (RA), psoriasis (PA), celiac disease (CeD), multiple sclerosis (MS), type 1 diabetes (T1D), lupus (SLE), and inflammatory bowel disease (IBD). Although these GWAS studies have defined genetic signatures and associated immune/biochemical pathways that are either shared in common or that are specific for one disease, translating GWAS hit(s) into potential novel target(s) for drug discovery and therapeutic intervention is complicated by many factors, the most important being the lack of direct experimental validation of the biological role of the individual genes in onset/progression of disease. A positive correlation between the genes which inhibition in mice protects against acute inflammation, and genetic risks for either RA, PA, CeD, MS, T1D, SLE or IBD in published GWAS studies can bring biological validation on the role of this gene in human inflammatory diseases, a key criterium for prioritization of this gene and protein in our discovery pipeline.

It can also be determined if the expression of the inflammation-enabling target is modulated in tissue samples from patients suffering from chronic inflammatory diseases. One approach is based on reports showing that an important proportion (up to 30%) of allelic variants linked to disease (linked SNPs) are also associated with allele-specific differential expression of the associated genes (cis-acting eSNP, eQTL). These published datasets can be searched for presence of eSNPs in human counterparts of the inflammation-enabling mouse targets, and which differential expression may be associated with disease. In another approach, the expression of the inflammation-enabling target in normal intestinal mucosa can be compared to the expression of the inflammation-enabling target in inflammed mucosa from IBD patients (via, for example, RNA sequencing technology to capture in whole tissue RNA or following microdissection) whole transcriptome expressed in normal and inflamed tissues and PBLs. Finally, it can be tested whether rare sequence variants in human relatives of mouse targets are associated with specific clinical features of IBD, or with IBD onset and progression in certain genetically unique groups of patients such as familial cases, very early onset pediatric cases, and sporadic cases from genetically homogeneous isolated populations. In an embodiment, individual exons and exon-intron junctions of each gene can be PCR-amplified can subjected to DNA sequencing, looking for obvious loss of function alleles or for non-conservative and possibly pathological mis-sense mutations, using standard methodologies. In a complementary or alternative embodiment, intronic sequences can also be determined using standard methodologies.

If inflammation-enabling protein targets require further degrees of experimental characterization, for protein with a known biochemical function or ligand binding, tractable modification of substrate can be conducted. Bioinformatics tools can also be used to scrutinize the predicted amino acid sequence to a) deduce biochemical function by relatedness to other proteins which function is known, and b) sequence motifs and signatures, and associated structural folds associated with specific biochemical activity (nucleotide binding, protease site, DNA binding, kinase, phsophatase, etc.), and c) presence of secondary structure motifs indicative of protein localization (hydrophobic transmembrane domains, signal sequences, nuclear localization sequences), prioritizing for pharmacological access. Candidate biochemical activities for such proteins can be validated following transfection and overexpression in cultured cells and looking for appearance of candidate activity and/or changes in cellular components related to functional markers of inflammation, or conversely, looking for their disappearance following silencing by RNAi or ShRNA molecules.

In instances where an inflammation-enabling protein may not be an attractive pharmacological target, but may belong to a pathway that harbors other "druggable" sites, the pathways of the inflammation-enabling protein can be identified (using, for example, bioinformatic tools, or published results from large scale transcriptomics or proteomics analyses). In addition, direct experimentation in transfected cells may also be undertaken, for example chromatin immunoprecipitation/sequencing to identify targets of a DNA binding protein, and proteomic analyses to identify substrates of a modifying enzyme (protease, kinase, phosphatase, ubiquitin ligase). Such pathways may also harbor proteins for which small molecule modulators are already available.

### Screening methods for therapeutic agents

The screening methods described herein are designed to capture the relationship between the inflammation-enabling polypeptides' expression and/or activity (collectively referred as the IEP's biological activity) and inflammation to generate valuable information about the agent that is being screened.

Since the expression/activity of the inflammation-enabling polypeptides is shown to be up-regulated during inflammation, the agents identified by the screening methods provided herewith also likely to have the advantage of limiting inflammation and providing therapeutic benefits in conditions associated with exacerbated inflammation if they are shown to reduced the IEP's biological activity.

In screening applications, an agent to be screened is placed in contact with a reagent. A reagent suitable for this type of application has a parameter which is associated (directly or indirectly) to the biological activity of the inflammation-enabling polypeptide. If the biological activity of the inflammation-enabling polypeptide is limited, impeded or event inhibited by contacting the screened agent, it is expected that such limitation, impediment or inhibition be also reflected in the reagent's parameter. In one embodiment, the parameter's level or measure is lowered (with respect to a control level) when the biological activity of the inflammation-enabling polypeptide is lowered by the presence and/or contact with the screened agent.

In a first embodiment, the reagent is a nucleic acid molecule. The nucleic acid molecule can be a promoter or a fragment thereof derived from one of the inflammation-enabling polypeptides and being capable of modulating the expression of its downstream operably-linked open reading frame (such as, for example, the gene encoding for the inflammation-enabling polypeptide or another suitable reporter gene). When the nucleic acid molecule is a promoter (or a functional fragment associated thereto), the parameter that is being measured is the level of expression of the downstream operably-linked reporter gene, which is associated with the ability (or lack thereof) of the screened agent to limit, impede or inhibit the genetic expression driven from the promoter (or functional fragment). If an agent is capable of limiting, impeding or inhibiting the expression of the reporter gene, it is assumed that the expression from the promoter is also limited, impeded or inhibited by the agent. Alternatively, if the agent is not capable of limiting, impeding or inhibiting the genetic expression of the reporter gene from the promoter, it is assumed that the expression from the promoter is not limited, impeded or inhibited by the agent (and that therefore the agent can lack the therapeutic utility).

In another embodiment, the nucleic acid molecule is a transcript, such as, for example a mRNA or its corresponding cDNA. As used herein, a transcript is a nucleic acid molecule copy of at least one section of a coding region of a gene. The transcript can be, for example, coding for the inflammation-enabling polypeptide. In this particular embodiment, the parameter is the stability and/or amount of the transcript. If the agent is capable of limiting, impeding or inhibiting the stability and/or amount of the transcript encoding the inflammation-associated polypeptide, it is assumed that the level of the transcript is limited, impeded or inhibited by the agent. Alternatively, if the agent is not capable of limiting, impeding or inhibiting the stability and/or amount of the transcript, it is assumed that the level of the transcript is not limited, impeded or inhibited by the agent.

In still another embodiment, the nucleic acid molecule is a transcript expressed from a gene whose expression is modulated by the inflammation-enabling polypeptide. This embodiment is particularly advantageous when the inflammation-enabling polypeptide is a transcription factor modulating the expression of other gene (also referred to as downstream genes). In this particular embodiment, the parameter is the stability and/or amount of the transcript. Even though the transcript of a single gene whose expression is modulated by the inflammation-associated polypeptide can used in this method, it is also contemplated that the level of transcripts associated with at least 5, at least 10, at least 15, at least 20, at least 25, at least 50, at least 100, at least 200 or at least 250 genes whose expression is modulated by the inflammation-enabling polypeptide be used to perform the method. If the agent is capable of modulating the stability and/or amount of at least one, at least 5, at least 10, at least 15, at least 20, at least 25, at least 50, at least 100, at least 200 or at least 250 transcript(s) of gene whose expression is modulated by the inflammation-associated polypeptide, it is assumed that the level of the transcript(s) is modulated by the agent. Alternatively, if the agent is not capable of modulating the stability and/or amount of at least one, at least 5, at least 10, at least 15, at least 20 at least 25, at least 50, at least 100, at least 200 or at least 250 transcript(s) of gene whose expression is modulated by the inflammation-associated polypeptide, it is assumed that the level of the transcript is not modulated by the agent.

In a further embodiment, the reagent is a polypeptide. The polypeptide can be, for example, the inflammation-enabling polypeptide or a functional fragment thereof. In this particular embodiment, the parameter can be the stability, amount and/or biological activity of the polypeptide. If the agent is capable of limiting, impeding or inhibiting the stability, amount and/or the biological activity of the inflammation-associated polypeptide, it is assumed that the polypeptide is limited, impeded or inhibited by the agent. Alternatively, if the agent is not capable of limiting, impeding or inhibiting the stability, amount and/or biological activity of the polypeptide, it is assumed that the polypeptide is not limited, impeded or inhibited by the agent.

In still a further embodiment, the reagent is a polypeptide (or a functional fragment thereof) in the same signaling pathway as the inflammation-enabling polypeptide. In this particular embodiment, the parameter can be the stability, amount, chemical structure/modification (e.g. phorphorylation state) and/or biological activity of the polypeptide. If the agent is capable of limiting, impeding or inhibiting the stability, amount and/or the biological activity of the inflammation-associated polypeptide, it is assumed that the polypeptide is limited, impeded or inhibited by the agent. Alternatively, if the agent is not capable of limiting, impeding or inhibiting the stability, amount and/or biological activity of the polypeptide, it is assumed that the polypeptide is not limited, impeded or inhibited by the agent.

In yet a further embodiment, the reagent is a polypeptide (or a functional fragment thereof) capable of physically interacting (either directly or indirectly) with the inflammation-enabling polypeptide. In this particular embodiment, the parameter can be a measure of association between the inflammation-enabling polypeptide and its binding partner. In instances where the binding of the IEP with its partner can cause a modification of the partner's chemical structure (phosphorylation state or ubiquitination state),the parameter can be the stability, amount, chemical structure (e.g. phorphorylation state) and/or biological activity of the partner. If the agent is capable of limiting, impeding or inhibiting the association of the two polypeptides or the chemical modification of the partner, it is assumed that the inflammation-enabling polypeptide's biological activity is limited, impeded or inhibited by the agent. Alternatively, if the agent is not capable of limiting, impeding or inhibiting the association of the two polypeptides, it is assumed that the inflammation-enabling polypeptide is not limited, impeded or inhibited by the agent.

Even though a single parameter is required to enable the characterization of the agent, it is also provided that more than one parameters of the reagent may be measured and even that more than one reagents may be used in the screening applications provided herewith.

As it will be further discussed below, the contact between the agent and the reagent can be made *in vitro* (in a reaction vessel that can accommodate the measurement of a reagent's parameter for example) or *in vivo* (in an animal for example). For screening applications, a suitable *in vitro* environment for the screening assay described herewith can be an isolated reagent (such as an isolated nucleic acid molecule or polypeptide) in an appropriate buffer with the necessary other reagents. Another suitable *in vitro* environment can be a cell (such as a cultured cell) or a cellular extract. When a cultured cell is used, in some embodiment, it is advisable to maintain viability its viability in culture. The cultured cell(s) should be able to provide the reagent. The cell is preferably derived from a myeloid or lymphoid tissue (primary cell culture or cell line). If a primary cell culture is used, the cell may be isolated or in a tissue-like structure.

For *in vivo* screening applications, a further suitable environment is a non-human model, such as an animal model. If the characterization of the agent occurs in a non-human model, then the model (such as a rodent) is administered with the agent. Various dosage and modes of administration maybe used to fully characterize the agent's ability to prevent, treat and/or alleviate the symptoms of an inflammatory condition.

Once the agent has been contacted with the reagent, a measurement or value of a parameter of the reagent is made. This assessment may be made directly in the reaction vessel where the contact took place (by using a probe for example) or on a sample of such reaction vessel. The measurement of the parameter of the reagent can be made either at the DNA level, the RNA level and/or the polypeptide level.

The measuring step can rely on the addition of a quantifier specific to the parameter to be assessed to the reaction vessel or a sample thereof. The quantifier can specifically bind to a parameter of the reagent that is being assessed. In those instances, the amount of the quantifier that specifically bound (or that did not bind) to the reagent can be determined to provide a measurement of the parameter of the reagent. In another embodiment, the quantifier can be modified by a parameter of the reagent. In this specific instance, the amount of modified (or unmodified) quantifier will be used to provide a measurement of the parameter of the reagent. In an embodiment, the signal of the quantifier can be provided by a label that is either directly or indirectly attached to a quantifier.

To assess the transcription activity from a promoter of a gene of interest (either associated with the gene encoding the inflammation-enabling polypeptide or associated with target genes whose expression is modulated by the inflammation-enabling polypeptide), a reporter assay can be used. In reporter assays, a reporter vector is placed in contact with an agent and the level of expression (via the amount of the transcript) of the reporter gene is measured to provide for transcription activity from the promoter. The reporter vectors can include, but are not limited to, the promoter region (or a functional fragment thereof) of the gene of interest operably linked to a nucleotide encoding a reporter polypeptide (such as, for example, luciferase, β-galactosidase, green-fluorescent protein, yellow-fluorescent protein, *etc*.). Upon the contact of the agent with the reagent, the promotion of transcription from the promoter is measured indirectly by measuring the transcription of the reporter polypeptide. In this particular embodiment, the quantifier is the reporter polypeptide and the signal associated to this quantifier that is being measured will vary upon the reporter polypeptide used.

Alternatively or complementarily, the stability and/or the expression level of the nucleic acid molecules can be assessed by quantifying the amount of the nucleic acid molecule (for example using qPCR or real-time PCR) or the stability of such molecule (for example by providing at least two measurements in function of time). In one assay format, the expression of a nucleic acid molecule in a cell or tissue sample is monitored via nucleic acid-hybridization techniques (*in situ* hybridization for example). In another assay format, cell lines or tissues can be exposed to the agent to be tested under appropriate conditions and time, and total RNA or mRNA isolated, optionally amplified, and quantified.

In some embodiments, the nucleic acid identity of a nucleic acid molecule or transcripts can be performed. Various methods of determining the nucleic acid sequence of a nucleic acid molecule are known to those skilled in the art and include, but are not limited to, chemical sequencing (*e.g.* Maxam-Gilbert sequencing), chain termination methods (*e.g.* Sanger sequencing, and dye-terminator sequencing), restriction digestion-based sequencing (*e.g.* RFLP), hybridization-based sequencing (*e.g.* DNA micro-array, RNA micro array, Molecular Beacon probes, TaqMan probes), mass spectrometry-based sequencing, next generation sequencing (e.g. whole exome sequencing, Massively Parallel Signature Sequencing or MPSS, Polony sequencing, pyrosequencing, Illumina™ (Solexa) sequencing, SOLiD™ sequencing, ion semiconductor sequencing, DNA nanoball sequencing, Helioscope™ single molecule sequencing, Single Molecule SMRTTM sequencing, Single Molecule real time (RNAP) sequencing, and Nanopore DNA sequencing).

If the measurement of the parameter is performed at the polypeptide level, an assessment of the polypeptide level of expression can be performed. In an embodiment, specifically the level of expression of the polypeptide is measured for example, through an antibody-based technique (such as an immunohistochemitry, BioPlex, ELISA, flow cytometry, protein micro-array, immunodetection), spectrometry, etc. In one embodiment, this assay is performed utilizing antibodies (or derivatives therefrom) specific to target polypeptides.

As shown below, some of the inflammation-enabling polypeptide (such as FOXN1) are transcription factors which modulates the expression of downstream target genes involved in the inflammation process. As such, one of the biological activity of these inflammation-enabling polypeptide is to bind to other transcription regulators (also referred to as binding partners) as well as to bind to its target nucleotide sequences to modulate gene expression and, ultimately facilitate the inflammatory response. A reduction of the transcription factor activity of an inflammation-enabling polypeptide, either by limiting the IEP to bind to its binding partner and/or to its target nucleic acid sequence, will be considered useful for preventing, treating and/or alleviating the symptoms associated to an inflammatory disorder.

Evaluation of transcription factor activity can be made by determining the ability of the inflammation-enabling to form a multimeric complex with at least one of its binding partners. *In vitro,* the reaction mixture can include, *e.g.* a co-factor, a substrate or other binding partner or potentially interacting fragment thereof. Exemplary binding partners of IRF8 include, but are not limited to, members of the IRF (IRF1, IRF4) or ETS (PU.1). Exemplary binding partners of USP15 include, but are not limited to, the COP9 signalosome and the receptor-activated SMAD transcription factors. Preferably, the binding partner is a direct binding partner. This type of assay can be accomplished, for example, by coupling one of the components (either the inflammation-enabling polypeptide or its binding partner), with a label such that binding of the labeled component to the other can be determined by detecting the labeled compound in a complex. A component can be labeled with ¹²⁵I, ³⁵S, ¹⁴C, or ³H, either directly or indirectly, and the radioisotope detected by direct counting of radioemmission or by scintillation counting. Alternatively, a component can be enzymatically labeled with, for example, horseradish peroxidase, alkaline phosphatase, or luciferase, and the enzymatic label detected by determination of conversion of an appropriate substrate to product. Competition assays can also be used to evaluate a physical interaction between a test compound and a target.

In another assay format, when the inflammation-enabling polypeptide is a transcription factor, its activity can be measured by quantifying (or semi-quantifying) the expression levels of its target genes whose expression is modulated by the presence and activity of the inflammation-enabling polypeptide. Such measure can be made, for example, by PCR (such as qPCR). For example, the target genes of IRF8 include, but are not limited to, the genes listed in Tables 1, 2 and/or 3.

In yet another assay format, when the inflammation-enabling polypeptide is a transcription factor, its activity can be measured by determining the affinity of the transcription to at least one of its nucleic acid recognition motifs. Such measure can be made, for example, through gel-retardation shift assay. For example, the nucleic acid motifs recognized IRF8 include, but are not limited to, GAAAnnGAAA (SEQ ID NO: 1) and GGAAAnnGAAA (SEQ ID NO: 2).

As shown herewith, some inflammation-enabling polypeptides participate in various signaling pathways and interact with other polypeptides (e.g. CCDC88B). An agent capable of limiting the inflammation-enabling polypeptide (such as CCDC88B) to participate in the signaling cascade will be considered useful for preventing, treating and/or alleviating the symptoms associated to an inflammatory conditions. To identify agents that modulate with the interaction between the inflammation-enabling polypeptide and its binding partner(s), for example, a reaction mixture containing the reagent (e.g. the inflammation-enabling polypeptide) and the binding partner is prepared, under conditions and for a time sufficient, to allow the two polypeptides to form complex. In order to test if an agent modulates the interaction between the inflammation-enabling polypeptide and its binding partner, the reaction mixture can be provided in the presence and absence of the test agent. The test agent can be initially included in the reaction mixture, or can be added at a time subsequent to the addition of the target and its cellular or extracellular binding partner. Control reaction mixtures are incubated without the test agent or with vehicle. The formation of any complexes between the target product and the cellular or extracellular binding partner is then detected. The formation of a complex in the reaction mixture containing the test compound, but not in the control reaction, indicates that the test agent facilitates the interaction of the inflammation-enabling polypeptide and the interactive binding partner.

In an embodiment, it is possible to detect the formation of the inflammation-enabling polypeptide complex indirectly by measuring the level of expression of a reporter gene whose expression is modulated by the presence (or absence) of the complex.

In still another assay format, the direct interaction between two molecules (especially two polypeptides) can also be detected. Signal generation or detection systems that may be used in the methods include, but are not limited to, fluorescence methods such as fluorescence resonance energy transfer (FRET), bioluminescence resonance energy transfer (BRET), protein fragment complementation assay (PCA), Biomolecular Interaction Analysis (BIA), fluorescence quenching, fluorescence polarization as well as other chemiluminescence, electrochemiluminescence, Raman, radioactivity, colometric methods, hybridization protection assays and mass spectrometry methods.

An additional example for determining the biological activity of an inflammation-enabling polypeptide is the determination of the ability of the inflammation-enabling polypeptide to add and/or remove ubiquitin moieties to proteins. As shown herein, USP15 (an inflammation-enabling polypeptide) is a cysteine hydrolase which cleaves both the isopeptide bonds between ubiquitin (Ub) units, as well as the peptide bond between Ub (C-terminal Glycine) and the bound protein. When USP15 is the inflammation-enabling polypeptide, agent capable of limiting (even inhibiting) its de-ubiquitinase activity are considered useful for preventing, treating and/or alleviating the symptoms associated to the inflammatory condition. For example, a cell-free assay can be used. This assay can be based on the polypeptide-dependent removal of Ub moieties from a ubiquinated immobilized target (for example a ubiquinated SMAD3), coupled to ELISA-based immunological detection of appearance of free Ub in the reaction. Alternately, if the inflammation-enabling polypeptide shows thiol-dependent hydrolysis of ester, thioester, as well as amide and isopeptide bonds, screens using fluorogenic peptides could also be used as primary assays. Indirectly, the modulation of expression of the inflammation-enabling polypeptide-dependent target (such as, for example, for USP15, TGFβ or BMP or reporter genes (pCAGA12-lux; ID1-BRE-lux)) can be detected.

Cell-free screening assays usually involve preparing a reaction mixture of the reagent and the test compound under conditions and for a time sufficient to allow the two components to interact and bind, thus forming a complex that can be removed and/or detected. In one embodiment, the reagent is anchored onto a solid phase. The reagent-related complexes anchored on the solid phase can be detected at the end of the reaction, *e.g*. the binding reaction. For example, the reagent can be anchored onto a solid surface, and the test compound, (which is not anchored), can be labeled, either directly or indirectly, with detectable labels discussed herein. Examples of such solid phase include microtiter plates, test tubes, array slides, beads and micro-centrifuge tubes. In one embodiment, an inflammation-enabling protein can be provided which adds a domain that allows one or both of the proteins to be bound to a matrix. Following incubation, the vessels are washed to remove any unbound components, the matrix immobilized in the case of beads, complex determined either directly or indirectly, for example, as described above. Alternatively, the complexes can be dissociated from the matrix, and the level of protein binding or activity determined using standard techniques.

In order to conduct such assay, the non-immobilized component (agent) is added to the coated surface containing the anchored component. After the reaction is complete, unreacted components are removed (*e.g*. by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface, e.g. using a labeled antibody specific for the immobilized component (the antibody, in turn, can be directly labeled or indirectly labeled with, e.g. a labeled anti-Ig antibody).

Alternatively, cell free assays can be conducted in a liquid phase. In such an assay, the reaction products are separated from unreacted components, by any of a number of standard techniques, including but not limited to differential centrifugation, chromatography (gel filtration chromatography, ion-exchange chromatography) and/or electrophoresis. Such resins and chromatographic techniques are known to one skilled in the art. Further, fluorescence energy transfer may also be conveniently utilized, as described herein, to detect binding without further purification of the complex from solution.

The assays described herewith can be conducted in a heterogeneous or homogeneous format. Heterogeneous assays involve anchoring either the reagent or the binding partner onto a solid phase, and detecting complexes anchored on the solid phase at the end of the reaction. In homogeneous assays, the entire reaction is carried out in a liquid phase. In either approach, the order of addition of reactants can be varied to obtain different information about the agents being tested. For example, test agents that interfere with the interaction between the reagent and the binding partners, e.g. by competition, can be identified by conducting the reaction in the presence of the test substance. Alternatively, test agents that facilitates preformed complexes, can be tested by adding the test compound to the reaction mixture prior to complexes have been formed. The various formats are briefly described below.

In a heterogeneous assay system, either the reagent or the binding partner, is anchored onto a solid surface (*e.g*. a microtiter plate), while the non-anchored species is labeled, either directly or indirectly. The anchored species can be immobilized by non-covalent or covalent attachments. Alternatively, an immobilized antibody specific for the species to be anchored can be used to anchor the species to the solid surface.

In order to conduct such assay, the partner of the immobilized species is exposed to the coated surface with or without the agent. After the reaction is complete, unreacted components are removed (*e.g.* by washing) and any complexes formed will remain immobilized on the solid surface. Where the non-immobilized species is pre-labeled, the detection of label immobilized on the surface indicates that complexes were formed. Where the non-immobilized species is not pre-labeled, an indirect label can be used to detect complexes anchored on the surface, e.g. using a labeled antibody specific for the initially non-immobilized species (the antibody, in turn, can be directly labeled or indirectly labeled with, *e.g.,* a labeled anti-Ig antibody). Depending upon the order of addition of reaction components, agents that enable complex formation or that promote the stability of preformed complexes can be detected.

Alternatively, the reaction can be conducted in a liquid phase in the presence or absence of the agent, the reaction products separated from unreacted components, and complexes detected, *e.g.* using an immobilized antibody specific for one of the binding components to anchor any complexes formed in solution, and a labeled antibody specific for the other partner to detect anchored complexes. Again, depending upon the order of addition of reactants to the liquid phase, test compounds that enable complex or that promote the stability of preformed complexes can be identified.

In an alternate embodiment, a homogeneous assay can be used. For example, a preformed complex of the reagent and the interactive cellular or extracellular binding partner product is prepared in that either the target products or their binding partners are labeled, but the signal generated by the label is quenched due to complex formation. The addition of agent that favors the formation of the complex will result in the generation of a signal below the control value. In this way, agents that promote binding partner interaction can be identified.

In yet another aspect, the reagent can be used as "bait proteins" in a two-hybrid assay or three-hybrid assay, to identify other proteins, which bind to or interact with binding partners and are involved with the inflammation-enabling polypeptide activity. Such binding partners can be activators or inhibitors of signals or transcriptional control.

In another embodiment, the assay for selecting compounds which interact with the inflammation-enabling polypeptide can be a cell-based assay. Useful assays include assays in which a marker of inflammation is measured. The cell-based assay can include contacting a cell expressing the inflammation-enabling polypeptide with an agent and determining the ability of the test compound to modulate (*e.g.* stimulate or inhibit) the activity of the inflammation-enabling polypeptide, and/or determine the ability of the agent to modulate expression of the inflammation-enabling polypeptide or related proteins in the cell. Cell-based systems can be used to identify compounds that decrease the expression and/or activity and/or effect of the inflammation-enabling polypeptide. Such cells can be recombinant or non-recombinant, such as cell lines that express the inflammation-enabling gene. In some embodiments, the cells can be recombinant or non-recombinant cells which express a binding partner of the inflammation-enabling polypeptide. Exemplary systems include mammalian or yeast cells that express the inflammation-enabling polypeptide (for example from a recombinant nucleic acid). In utilizing such systems, cells are exposed to agents suspected of decreasing expression and/or activity of the inflammation-enabling polypeptide. After exposure, the cells are assayed, for example, for expression or activity of the inflammation-enabling polypeptide. A cell can be from a stable cell line or a primary culture obtained from an organism (for example an organism treated with the agent).

In addition to cell-based and *in vitro* assay systems, non-human organisms, *e.g.* transgenic non-human organisms or a model organism, can also be used. A transgenic organism is one in which a heterologous DNA sequence is chromosomally integrated into the germ cells of the animal. A transgenic organism will also have the transgene integrated into the chromosomes of its somatic cells. Organisms of any species, including, but not limited to: yeast, worms, flies, fish, reptiles, birds, mammals (*e.g.* mice, rats, rabbits, guinea pigs, pigs, micro-pigs, and goats), and non-human primates (*e.g.* baboons, monkeys, chimpanzees) may be used in the methods described herein.

A transgenic cell or animal used in the methods described herein can include a transgene that encodes the inflammation-enabling polypeptide, fragment or variant. The transgene can encode a protein that is normally exogenous to the transgenic cell or animal, including a human protein, e.g., a human inflammation-enabling polypeptide or one of its binding partner. The transgene can be linked to a heterologous or a native promoter. Methods of making transgenic cells and animals are known in the art.

In another assay format, the specific activity of the inflammation-enabling polypeptide, normalized to a standard unit, may be assayed in a cell-free system, a cell line, a cell population or animal model that has been exposed to the agent to be tested and compared to an unexposed control cell-free system, cell line, cell population or animal model. The specific activity of an screened compound can also be assessed using inflammation-enabling polypeptide-deficient systems (*e.g.* where at least one copy of the gene codes for a non-functional inflammation-enabling polypeptide).

In one embodiment, the measuring step includes (or consists in) measuring the test level of the parameter below a control level (usually associated with a lack of prevention, treatment and/or alleviation of symptoms associated with the inflammatory condition). In this embodiment, the presence of the measurement is indicative of the ability of the screened agent to prevent, treat and/or alleviate the symptoms associated with the inflammatory condition. On the other hand, the absence of the measurement is indicative of the lack of ability of the screened agent to prevent, treat and/or alleviate the symptoms associated with the inflammatory condition.

In other embodiments, once the measurement has been made, the value associated thereto can be extracted and compared to a control value. In screening application, the effect of the agent on the inflammation-enabling polypeptide's expression and/or activity is compared to a control value. In an embodiment, the control value is associated with a lack of prevention, treatment and/or alleviation of symptoms of the inflammatory condition and as such, agents useful in the prevention, treatment and/or alleviation of symptoms of the inflammatory condition are capable of decreasing the measured parameter below the control value. In this embodiment, agents which are not considered useful in the prevention, treatment and/or alleviation of symptoms of the inflammatory conditions will present a parameter which is either equal to or higher than the control value.

In another embodiment, the control value is associated with prevention, treatment and/or alleviation of symptoms of the inflammatory condition and as such the measured parameter associated agents useful in the prevention, treatment and/or alleviation of the inflammatory condition equal to or lower than the control value. In such embodiment, agents that are not useful in the prevention, treatment and/or alleviation of symptoms of the inflammatory condition show a test value that is higher than the control value.

In an embodiment, the comparison can be made by an individual. In another embodiment, the comparison can be made in a comparison module. Such comparison module may comprise a processor and a memory card to perform an application. The processor may access the memory to retrieve data. The processor may be any device that can perform operations on data. Examples are a central processing unit (CPU), a front-end processor, a microprocessor, a graphics processing unit (PPU/VPU), a physics processing unit (PPU), a digital signal processor and a network processor. The application is coupled to the processor and configured to determine the effect of the agent on the parameter of the based reagent with respect to the control value. An output of this comparison may be transmitted to a display device. The memory, accessible by the processor, receives and stores data, such as measured parameters of the reagent or any other information generated or used. The memory may be a main memory (such as a high speed Random Access Memory or RAM) or an auxiliary storage unit (such as a hard disk, a floppy disk or a magnetic tape drive). The memory may be any other type of memory (such as a Read-Only Memory or ROM) or optical storage media (such as a videodisc or a compact disc).

Once the comparison between the parameter of the reagent and the control value is made, then it is possible to characterize the agent. This characterization is possible because, as shown herein, the expression and/or activity of the inflammation-enabling polypeptide is downregulated for agents capable of treating, preventing and/or alleviating the symptoms associated an inflammatory disorder.

In an embodiment, the characterization can be made by an individual. In another embodiment, the characterization can be made with a processor and a memory card to perform an application. The processor may access the memory to retrieve data. The processor may be any device that can perform operations on data. Examples are a central processing unit (CPU), a front-end processor, a microprocessor, a graphics processing unit (PPU/VPU), a physics processing unit (PPU), a digital signal processor and a network processor. The application is coupled to the processor and configured to characterize the individual or the agent being screened. An output of this characterization may be transmitted to a display device. The memory, accessible by the processor, receives and stores data, such as measured parameters of the reagent or any other information generated or used. The memory may be a main memory (such as a high speed Random Access Memory or RAM) or an auxiliary storage unit (such as a hard disk, a floppy disk or a magnetic tape drive). The memory may be any other type of memory (such as a Read-Only Memory or ROM) or optical storage media (such as a videodisc or a compact disc).

The screening methods described herein can be used to determine an agent's ability to prevent, treat or alleviate the symptoms of an inflammatory condition. The premise behind this screening method is that the inflammation-enabling polypeptide activity or expression is upregulated during inflammation. As such, by assessing if a downregulation of the inflammation-enabling polypeptide's activity or expression made by the agent, it can be linked to its ability to prevent, treat or alleviate the symptoms of an inflammatory disorder. In these methods, the control value may be the parameter of the reagent in the absence of the agent. In this particular embodiment, the parameter of the reagent can be measured prior to the combination of the agent with the reagent or in at least two replicates of the same reaction vessel where one of the screening system does not comprise the agent. The control value can also be the parameter of the reagent in the presence of a control agent that is known not to limit inflammation or prevent/treat/alleviate the symptoms of an inflammatory disorder. Such control agent may be, for example, a pharmaceutically inert excipient. The control value can also be the parameter of the reagent obtained from a reaction vessel comprising cells or tissues from a healthy subject that is not afflicted by inflammation. The control value can also be a pre-determined value associated with a lack of inflammation (or inflammatory disorder). The ability of the agent is determined based on the comparison of the value of the parameter of the reagent with respect to the control value.

The agent is characterized as being able to prevent, treat or alleviate the symptoms of an inflammatory disorder when the value of the parameter of the reagent is lower than the control value. On the other hand, the agent is characterized as lacking the ability to prevent, treat or alleviate the symptoms of an inflammatory disorder when the measurement of the parameter of the reagent is lower than or equal to the control value.

The present invention also provides screening systems for performing the characterizations and methods described herein. These systems comprise a reaction vessel for placing the agent and the reagent, a processor in a computer system, a memory accessible by the processor and an application coupled to the processor. The application or group of applications is(are) configured for receiving a test value of a parameter of the reagent in the presence of the agent; comparing the test value to a control value and/or characterizing the agent in function of this comparison.

The present invention also provides a software product embodied on a computer readable medium. This software product comprises instructions for characterizing the individual or the agent according to the methods described herein. The software product comprises a receiving module for receiving a test value of a parameter of the reagent in the presence of an agent; a comparison module receiving input from the measuring module for determining if the test value is lower than, equal to or higher than a control value; a characterization module receiving input from the comparison module for performing the characterization based on the comparison.

In an embodiment, an application found in the computer system of the system is used in the comparison module. A measuring module extracts/receives information from the reaction vessel with respect to the parameter of the reagent. The receiving module is coupled to a comparison module which receives the value(s) of the parameter of the reagent and determines if this value is lower than, equal to or higher than a control value. The comparison module can be coupled to a characterization module. In another embodiment, an application found in the computer system of the system is used in the characterization module. The comparison module is coupled to the characterization module which receives the comparison and performs the characterization based on this comparison. In a further embodiment, the receiving module, comparison module and characterization module are organized into a single discrete system. In another embodiment, each module is organized into different discrete system. In still a further embodiment, at least two modules are organized into a single discrete system.

### Heterozygote animals for the inflammation-enabling polypeptide for validating therapeutic compounds

In some instances, it may be necessary to validate the results of an *in vitro* screening (from either a cell-free or a cell-based assay) in an animal model. In other instances, it may also be necessary to perform the initial screening directly in an animal. For those circumstances, an heterozygote animal is herewith provided to provide an animal model of inflammation for determining if a particular agent is useful for the prevention, treatment or alleviations of symptoms associated with an inflammatory condition.

As discussed herein, mouse bearing two copies of a gene coding for an non-functional inflammation-enabling polypeptide are not capable of inducing, mounting a complete inflammatory response when challenged with *P. berghei.* The animals, homozygote at the loci encompassing the gene coding for the inflammation-enabling polypeptide, are expected not to show a differential response to potential anti-inflammatory agents since they cannot induce an inflammatory response.

On the other hand, mouse bearing two copies of a gene coding for a functional inflammation-enabling polypeptide are capable of inducing, mounting and maintaining a complete and full inflammatory response when challenged with *P. berghei.* The animals, homozygote at the loci encompassing the gene coding for the inflammation-enabling polypeptide, are expected to show a very little differential response to potential anti-inflammatory agents because they mount a robust inflammatory response and died very rapidly.

Consequently, the present invention provides an animal heterozygote at the loci encompassing the gene coding for the inflammation-enabling polypeptide. As a first gene copy, a functional inflammation-enabling polypeptide is provided and allows the animal to mount an inflammatory response. As the second gene copy, a non functional inflammation-enabling polypeptide is provided and limits the animal in mounting a robust inflammatory response. The non-functional copy can be, for example, encoding a mutated protein having lost its biological activity, a knock-out gene or a knocked-in gene. Consequently, the animal herewith provided is capable of mounting an inflammatory response, but not a robust one, and are capable of showing a differential response to potential anti-inflammatory agents. The present invention also provides the use of the heterozygous animals for assessing the usefulness of a screened agent for preventing, treating and/or alleviating the symptoms associated to an inflammatory condition. This is defined as a sensitized screen for inhibitors against a given target identified by the method described in this invention.

In order to determine if an agent is capable of treating or alleviating the symptoms associated with an inflammatory condition, an inflammatory response is first induced (by administering a trigger) in the animal and then a screened agent is administered. On the other hand, to determine if an agent is capable of preventing the onset of an inflammatory condition, the screened agent is first administered and then a trigger is provided to the animal. The trigger (*e.g.* an infectious agent such as, for example, *P. berghei*)*,* in the absence of an agent, is capable of inducing an inflammatory response in the animal.

Once the agent and the inflammatory trigger have been administered, then a parameter of an inflammatory response is measured. In embodiments where an infectious agent is administered as the inflammation trigger, and it is known that such trigger ultimately causes death in infected animals, the parameter may be the survival rate or the death rate. Although death is the end point of acute inflammation in the *P. berghei* infection model, other intermediate phenotypes precede death and are predictor of lethality; these include appearance of neurological symptoms such as fever, termors, lethargy, hind limb paralysis and coma. Other inflammation-associated parameters (surrogate markers) can also be measured such as, immune cells number and types, cytokine profiles, chemokine profiles, immunoglobulin profiles, edema, blood-brain-barrier permeability, etc.

In an embodiment, the measure can also include measuring a parameter in function of a control value. For example, when blood-brain-barrier permeability is the parameter that is measured, the measuring step can include measuring the blood-brain-barrier permeability above a control value associated with a non-inflammatory state. The presence of the measure (e.g. because the measure is higher than the control value) is indicative that the agent is useful. On the other hand, the absence of the measure (e.g. because the measure is below than the control value) is indicative that the agent lacks the utility.

Once measured, the test parameter (also referred to as the test level) can be compared to a control and the agent is characterized based on this comparison. Such control can be associated with the prevention, treatment and/or alleviation of the symptoms associated to the inflammatory condition or with the lack of prevention, treatment and/or alleviation of the symptoms associated with the inflammatory condition. The control can be obtained, for example, from animals that have not been treated with the agent or that have been treating with a mock agent that as been shown not to prevent, treat or alleviate the symptoms associated with the inflammatory condition (for example a pharmaceutically acceptable excipient).

For example, when the parameter that is assessed is the survival rate and the control that is used is associated with a lack of prevention, treatment and/or alleviation of the symptoms associated with the inflammatory condition, an agent is considered useful if the survival rate of the treated animals is higher than the survival rate of the non-treated/mock treated animals (*e.g.* control associated with a lack of prevention, treatment and/or alleviation of the symptoms associated with the inflammatory condition). On the other hand, the agent will not be considered useful (or will be considered as lacking the utility) if the survival rate of the treated animals is equal to or higher than the survival rate of the non-treated/mock treated animals (*e.g.* control associated with a lack of prevention, treatment and/or alleviation of the symptoms associated with the inflammatory condition).

In order to conduct use these animal models, it is envisaged that a copy of a single gene of an inflammation-enabling polypeptide (such as, for example, CCDC88B, FOXN1 or USP15) be modified to code for a non-functional polypeptide. However, it is also contemplated that a copy of at least two distinct genes each coding for a different inflammation-enabling polypeptide be modified in the animal (such as for example, a combination of CCDC88B and FOXN1, a combination of CCDC88B and USP15 or a combination of FOXN1 and USP15). It is also completed that a copy at least three distinct genes each coding for a different-inflammation-enabling polypeptide be modified in the animal (such as, for example, a combination of CCDC88B, FOXN1 and USP15). In some instances, it may be advisable to provide further genetic mutations associated with other loci implicated in inflammation in the animal. For example, it is possible to obtain an animal additionally bearing a copy of a non-functional gene coding for at least one of JAK3, THEMIS, IRGM1, IRF1 and IRF8, at least two of JAK3, THEMIS, IRGM1, IRF1 and IRF8, at least three of JAK3, THEMIS, IRGM1, IRF1 and IRF8, at least four of JAK3, THEMIS, IRGM1, IRF1 and IRF8 or all five JAK3, THEMIS, IRGM1, IRF1 and IRF8.

Methods for providing a non-functional gene copy include, but are not limited to, chemical mutagensis (*e.g*. ENU-directed mutagenesis) and transgenesis. The heterozygote animal can also be obtained by breeding an animal homozygote at the loci encompassing the gene coding for a non-functional inflammation-enabling polypeptide with an animal homozygote at the loci encompassing the gene coding for a functional inflammation-enabling polypeptide.

Various animals can be obtained and used in this method. In an embodiment, the animal is a non-human mammal, such as a mammal. In some embodiments, the animal is rodent (a mouse for example).

### Predictive methods using the inflammation-enabling polypeptide targets

The predictive methods described herein are designed to capture the relationship between the biological activity of the inflammation-enabling polypeptides and inflammatory conditions to generate valuable information about the therapeutic agent that is being administered to an individual or the individual that is being characterized. As shown herein, the biological activity of the inflammation-enabling polypeptides is positively correlated with the ability of the individual to induce, mount and/or sustain an inflammatory response. When the inflammation-enabling polypeptide(s) is(are) expressed and functional in the individual (e.g. when the inflammation-enabling polypeptides show biological activity), it indicates that such individual is capable of developing or maintaining an inflammatory response. Alternatively, when the inflammation-enabling polypeptide(s) is(are) expressed at a lower level, are not expressed or are not functional in an individual (e.g. when the inflammation-enabling polypeptides show reduced or non-existent biological activity), it indicates that such individual is not capable of developing or maintaining a full and complete inflammatory response.

This correlation between the biological activity of the inflammation-enabling polypeptide and inflammation also provides a basis for determining if a therapeutic regimen is successful in the individual, either for preventing, treating and/or alleviating the symptoms associated to the inflammatory condition. If the biological activity of the inflammation-enabling polypeptide(s) is(are) reduced by the therapeutic regimen, it is assumed that the regimen is successful in preventing, treating and/or alleviating the symptoms associated with the inflammatory response in the individual. Alternatively, if the biological activity of the inflammation-enabling polypeptide(s) is(are) not reduced (e.g. remains the same or increases) upon the administration of the therapeutic regimen, it is assumed that the regimen is not successful for preventing, treating and/or alleviating the inflammatory condition in the individual.

This correlation between the biological activity of the inflammation-enabling polypeptide and inflammation further provides a basis for assessing the risk of an individual of developing or being afflicted with an inflammatory condition. If the biological activity of the inflammation-enabling polypeptide(s) is(are) higher in the screened individual with respect to a control individual (for example a healthy individual known not to be at risk of developing or being afflicted with an inflammatory condition), then it is assumed that the screen individual is at risk of developing (predisposed) or being afflicted with an inflammatory condition. Alternatively, if the biological activity of the inflammation-enabling polypeptide(s) in the screened individual is(are) similar or lower than the biological activity observed in a control individual (for example a healthy individual known not to be at risk of developing or being afflicted with an inflammatory condition), then it is assumed that the screened individual is not at risk (predisposed) of developing or being afflicted with an inflammatory condition.

In these predictive applications, a biological sample of an individual is obtained and can placed in a reaction vessel. The biological sample comprises an analyte (also referred to as a reagent) associated with an inflammation-enabling polypeptide. In the assays, the reaction vessel can be any type of container that can accommodate the determination of the parameter of the analyte/reagent.

Once the biological sample has been provided, one of the parameters of the analyte/reagent can be measured. This measure may be made directly in the reaction vessel (by using a probe) or on a sample of such reaction vessel. As indicated above, in the screening section, the measure can be made either at the DNA level, the RNA level and/or the polypeptide level. As also indicated above in the screening section, the analyte/reagent can either be the inflammation-enabling polypeptide itself (corresponding transcript and/or gene) and/or for a polypeptide associated with the biological activity inflammation-enabling polypeptide (corresponding transcript and/or gene). The measure can concern a single inflammation-enabling polypeptide or a combination of inflammation-enabling polypeptides (for example two or three inflammation-enabling polypeptides).

In an embodiment, the measuring step can also comprise measuring the parameter in function of a control value. For example, when IEP's amount is the parameter that is measured, the measuring step can include measuring the IEP's amount below a control value associated with a non-inflammatory state. The presence of the measure (e.g. because the measure is lower than the control value) is indicative that the agent is useful in the treated individual. On the other hand, the absence of the measure (*e.g.* because the measure is higher than the control value) is indicative that the agent lacks the utility in the treated individual.

The methodology described in the "Screening methods for therapeutic agents" section above for measuring the reagent and obtaining a test level of the parameter of the biological activity of the IEP can be used in the predictive methods described herein.

Once the measure of the parameter has been made, a comparison to a control level is done. For example, if the method is for determining the usefulness of a therapeutic agent in an individual and the analyte/reagent that is being measure is the mRNA expression profile (identity and/or amount of transcripts of a plurality of genes whose expression is modulated by an inflammation-enabling polypeptide, for example IRF8), the comparison step can comprise determining the identity of mRNA transcripts and/or the amount of each mRNA transcripts associated with a plurality of genes whose expression is modulated by an inflammation-enabling polypeptide. The mRNA profile obtained (either the genetic identity of the transcripts and/or the amount of the transcripts) is compared either to a healthy mRNA profile derived from a healthy individual (a control associated with a lack of an inflammatory condition) and/or to a disease mRNA profile derived from an afflicted and untreated individual (a control associated with the presence of an inflammatory condition and consequently the lack of prevention, treatment and/or alleviation of symptoms) to determine to which mRNA profile the obtained mRNA profile is more similar. If the obtained mRNA profile is more similar to the healthy mRNA profile than to the disease mRNA profile, then it can be assumed that the therapeutic agent is useful in the prevention, treatment and/or alleviation of symptoms associated to an inflammatory condition. Alternatively, if the obtained mRNA profile is more similar to the disease mRNA profile than to the healthy mRNA profile, then it can be assumed that the therapeutic agent is not useful in the prevention, treatment and/or alleviation of symptoms associated to an inflammatory condition.

In another example, if the method is for determining the predisposition or affliction of in an individual to the inflammatory condition and the analyte/reagent that is being measure is the biological activity of the inflammation-enabling polypeptide (for example USP15), the comparison step can comprise comparing the test level of biological activity in the screened individual identity to a control level of the inflammation-enabling polypeptide. The biological activity measured can be compared either to a healthy control level that can be derived from a healthy individual (a control associated with a lack of an inflammatory condition) and/or to a disease control level derived from an afflicted and untreated individual (a control associated with the presence of an inflammatory condition and consequently the lack of prevention, treatment and/or alleviation of symptoms) to determine to which control level the measured biological activity is more similar. If the measured test level is more similar (e.g. closer) to the healthy level than to the disease level, then it can be assumed that the individual is either not predisposed (or at least as predisposed as the healthy individual) or not afflicted with the inflammation-enabling polypeptide. Alternatively, if the measured is more similar (e.g. closer) to the disease control level than to the healthy control level, then it can be assumed that the individual is either predisposed or afflicted by the inflammatory condition.

In an embodiment, the comparison can be made by an individual. In another embodiment, the comparison can be made in a comparison module. Such comparison module may comprise a processor and a memory card to perform an application. The processor may access the memory to retrieve data. The processor may be any device that can perform operations on data. Examples are a central processing unit (CPU), a front-end processor, a microprocessor, a graphics processing unit (PPU/VPU), a physics processing unit (PPU), a digital signal processor and a network processor. The application is coupled to the processor and configured to compare the test level to a control level. An output of this comparison may be transmitted to a display device. The memory, accessible by the processor, receives and stores data or any other information generated or used. The memory may be a main memory (such as a high speed Random Access Memory or RAM) or an auxiliary storage unit (such as a hard disk, a floppy disk or a magnetic tape drive). The memory may be any other type of memory (such as a Read-Only Memory or ROM) or optical storage media (such as a videodisc or a compact disc).

Once the comparison is made, then it is possible to characterize the therapeutic agent's usefulness or the individual's predisposition. This characterization is possible because, as shown herein, the biological activity of at least one inflammation-enabling polypeptide is associated with the onset and maintenance of inflammation.

In an embodiment, the characterization can be made by an individual. In another embodiment, the characterization can be made with a processor and a memory card to perform an application. The processor may access the memory to retrieve data. The processor may be any device that can perform operations on data. Examples are a central processing unit (CPU), a front-end processor, a microprocessor, a graphics processing unit (PPU/VPU), a physics processing unit (PPU), a digital signal processor and a network processor. The application is coupled to the processor and configured to characterize the individual being tested. An output of this characterization may be transmitted to a display device. The memory, accessible by the processor, receives and stores data or any other information generated or used. The memory may be a main memory (such as a high speed Random Access Memory or RAM) or an auxiliary storage unit (such as a hard disk, a floppy disk or a magnetic tape drive). The memory may be any other type of memory (such as a Read-Only Memory or ROM) or optical storage media (such as a videodisc or a compact disc).

The predictive methods described herein are useful for determining if a therapeutic regimen that is being administered is useful or not in preventing, treating and/or alleviating the symptoms associated with an inflammatory condition. If a therapeutic regimen is useful, then it is assumed that the biological activity of at least one inflammation-enabling polypeptide will be reduced in the individual upon the administration of at least one dose of therapeutic agent. In some instances, it may be necessary to administer the therapeutic agent more than once to observe some therapeutic benefit(s). As such, it is possible to repeat the method in time to determine if the therapeutic agent (i) continues to provide therapeutic benefit or (ii) can provide therapeutic benefits when administered more than once. If warranted, it is also possible to perform this method before and after the intake of the therapeutic agent.

The predictive methods described herein are useful for determining if an individual is predisposed to/afflicted by an inflammatory condition. Such methods can be used in conjunction with other methods to provide a diagnosis of an inflammatory condition in the individual. If the individual is predisposed/afflicted, then it is assumed that the biological activity of at least one inflammation-enabling polypeptide will be higher than a control healthy individual. In some instances, it may be necessary to perform the method at more than once to determine if the predisposition further increases or the inflammatory condition continue to worsen.

The predictive methods presented herein can also be useful in classifying individuals already diagnosed with an inflammatory condition based on the level of activity of the inflammation-enabling polypeptides. The predictive methods presented herein can also be useful in determining the re-occurrence of an inflammatory condition in individuals previously diagnosed (and, optionally treated) with the condition.

The present invention also provides predictive systems for performing the characterizations and methods described herein. These systems comprise a reaction vessel for placing the biological sample, a processor in a computer system, a memory accessible by the processor and an application coupled to the processor. The application or group of applications is(are) configured for receiving a test level of parameter of the reagent; comparing the test level to a control level and/or characterizing the therapeutic agent/individual in function of this comparison.

The present invention also provides a software product embodied on a computer readable medium. This software product comprises instructions for characterizing the individual according to the methods described herein. The software product comprises a receiving module for receiving a test level from a parameter of a reagent in a biological sample; a comparison module receiving input from the measuring module for comparing the test level to a control level; a characterization module receiving input from the comparison module for performing the characterization based on the comparison.

In an embodiment, an application found in the computer system of the system is used in the comparison module. A measuring module extracts/receives information from the reaction vessel with respect to the test level. The receiving module is coupled to a comparison module which receives the value(s) of the test level and determines if this value is identical or different from the control level. The comparison module can be coupled to a characterization module.

In another embodiment, an application found in the computer system of the system is used in the characterization module. The comparison module is coupled to the characterization module which receives the comparison and performs the characterization based on this comparison.

In a further embodiment, the receiving module, comparison module and characterization module are organized into a single discrete system. In another embodiment, each module is organized into different discrete system. In still a further embodiment, at least two modules are organized into a single discrete system.

### Research tools

The present invention also provides research tools based either on the altered (e.g. reduced) expression of inflammatory enabling polypeptides (IEP) or the expression of a mutant IEP.

One of the research tools that can be useful for the characterization of inflammation conditions, are mutant polypeptides of IEP. Mutant IEPs, when expressed in a subject, have the ability to prevent the onset and/or maintenance of an inflammatory condition in the subject. Mutant IEPs include truncated versions of IEP as well mutated versions of IEP having the ability to prevent the onset and/or maintenance of an inflammatory condition in the subject. A truncated version of an IEP is a polypeptide which is at least one amino acid shorter than the wild-type IEP. A mutated version of an IEP is a polypeptide which has at least one amino acid substitution or addition when compared to the wild-type IEP. A polypeptide or fragment thereof is "substantially homologous" or "substantially identical" to another if, when optimally aligned (with appropriate insertions and/or deletions) with the other polypeptide, there is nucleotide sequence identity in at least 60% of the nucleotide bases, usually at least 70%, more usually at least 80%, preferably at least 90%, and more preferably at least 95-98% of the amino acid residues. The length of homology or identity comparison, as described, may be over longer stretches, and in certain embodiments will often be over a stretch of at least 5 amino acids, at least 14 amino acids, at least 20 amino acids, more usually at least 24 amino acids, typically at least 28 amino acids, more typically at least 32 amino acids, and preferably at least 36 or more amino acids. In an embodiment, the mutant IEP is a truncated or mutated version of CCDC88B or USP15. One examplary mutant IEP is the amino acid sequence shown in SEQ ID NO: 4.

Another research tool that can be used is a nucleic acid vector encoding the isolated IEP or mutant IEP described herein. As used herein, the term "vector" to expression vectors (derived, for example, from retroviruses, adenovirus, herpes or vaccinia viruses, bacterial or fungal plasmids) as well as integrative vectors (designed to, for example, specifically disrupt the appropriate expression of an IEP).

Cells (in some embodiments isolated cells) can also be used in the methods described herein. Some cells can be designed to be heterozygous for a gene encoding an inflammatory enabling polypeptide (IEP). In such cells, two different gene copy at the same loci are found. For example, the cell can bear a first gene copy encoding IEP and a second gene copy encoding a mutant of the IEP. Other cells can be designed to be homozygous for a gene encoding a mutant inflammatory enabling polypeptide (IEP). Combinations of heterozygous and homozygous cells are also contemplated. In some embodiments, the cells are going to be transgenic cell and can even bear the vectors described herein.

Animals (in some embodiments cells and tissues isolated therefrom) can also be used in the methods described herein. Some animals can be designed to be heterozygous for a gene encoding an inflammatory enabling polypeptide (IEP). In such animals, two different gene copy at the same loci are found. For example, the animal can bear a first gene copy encoding IEP and a second gene copy encoding a mutant of the IEP. Other animals can be designed to be homozygous for a gene encoding a mutant inflammatory enabling polypeptide (IEP). Combinations of heterozygous and homozygous animals are also contemplated. In some embodiments, the animals are going to be transgenic ones and can even bear the vectors described herein.

### Therapeutic method

The present invention does hereby provide that the biological activity of the inflammation-enabling polypeptide(s) is increased with inflammation and that, conversely a reduction in the biological activity of the inflammation-enabling polypeptide(s) would be beneficial for preventing, treating and/or alleviating the symptoms associated to an inflammatory disorder. Consequently, it is expected that the reduction in expression of at least one (or two or three) inflammation-enabling polypeptide would be beneficial for reducing the level or length of a pathologic inflammation response. The present application thus provides a method for preventing, treating and/or alleviating the symptoms associated to an inflammatory conditions based on the inhibition of the biological activity of at least one IEP as well as the use of IEP's inhibitors for the prevention, treatment and/or alleviation of symptoms associated with an inflammatory condition.

The agents that can be administered for this purpose include, but are not limited to, small molecules, peptides, antibodies, nucleic acids, analogs thereof, multimers thereof, fragments thereof, derivatives thereof and combinations thereof.

In order to limit and even shut down the expression of the inflammation-enabling polypeptide, it is possible to use a nucletotide-based agent such as, an antisense nucleic acid or oligonucleotide wholly or partially complementary to, and can hybridize with, a target nucleic acids encoding the inflammation-enabling polypeptide (either DNA or RNA). For example, an antisense nucleic acid or oligonucleotide can be complementary to 5' or 3' untranslated regions, or can overlap the translation initiation codon (5' untranslated and translated regions) of at least one nucleic acid molecule encoding for an inflammation-enabling polypeptide. As non-limiting examples, antisense oligonucleotides may be targeted to hybridize to the following regions: mRNA cap region; translation initiation site; translational termination site; transcription initiation site; transcription termination site; polyadenylation signal; 3' untranslated region; 5' untranslated region; 5' coding region; mid coding region; 3' coding region; DNA replication initiation and elongation sites. Preferably, the complementary oligonucleotide is designed to hybridize to the most unique 5' sequence of a nucleic acid molecule encoding for an inflammation-enabling polypeptide, including any of about 15-35 nucleotides spanning the 5' coding sequence.

In another embodiment, oligonucleotides can be constructed which will bind to duplex nucleic acid (*i.e.* DNA:DNA or DNA:RNA), to form a stable triple helix containing or triplex nucleic acid. Such triplex oligonucleotides can inhibit transcription and/or expression of a nucleic acid encoding an inflammation-enabling polypeptide. Triplex oligonucleotides are constructed using the base-pairing rules of triple helix formation.

In yet a further embodiment, oligonucleotides can be used in the present method. In the context of this application, the term "oligonucleotide" refers to naturally-occurring species or synthetic species formed from naturally-occurring subunits or their close homologs. The term may also refer to moieties that function similarly to oligonucleotides, but have non-naturally-occurring portions. Thus, oligonucleotides may have altered sugar moieties or inter-sugar linkages. Exemplary among these are phosphorothioate and other sulfur containing species which are known in the art. In preferred embodiments, at least one of the phosphodiester bonds of the oligonucleotide has been substituted with a structure that functions to enhance the ability of the compositions to penetrate into the region of cells where the RNA whose activity is to be modulated is located. It is preferred that such substitutions comprise phosphorothioate bonds, methyl phosphonate bonds, or short chain alkyl or cycloalkyl structures. In accordance with other preferred embodiments, the phosphodiester bonds are substituted with structures which are, at once, substantially non-ionic and non-chiral, or with structures which are chiral and enantiomerically specific. Persons of ordinary skill in the art will be able to select other linkages for use in the practice of the invention. Oligonucleotides may also include species that include at least some modified base forms. Thus, purines and pyrimidines other than those normally found in nature may be so employed. Similarly, modifications on the furanosyl portions of the nucleotide subunits may also be affected, as long as the essential tenets of this invention are adhered to. Examples of such modifications are 2'-O-alkyl- and 2'-halogen-substituted nucleotides. Some non-limiting examples of modifications at the 2' position of sugar moieties which are useful in the present invention include OH, SH, SCH₃, F, OCH₃, OCN, O(CH₂), NH₂ and O(CH₂)ₙCH₃, where n is from 1 to about 10. Such oligonucleotides are functionally interchangeable with natural oligonucleotides or synthesized oligonucleotides, which have one or more differences from the natural structure. All such analogs are comprehended herewith so long as they function effectively to hybridize with at least one nucleic acid molecule encoding an inflammation-enabling polypeptide to inhibit the function thereof.

Alternatively, expression vectors derived from retroviruses, adenovirus, herpes or vaccinia viruses or from various bacterial plasmids may be used for delivery of nucleotide sequences to the targeted organ, tissue or cell population. Methods which are well known to those skilled in the art can be used to construct recombinant vectors which will express nucleic acid sequence that is complementary to the nucleic acid sequence encoding an inflammation-enabling polypeptide.

RNA interference (RNAi) is a post-transcriptional gene silencing process that is induced by a miRNA or a dsRNA (a small interfering RNA; siRNA), and has been used to modulate gene expression. RNAi can be used in the therapeutic method describe herewith. Generally, RNAi is being performed by contacting cells with a double stranded siRNA ou a small hairpin RNA (shRNA). However, manipulation of RNA outside of cells is tedious due to the sensitivity of RNA to degradation. It is thus also encompassed herein a deoxyribonucleic acid (DNA) compositions encoding small interfering RNA (siRNA) molecules, or intermediate siRNA molecules (such as shRNA), comprising one strand of an siRNA be used. Accordingly, the present application provides an isolated DNA molecule, which includes an expressible template nucleotide sequence of at least about 16 nucleotides encoding an intermediate siRNA, which, when a component of an siRNA, mediates RNA interference (RNAi) of a target RNA. The present application further concerns the use of RNA interference (RNAi) to modulate the expression of nucleic acid molecules encoding the inflammation-enabling polypeptide in target cells. While the therapeutic applications are not limited to a particular mode of action, RNAi may involve degradation of messenger RNA (e.g. mRNA of genes of inflammation-enabling polypeptide) by an RNA induced silencing complex (RISC), preventing translation of the transcribed targeted mRNA. Alternatively, it may also involve methylation of genomic DNA, which shuts down transcription of a targeted gene. The suppression of gene expression caused by RNAi may be transient or it may be more stable, even permanent.

"Small interfering RNA" or siRNA can also be used in the present methods. It o refers to any nucleic acid molecule capable of mediating RNA interference "RNAi" or gene silencing. For example, siRNA can be double stranded RNA molecules from about 10 to about 30 nucleotides long that are named for their ability to specifically interfere with protein expression (e.g. the inflammation-enabling polypeptide expression). In one embodiment, siRNAs of the present invention are 12-28 nucleotides long, more preferably 15-25 nucleotides long, even more preferably 19-23 nucleotides long and most preferably 21-23 nucleotides long. Therefore preferred siRNA are 12, 13, 14, 15, 16, 17, 18, 19, 20, 21, 22, 23, 24, 25, 26, 27, 28 nucleotides in length. As used herein, siRNA molecules need not to be limited to those molecules containing only RNA, but further encompass chemically modified nucleotides and non-nucleotides. siRNA can be designed to decrease expression of inflammation-enabling polypeptide in a target cell by RNA interference. siRNAs can comprise a sense region and an antisense region wherein the antisense region comprises a sequence complementary to an mRNA sequence for a nucleic acid molecule encoding inflammation-enabling polypeptide and the sense region comprises a sequence complementary to the antisense sequence of the gene's mRNA. An siRNA molecule can be assembled from two nucleic acid fragments wherein one fragment comprises the sense region and the second fragment comprises the antisense region of siRNA molecule. The sense region and antisense region can also be covalently connected via a linker molecule. The linker molecule can be a polynucleotide linker or a non-polynucleotide linker.

A ribozyme (from ribonucleic acid enzyme, also called RNA enzyme or catalytic RNA) is an RNA molecule that catalyzes a chemical reaction. Some ribozymes may play an important role as therapeutic agents, as enzymes which target defined RNA sequences, as biosensors, and for applications in functional genomics and gene discovery. Ribozymes can be genetically engineered to specifically cleave a transcript of a gene from a nucleic acid molecule encoding inflammation-enabling polypeptide whose expression is upregulated with the disease.

The delivery of the gene or genetic material into the cell is the first step in gene therapy treatment of any disorder. A large number of delivery methods are well known to those of skill in the art. Preferably, the nucleic acids are administered for *in vivo* or *ex vivo* gene therapy uses. Non-viral vector delivery systems include DNA plasmids, naked nucleic acid, and nucleic acid complexed with a delivery vehicle such as a liposome. Viral vector delivery systems include DNA and RNA viruses, which have either episomal or integrated genomes after delivery to the cell.

The use of RNA or DNA based viral systems for the delivery of nucleic acids take advantage of highly evolved processes for targeting a virus to specific cells in the body and trafficking the viral payload to the nucleus. Viral vectors can be administered directly to patients (*in vivo*) or they can be used to treat cells in vitro and the modified cells then administered to patients (*ex vivo*). Conventional viral based systems for the delivery of nucleic acids could include retroviral, lentiviral, adenoviral, adeno-associated and herpes simplex virus vectors for gene transfer. Viral vectors are currently the most efficient and versatile method of gene transfer in target cells and tissues. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

In applications where transient expression of the nucleic acid is preferred, adenoviral based systems are typically used. Adenoviral based vectors are capable of very high transduction efficiency in many cell types and do not require cell division. With such vectors, high titer and levels of expression have been obtained. This vector can be produced in large quantities in a relatively simple system. Adeno-associated virus ("AAV") vectors are also used to transduce cells with target nucleic acids, e.g., in the in vitro production of nucleic acids and peptides, and for in vivo and ex vivo gene therapy procedures.

Recombinant adeno-associated virus vectors (rAAV) are a promising alternative gene delivery systems based on the defective and nonpathogenic parvovirus adeno-associated type 2 virus. All vectors are derived from a plasmid that retains only the AAV 145 bp inverted terminal repeats flanking the transgene expression cassette. Efficient gene transfer and stable transgene delivery due to integration into the genomes of the transduced cell are key features for this vector system.

Replication-deficient recombinant adenoviral vectors (Ad) are predominantly used in transient expression gene therapy; because they can be produced at high titer and they readily infect a number of different cell types. Most adenovirus vectors are engineered such that a transgene replaces the Ad E1a, E1b, and E3 genes; subsequently the replication defective vector is propagated in human 293 cells that supply the deleted gene function in trans. Ad vectors can transduce multiple types of tissues in vivo, including non-dividing, differentiated cells such as those found in the liver, kidney and muscle tissues. Conventional Ad vectors have a large carrying capacity.

In many gene therapy applications, it is desirable that the gene therapy vector be delivered with a high degree of specificity to a particular tissue type, such as for example, the myeloid or lymphoid cells. A viral vector is typically modified to have specificity for a given cell type by expressing a ligand as a fusion protein with a viral coat protein on the viruses outer surface. The ligand is chosen to have affinity for a receptor known to be present on the cell type of interest.

Gene therapy vectors can be delivered *in vivo* by administration to an individual subject, typically by systemic administration (*e.g*. intravenous, intratumoral, intraperitoneal, intramuscular, subdermal, or intracranial infusion) or topical application. Alternatively, vectors can be delivered to cells *ex vivo,* such as cells explanted from an individual patient (*e.g*. lymphocytes, bone marrow aspirates, and tissue biopsy) or universal donor hematopoietic stem cells, followed by re-implantation of the cells into the subject, usually after selection for cells which have incorporated the vector.

In one embodiment, stem cells are used in *ex vivo* procedures for cell transfection and gene therapy. The advantage to using stem cells is that they can be differentiated into other cell types *in vitro,* or can be introduced into a mammal (such as the donor of the cells) where they will engraft at an appropriate location (such as in the bone marrow). Methods for differentiating CD34+ cells in vitro into clinically important immune cell types using cytokines such as for example GM-CSF, IFN-γ and TNF-α are known.

Stem cells can be isolated for transduction and differentiation using known methods. For example, stem cells can be isolated from bone marrow cells by panning the bone marrow cells with antibodies which bind unwanted cells, such as CD4+ and CD8+ (T cells), CD45+ (panB cells), GR-1 (granulocytes), and lad (differentiated antigen presenting cells).

In another embodiment, the therapeutic agent can be an antibody (or a variant thereof) capable of limiting or even inhibiting the biological activity of the inflammation-enabling protein. Such antibodies are also known in the art as neutralizing antibodies.

Naturally occurring immunoglobulins have a common core structure in which two identical light chains (about 24 kD) and two identical heavy chains (about 55 or 70 kD) form a tetramer. The amino-terminal portion of each chain is known as the variable (V) region and can be distinguished from the more conserved constant (C) regions of the remainder of each chain. Within the variable region of the light chain is a C-terminal portion known as the J region. Within the variable region of the heavy chain, there is a D region in addition to the J region. Most of the amino acid sequence variation in immunoglobulins is confined to three separate locations in the V regions known as hypervariable regions or complementarity determining regions (CDRs) which are directly involved in antigen binding. Proceeding from the amino-terminus, these regions are designated CDR1, CDR2 and CDR3, respectively. The CDRs are held in place by more conserved framework regions (FRs). Proceeding from the amino-terminus, these regions are designated FR1, FR2, FR3, and FR4, respectively.

Antibody derivatives include, but are not limited to, chimeric and humanized antibodies. As used herein, the term "chimeric" antibodies refers to an antibody molecule derived from antibodies from two different species. A humanized antibody is a type of chimeric antibody. As used herein, the term "humanized antibody" refers to an immunoglobulin that comprises both a region derived from a human antibody or immunoglobulin and a region derived from a non-human antibody or immunoglobulin. The action of humanizing an antibody consists in substituting a portion of a non-human antibody with a corresponding portion of a human antibody. For example, a humanized antibody as used herein could comprise a non-human region variable region (such as a region derived from a murine antibody) capable of specifically recognizing the inflammation-enabling polypeptide and a human constant region derived from a human antibody. In another example, the humanized immunoglobulin can comprise a heavy chain and a light chain, wherein the light chain comprises a complementarity determining region derived from an antibody of non-human origin which binds (specifically) the inflammation-enabling polypeptide and a framework region derived from a light chain of human origin, and the heavy chain comprises a complementarity determining region derived from an antibody of non-human origin which binds (specifically) the inflammation-enabling polypeptide and a framework region derived from a heavy chain of human origin.

In an embodiment, the antibody can be a monoclonal antibody (e.g. derived from a single antibody-producing clone). In some embodiment, the present application also provides fragments of the monoclonal antibodies. As used herein, a "fragment" of an antibody (e.g. a monoclonal antibody) is a portion of an antibody that is capable of specifically recognizing the same epitope as the full version of the antibody. In the present patent application, antibody fragments are capable of specifically recognizing the inflammation-enabling polypeptide. Antibody fragments include, but are not limited to, the antibody light chain, single chain antibodies, Fv, Fab, Fab' and F(ab')₂ fragments. Such fragments can be produced by enzymatic cleavage or by recombinant techniques. For instance, papain or pepsin cleavage can be used to generate Fab or F(ab')₂ fragments, respectively. Antibodies can also be produced in a variety of truncated forms using antibody genes in which one or more stop codons have been introduced upstream of the natural stop site. For example, a chimeric gene encoding the heavy chain of an F(ab')₂ fragment can be designed to include DNA sequences encoding the CH1 domain and hinge region of the heavy chain. Antibody fragments can also be humanized. For example, a humanized light chain comprising a light chain CDR (*i.e.* one or more CDRs) of non-human origin and a human light chain framework region. In another example, a humanized immunoglobulin heavy chain can comprise a heavy chain CDR (*i.e.* one or more CDRs) of non-human origin and a human heavy chain framework region. The CDRs can be derived from a non-human immunoglobulin.

In other embodiment, a polyclonal antibody composition can be used. The polyclonal antibody composition can be used directly as it is generated by the method, or can be further processed prior to its use. For example, the polyclonal antibody composition can be further fragmented, humanized, linked to another agent, etc.

Administration is by any of the routes normally used for introducing the therapeutic agent into ultimate contact with blood or tissue cells. The nucleic acids molecules described herein can be administered in any suitable manner, preferably with the pharmaceutically acceptable carriers or excipients. The terms "pharmaceutically acceptable carrier", "excipients" and "adjuvant" and "physiologically acceptable vehicle" and the like are to be understood as referring to an acceptable carrier or adjuvant that may be administered to a patient, together with a compound of this invention, and which does not destroy the pharmacological activity thereof. Further, as used herein "pharmaceutically acceptable carrier" or "pharmaceutical carrier" are known in the art and include, but are not limited to, 0.01-0.1 M and preferably 0.05 M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, collating agents, inert gases and the like.

As used herein, "pharmaceutical composition" means therapeutically effective amounts (dose) of the agent together with pharmaceutically acceptable diluents, preservatives, solubilizers, emulsifiers, adjuvants and/or carriers. A "therapeutically effective amount" as used herein refers to that amount which provides a therapeutic effect for a given condition and administration regimen. Such compositions are liquids or lyophilized or otherwise dried formulations and include diluents of various buffer content (*e.g.* Tris-HCI, acetate, phosphate), pH and ionic strength, additives such as albumin or gelatin to prevent absorption to surfaces, and detergents (*e.g.* Tween 20™, Tween 80™, Pluronic F68™, bile acid salts). The pharmaceutical composition can comprise pharmaceutically acceptable solubilizing agents (*e.g.* glycerol, polyethylene glycerol), anti-oxidants (e.g., ascorbic acid, sodium metabisulfite), preservatives (*e.g.* thimerosal, benzyl alcohol, parabens), bulking substances or tonicity modifiers (*e.g.* lactose, mannitol), covalent attachment of polymers such as polyethylene glycol to the protein, complexation with metal ions, or incorporation of the material into or onto particulate preparations of polymeric compounds such as polylactic acid, polyglycolic acid, hydrogels, etc, or onto liposomes, microemulsions, micelles, unilamellar or multilamellar vesicles, erythrocyte ghosts, or spheroplasts. Such compositions will influence the physical state, solubility, stability, rate of in vivo release, and rate of in vivo clearance. Controlled or sustained release compositions include formulation in lipophilic depots (*e.g.* fatty acids, waxes, oils). Also comprehended by the invention are particulate compositions coated with polymers (*e.g.* poloxamers or poloxamines).

Suitable methods of administering the therapeutic agents are available and well known to those of skill in the art, and, although more than one route can be used to administer a particular composition, a particular route can often provide a more immediate and more effective reaction than another route. The preventive or therapeutic agents of the present invention may be administered, either orally or parenterally, systemically or locally. For example, intravenous injection such as drip infusion, intramuscular injection, intraperitoneal injection, subcutaneous injection, suppositories, intestinal lavage, oral enteric coated tablets, and the like can be selected, and the method of administration may be chosen, as appropriate, depending on the age and the conditions of the patient. The effective dosage is chosen from the range of 0.01 mg to 100 mg per kg of body weight per administration. Alternatively, the dosage in the range of 1 to 1000 mg, preferably 5 to 50 mg per patient may be chosen.

### Inflammation-Enabling Polypeptides

The inflammation-enabling polypeptide sequences (and nucleic acids associated thereto) encompass host polypeptides (also refer to as targets) which are herein shown to enable the induction and/or persistence of a pathological inflammatory response. These polypeptides are considered to be involved in any pathological inflammation response, regardless of the etiology of the disease. In an embodiment, the IEP include CCDC88B, FOXN1 and USP15. In yet another embodiment the IEP include CCDC88, FOXN1, USP15, THEMIS and IRF8. In another embodiment, the IEP include, CCDC88B, FOXN1, USP15, THEMIS, IRF1, IRGM1 and IRF8. In still another embodiment, JAK3 is not considered to be an IEP. For nucleic acid molecules, this encompasses sequences that are identical or complementary to the coding sequences of the IEP, as well as sequence-conservative and function-conservative variants thereof. For IEP, this encompasses sequences that are identical to the polypeptide, as well as function-conservative variants thereof. Included are the alleles of naturally-occurring polymorphisms of the IEP-encoding genes which do not cause altered expression of their respective genes and alleles that do not cause altered protein levels, activity or stability.

Function-conservative variants are those in which a change in one or more nucleotides in a given codon position results in a polypeptide sequence in which a given amino acid residue in the polypeptide has been replaced by a conservative amino acid substitution. Function-conservative variants also include analogs of a given polypeptide and any polypeptides that have the ability to elicit antibodies specific to a designated polypeptide.

Sequence-conservative variants consists of variants in which a change of one or more nucleotides in a given codon position results in no alteration in the amino acid encoded at that position (*e.g.*, silent mutation).

A nucleic acid or fragment thereof is "substantially homologous" or "substantially identical" to another if, when optimally aligned (with appropriate nucleotide insertions and/or deletions) with the other nucleic acid (or its complementary strand), there is nucleotide sequence identity in at least 60% of the nucleotide bases, usually at least 70%, more usually at least 80%, preferably at least 90%, and more preferably at least 95-98% of the nucleotide bases. Alternatively, substantial homology or substantial identity exists when a nucleic acid or fragment thereof will hybridize, under selective hybridization conditions, to another nucleic acid (or a complementary strand thereof). Selectivity of hybridization exists when hybridization which is substantially more selective than total lack of specificity occurs. Typically, selective hybridization will occur when there is at least about 55% sequence identity over a stretch of at least about nine or more nucleotides, preferably at least about 65%, more preferably at least about 75%, and most preferably at least about 90%. The length of homology or identity comparison, as described, may be over longer stretches, and in certain embodiments will often be over a stretch of at least 5 nucleotides, at least 14 nucleotides, at least 20 nucleotides, more usually at least 24 nucleotides, typically at least 28 nucleotides, more typically at least 32 nucleotides, and preferably at least 36 or more nucleotides.

A polypeptide or fragment thereof is "substantially homologous" or "substantially identical" to another if, when optimally aligned (with appropriate insertions and/or deletions) with the other polypeptide, there is nucleotide sequence identity in at least 60% of the nucleotide bases, usually at least 70%, more usually at least 80%, preferably at least 90%, and more preferably at least 95-98% of the amino acid residues. The length of homology or identity comparison, as described, may be over longer stretches, and in certain embodiments will often be over a stretch of at least 5 amino acids, at least 14 amino acids, at least 20 amino acids, more usually at least 24 amino acids, typically at least 28 amino acids, more typically at least 32 amino acids, and preferably at least 36 or more amino acids.

*CCDC88B.* This IEP is also referred to as coiled-coil domain containing 88B (*Homo sapiens* Gene ID 283234). Its function has not been established yet, but it is a member of the hook-related protein family. Members of this family are characterized by an N-terminal potential microtubule binding domain, a central coiled-coiled and a C-terminal Hook-related domain. The encoded protein may be involved in linking organelles to microtubules. The amino acid mutant this protein is provided as SEQ ID NO: 4 (murine version).

*FOXN1.* This IEP is also referred to as forkhead box N1, WHN, RONU as well as FKHL20. FOXN1 is a winged-helix transcriptional regulator *(Homo sapiens* Gene ID 8456). FoxN1 mouse mutants show absence of thymus and are severely immuno-compromised. Known human FOXN1 mutations cause T-cell immunodeficiency, congenital alopecia and nail dystrophy.

*USP15.* This IEP is also referred to as ubiquitin specific peptidase 15 (*Homo sapiens* Gene ID 9958). It is a member of the ubiquitin specific protease (USP) family of deubiquitinating enzymes. USP enzymes play critical roles in ubiquitin-dependent processes through polyubiquitin chain disassembly and hydrolysis of ubiquitin-substrate bonds. The encoded protein associates with the COP9 signalosome, and also plays a role in transforming growth factor beta signalling through deubiquitination of receptor-activated SMAD transcription factors. Alternatively spliced transcript variants encoding multiple isoforms have been observed for the gene encoding this IEP.

*THEMIS.* This IEP is also known as thymocyte selection associated (*Homo sapiens* Gene ID 387357). This protein is known to play a regulatory role in both positive and negative T-cell selection during late thymocyte development. The protein functions through T-cell antigen receptor signaling, and seems necessary for proper lineage commitment and maturation of T-cells. Alternative splicing results of its corresponding gene in multiple transcript variants.

*IRF8.* This IEP is also known as interferon regulatory factor 8 (*Homo sapiens* Gene ID 3394). This interferon consensus sequence-binding protein (ICSBP) is a transcription factor of the interferon (IFN) regulatory factor (IRF) family. Proteins of this family are composed of a conserved DNA-binding domain in the N-terminal region and a divergent C-terminal region that serves as the regulatory domain. The IRF family proteins bind to the IFN-stimulated response element (ISRE) and regulate expression of genes stimulated by type I IFNs, namely IFNα and IFNβ. IRF family proteins also control expression of IFNα and IFNβ-regulated genes that are induced by viral infection.

*IRF1.* This IEP is also known as interferon regulatory factor 1 (*Homo sapiens* Gene ID 3659). IRF1 is a member of the interferon regulatory transcription factor (IRF) family. IRF1 serves as an activator of interferons alpha and beta transcription, and in mouse it has been shown to be required for double-stranded RNA induction of these genes. IRF1 also functions as a transcription activator of genes induced by interferons alpha, beta, and gamma. Further, IRF1 has been shown to play roles in regulating apoptosis and tumor-suppressoion. Finally, IRF1 physically interacts with and heterodimerizes with IRF8 in the transcriptional activation of many genes implicated in response to infections and to inflammation.

*IRGM1.* This IEP is also known as immunity-related GTPase family, M (*Homo sapiens* Gene ID 345611). It is a member of the p47 immunity-related GTPase family. It is suggested to play a role in the innate immune response by regulating autophagy formation in response to intracellular pathogens. Polymorphisms that affect the normal expression of this gene are associated with a susceptibility to Crohn's disease and tuberculosis.

The present invention will be more readily understood by referring to the following examples which are given to illustrate the invention rather than to limit its scope.

### EXAMPLE I - GENETIC SCREENING IN ENU-INDUCED DOMINANT NEGATIVE MUTATIONS

The genetic screening was performed as presented in Bongfen *et al.* Briefly, a population of male G0 ENU-mutated mice was first generated. The mutant males were backcrossed for two-generations (G1 and G2) followed by breeding homozygosity in multiple G3 pedigrees. These mice were then infected with *Plasmodium bergei* to induce a cerebral malaria. Mice bearing mutations which prevented them from developing a full neuroinflammatory response and showing an unusual resistance to neuroinflammation (and ultimately survived the *P. bergei* challenge) were selected and their genome was sequenced to identify the genetic trait responsible for protecting the mice from succumbing to a *P. bergei* challenge.

As shown in Bongfen *et al.,* the first phenodeviant pedigree characterized carries a mutation in Jak3 (Jak3^{W81R}), a cytosolic tyrosine kinase that interacts with the common γ_{c} chain of cytokine receptors, including IL-2, IL-4, IL-7, IL-9, IL-15 and IL-21. This recruitment is required for the engagement of STAT family members and the transcriptional activation of inflammatory pathways in NK, T and B cells. Jak3^{W81R} mutants show reduced numbers of NK cells, CD8+ T cells and B cells, and severely reduced production of the pro-inflammatory cytokine IFNγ by CD4+ T cells. Interestingly, genetic variants in JAK and STAT family members have been associated with inflammatory diseases (IBD, MS, RA, SLE). In addition, Jak3 is a known target for anti-inflammatory drugs, and a Jak3 inhibitor that is currently in clinical use for rheumatoid arthritis (RA) and Crohn's disease (CD) (e.g., tasocitinib; Pfizer) can blunt neuroinflammation and increase survival of Jak3-/+ heterozygotes infected with *P. berghei.* Therefore, the JAK3 inhibitor pharmacologically mimicks the effect of the genetic mutation obtained and characterized in Bongfen *et al.*

The first phenodeviant pedigree (number 48) was found to carry a mutation in Jak3 (Jak3W81 R), a cytosolic tyrosine kinase that interacts with the common γ_{c} chain of type 1 and 2 cytokine receptors expressed on lymphocytes, which includes IL-2, IL-4, IL-7, IL-9, IL-15 and IL-21. Jak3 phosphorylation causes recruitment and phosphorylation of STAT family members for transcriptional activation of inflammatory pathways in T lymphocytes. Jak3W81R mutants show reduced numbers of CD8+ T cells and B cells, and severely reduced production of pro-inflammatory cytokines (IFNγ) by CD4+ T cells. Interestingly, genetic variants at JAK (JAK2 in inflammatory bowel diseases) and STAT family members (STAT3 in IBD and multiple sclerosis; STAT4 in rheumatoid arthritis, and Lupus erythematosus) have been found to be genetic risks in certain chronic inflammatory diseases in humans. These results confirm that the screen for mutations that protect against *P. berghei* infection-associated neuroinflammation can identify novel targets for anti-inflammatory drug discovery.

Mice were treated s.c. with 15mg/kg/day of tasocitinib (a known JAK3 inhibitor) for 4 days. Mice infected on day 5 of treatment with 10⁶ PbA-pRBC, and treatment continued for 4 more days (total of 9 days of treatment). As shown on Figure 1, treatment of Jak3+/- heterozygote mice with (starting 3 days before infection, and continuing for 7 days during infection with *P. berghei*) can blunt neuroinflammation and can significantly increase survival of *P. berghei*-infected Jak3^{+/-} heterozygotes (from 0% in untreated animals to 45% in treated animals). These results indicate that pharmacological modulation of a target discovered in the genetic screen can mimic the protective effect of a genetic lesion in the same gene. These results also indicate that the *P. berghei* infection model can be used to screen drug candidates for anti-inflammatory activity.

### EXAMPLE II - THEMIS

A genetic screen has been performed as described in Example I and a further protective mutation was identified in Themis (I23N), a protein associated with the T-cell receptor (TCR), which phosphorylation induces binding to Lck and Grb2 to stimulate the ERK1/ERK2 pathway. Themis is required for TCR activation and cytokine production by CD4+ and CD8+ lymphocytes in response to class I and class II MHC-dependent antigen presentation. In addition, inhibitors of the ERK pathway (PD184352, U0126) have been described and are available for testing *in vivo* and can be used for modulating the inflammatory response in vivo.

An heterozygote mouse strain has been produced.

### EXAMPLE III - FOXN1

A genetic screen has been performed as described in Example I and a further protective mutation was identified in the winged-helix transcriptional regulator FoxN1. Two nude resistant mice show a splice-site mutation (A-to-C) at the exon 6 donor site. FoxN1 mouse mutants show absence of thymus and are severely immuno-compromised, while human FOXN1 mutations cause T-cell immunodeficiency, congenital alopecia and nail dystrophy.

An heterozygote mouse strain has been produced.

### EXAMPLE IV - CCDC88B

A genetic screen has been performed as described in Example I and a further protective mutation was identified in Ccdc88b, a gene expressed in the thymus, lymph nodes, and bone marrow. The Ccdc88b's mRNA expression in macrophages is increased upon engagement of innate immune receptors TLR4 (lipopolysaccharide) and TLR9 (CpG oligonucleotides). In addition, CCDC88B's expression is also increased in response to infection with *Mycobacterium tuberculosis* (lung), *Plasmodium berghei* (blood, liver cells), and *Lesihmania* (macrophages). The function, biochemical activity and mechanism of action of the CCDC88B protein in lymphoid cells and in myeloid cells remain unknown. Structurally, CCDC88B is characterized by an N-terminal potential microtubule binding domain, a central coiled-coiled domain, and a C-terminal Hook-related domain. These features plus additional observations suggest that CCDC88B may form scaffolds in T lymphocytes, possibly those required to assemble the T cell receptor signaling complex.

It was noted that mice bearing an homozygote mutation at CCDC88B (a mutation in the splice site in intron 22 that abrogate splicing of exon 22/23, and results in an out of frame transcript) show strongly reduced numbers of granulocytes in the spleen (neutropenia). More specifically, in the homozygous mutant, the T to C mutation in the donor splice site between exon 22 and exon 23 of CCDC88B results in the activation of an alternative donor splice site upstream of the mutation in exon 22. As a consequence, there is a five nucleotide deletion in exon 22 causing a frameshift in the mutant polypeptide sequence. Using the online Transeq Nucleotide to Protein Sequence Conversion tool by EBI, it is predicted that the frameshift leads to an early stop codon and thereby nonfunctional associated protein. The expected amino acid sequence of the mutant CCDC88B protein is provided in the sequence listing as SEQ ID NO: 4.

Stable cell lines expressing either CCDC88B or its mutant version were developed (e.g. in HEK293 EV cells). An heterozygote mouse strain has been produced. In addition, rabbit polyclonal sera specific either for the wild-type polypeptide or the mutant polypeptide have been prepared.

The expression of CCDC88B has been further characterized by *in situ* hybridization. Tissues were fixed in 4% formaldehyde and hybridized with ³⁵S-labeled cRNA antisense and sense probes overnight at 55°C in 50% deionized formamide, 0.3 M NaCl, 20 mM Tris-HCI, pH 7.4, 5 mM EDTA, 10 nM NaPO₄, 10% dextran sulfate, 1 x Denhardt's, 50 µg/ml total yeast RNA, and 50 to 80 000 cpm/µl ³⁵S-labeled cRNA probe. The tissues were subjected to stringent washing at 65°C in 50% formamide, 2 x SSC, and 10 mM DTT, followed by washing in PBS before treatment with 20 µg/ml RNAse A at 37°C for 30 minutes. After washes in 2 x SSC and 0.1 x SSC for 10 minutes at 37°C, the slides were dehydrated, apposed to x-ray film for 3 and 6 days, then dipped in Kodak NTB nuclear track emulsion, and exposed for 18 days in light-tight boxes with desiccant at 4°C. Photographic development was undertaken with Kodak D-19. The slides are lightly counterstained with cresyl violet and analyzed under both light- and darkfield optics. *In situ* hybridization (ISH) was performed in 10-day old mice (p10) using the following probes :
Sense probe (SEQ ID NO: 5) - italics shows section specific to T7 promoter : *GCGCTATAATACGACTCACTATAGGGAGA*TCCGAATCTTTGGACCTGCCTTCT
Antisense probe (SEQ ID NO: 6) - italics shows section specific for the SP6 promoter: *GCATTAATTTAGGTGACACTATAGAAGCG*AAGCTAGCCGTATCCACTGCTTCA

In these mice, Ccdc88b's mRNA expression was detected in the thymus (at a high-level), in the spleen and bone marrow (at a low-level), as well as in the ribs and hip bones (data not shown).

When ISH was performed in adult mice using the same approach, increased Ccdc88b's mRNA expression was found in the spleen, and bone marrow, as well as in the lymph node (data not shown). In the thymus, Ccdc88b's mRNA was observed both in the cortex (at a higher cell density) and in the medulla (data not show). In the spleen, Ccdc88b's mRNA expression was increased in the germinal centers close to lymphoid nodule around central artery (data not shown). In the lymph nodes, Ccdc88b's mRNA expression was observed in the medulla but not in lymphoid follicles (data not shown). Similar results were obtained by confocal microscopy using the rabbit polyclonal serum generated (data not shown).

To confirm the ISH results, qPCR was performed to assess the expression of Ccdc88b's mRNA levels. Real-time quantitative PCR (qPCR) amplifications were performed on the Roche LightCycler 480 system using 96-microwell plates in a total volume of 10 µL, containing 5 µL cDNA sample, 5 uL PerfeCTa SYBR Green SuperMix (Quanta BioSciences) and 150 nM each of forward and reverse primers. PCR amplifications were conducted in triplicate with one RT-control for each sample, using the temperature cycles: 10 min at 94°C, followed by 45 cycles of (15 s at 94°C and 1 min at 60°C). Ccdc88b cDNA of length 150bp was amplified using 5'-GAT CTG GGG GCA CAG CGG TTG (SEQ ID NO: 7) and 5'-GCG TCT CAG CTG GGC CTT GGC (SEQ ID NO: 8), respectively. Gene expression was normalized to the reference gene HPRT, which was amplified as a 122 bp product using 5'-TCC AGC AGG TCA GCA AAG AAC (SEQ ID NO: 9) and 5'-GGA CTG ATT ATG GAC AGG ACT G (SEQ ID NO: 10) primers. Five samples of 10X dilutions were made to determine the amplification efficiencies of Hprt and Ccdc88b. Relative gene expression was quantified using the Pfaffl method as per the manufacturer's protocol, and normalized to uninfected C57BL/10 brain sample.

As shown on Figure 2A, high Ccdc88b's mRNA expression was found in the spleen, the thymus, the bone marrow and the lungs. Since there was an increased expression of Ccdc88b's mRNA in organs involved in inflammation, expression of Ccdc88b's mRNA in various inflammatory cell types was determined. As shown in Figure 2B, elevated expression of Ccdc88b's mRNA was found in GR1+ granulocytes, CD4+ and CD8+ T cells.

Immunophenotyping was performed on hemizygote mutant animals (CCDC88B^{+/-}). As shown on Figure 2C, in the spleen, there was a significant decrease in Gr1+CD11b+ neutrophils in uninfected hemizyogte mice. Consequently, CCDC8^{∗}B^{+/-} animals had a severe neutropenia phenotype. It was also determined that, upon inflammatory challenge, no inflammatory cells were recruited to the brain (data not shown).

The expression of Ccdc88b's mRNA in human glial cells and blood brain barrier endothelial cells was characterized. All tissues samples taken from human epilepsy patients. Tissues were non-inflamed. Three different individuals were tested per cell types. As shown in Figure 2D, the expression level of Ccdc88b's mRNA was determined in human astrocytes, microglia or blood brain barrier endothelial cells following 48h stimulation by cytokines optionally in combination LPS. Expression of Ccdc88b's mRNA was increased in microglia following stimulation with IFNγ and LPS. No expression of Ccdc88b's mRNA was detected in astrocytes or in endothelial cells.

### EXAMPLE V - USP15

A genetic screen has been performed as described in Example I and a further protective mutation was identified in Usp15 (Ubiquitin carboxyl-terminal hydrolase 15), a deubiquitinating enzyme (DUB). The mutation (Usp15^{L749R}) is a non-conservative change at a highly conserved residue in the catalytic domain common to DUBs. USP15 has been shown to deubiquitinate receptor associated cytosolic SMADs to regulate downstream TGFβ signaling. Although the function of USP15 in inflammatory cells is unknown, addition/removal of ubiquitin from proteins is a common means of regulating immune signaling, and several genes associated with inflammatory diseases in humans either regulate ubiquitination (TNFAIP3), bind to ubiquitinated proteins (TNIP1, UBASH3A) or regulate enzymatic events in ubiquitination (UBE2L3). The USP15 protein appears to be expressed at least in the brain.

An heterozygote mouse strain has been produced.

### EXAMPLE VI - IRF8

IRF8 is a member of the Interferon Regulatory Factor (IRF) family of transcriptional regulators that plays a central role in interferon signaling, response to infection and maturation of myeloid lineages, including dendritic cells (DC). It is composed of a helix-turn-helix DNA binding domain and a trans-activation domain also known as the IRF association domain. In myeloid and lymphoid cells, IRF8 regulates constitutive gene expression and also activates or suppresses pathogen responsive transcription programs following exposure of these cells to type I or type II interferon, lipopolysaccharides, and a range of microbial products. Heterodimerization of IRF8 with members of the IRF (IRF1, IRF4) or ETS (PU.1) families leads to DNA binding and transcriptional regulation of target genes containing ISRE (GAAAnnGAAA) (SEQ ID NO: 1) and EICE-type canonical motifs (GGAAnnGAAA) (SEQ ID NO: 2), respectively, in their promoters.

During hematopoiesis, IRF8 promotes differentiation of myeloid progenitors towards the mononuclear phagocyte lineages (monocytes, macrophages, DC) by acting as an antagonist of the polymorphonuclear granulocyte pathway. This is accomplished through positive regulation of pro-apoptotic signals (Cdkn2b, Nf1, Bax), and negative regulation of pro-survival signals (Bcl2, Bcl-XL) in CD11b+ myeloid precursors. Mice harboring either complete (null7) or severe (Irf8^{R294C} from BXH2 mice) loss of function mutations at Irf8 show a complete or partial absence of all classes of DCs, both CD11c+CD8α+ DCs and plasmacytoid DCs, and display a chronic myeloid leukemia-like phenotype dominated by expansion of Gr1+/CD11b+ granulocyte precursors. Additionally, IRF8 has been shown to be required for B lymphocytes lineage specification, commitment and differentiation, including expression of biochemical pathways that play a key role in the specialized functions of these antigen-presenting cells.

In the context of infection, IRF8 is required for the activation of anti-microbial defenses of resident myeloid cells, for propagation of pro-inflammatory signals and for amplification of the early immune response by these cells. IRF8 is essential for antigen presenting cell-mediated Th1 polarization of early immune responses, as it is necessary for expression of the IL12p40, IL12p35 and IL-18 genes in response to IFNγ. Consequently, Irf8-deficient mice display defective Th1 response (absence of antigen specific CD4+, IFNγ producing T cells), show enhanced Th17 response, and are susceptible to *in vivo* infection with many intracellular pathogens including tuberculosis and blood-stage malaria. Furthermore, genome-wide transcript profiling, chromatin immunoprecipitation experiments and individual gene studies show that IRF8 regulates several aspects of anti-microbial defenses in mononuclear phagocytes, including antigen recognition and processing, phagosome maturation, production of lysosomal enzymes and of other cytoplasmic microbicidal pathways. As a result, Irf8-deficient macrophages are extremely susceptible to *ex vivo* infection with a variety of intracellular pathogens.

IRF8 mutations in humans cause pathologies remarkably similar to those observed in Irf8 mutant mice, and affect myeloid cells in general and DCs in particular. In one patient, homozygosity for a transcriptionally inactive and DNA-binding incompetent IRF8 mutant variant (IRF8^{K108E}) was associated with severe and recurrent perinatal bacterial and fungal infections, with absence of blood monocytes and DCs in lymph nodes and bone marrow, and a lack of IL-12 and IFNγ production following stimulation of blood cells *in vitro.* A milder autosomal dominant form of IRF8-deficiency (IRF8^{T80A}) was also recently discovered in two MSMD patients (Mendelian Susceptibility to Mycobacterial Disease) suffering from recurrent episodes of mycobacterial infections following perinatal vaccination with *M. bovis* BCG. These patients had a selective depletion of the CD11c+ CD1c+ DC subset, and impaired production of IL-12 by circulating peripheral blood cells. The IRF8^{T80A} variant displays negative dominance and can suppress the trans-activation potential of wild type IRF8 for known transcriptional targets such as NOS2 and IL-12. Finally, recent results from genome wide association studies (GWAS) have pointed to a role of IRF8 in the complex genetic etiology of multiple human diseases with important inflammatory components. Strong and independently replicated associations have been detected between polymorphic variants within or near IRF8 in patients with systemic lupus erythematosus, Crohn's disease(CD) and multiple sclerosis (MS). In one study of MS patients, the susceptibility allele at IRF8 is associated with higher IRF8 mRNA expression.

An experimental model of cerebral malaria (CM) induced by infection with *Plasmodium berghei* ANKA (PbA) was used to investigate the role of IRF8 in pathological inflammation. In this model, adherence of PbA-infected erythrocytes to brain microvasculature leads to acute and rapidly fatal neuroinflammation, which symptoms include tremors, ataxia and seizures begining to appear between d5-d8 post-infection. IRF8-deficient BXH2 mice (Irf8^{R294C}) do not develop any neurological symptoms and are completely resistant to PbA-induced CM. Comparative transcript profiling studies in PbA-infected brains of wild-type C57BL/6 and Irf8-deficient BXH2 mice, together with IRF8 chromatin immunoprecipitation coupled to high-throughput DNA sequencing (ChIP-seq) have identified a list of key IRF8 targets whose expression is associated with acute CM-associated neuroinflammation, but is also found activated in lungs infected with *M. tuberculosis.* The role of several of these genes in CM-pathology has been validated in vivo in corresponding mouse mutants infected with *P. berghei.* These studies identify IRF8 as a key regulator of acute neuroinflammation during CM.

*Mice.* C57BL/6J (B6), BXH2, Il12p40-/-, Irf1-/-, and Isg15-/- mutant mice were originally obtained from the Jackson Laboratory (Bar Harbor, ME). Stat1-/- mutant mice were purchased from Taconic Farms (Germantown, NY). Ifng-/- deficient mice were obtained from Dr. M.M. Stevenson (Montreal General Hospital Research Institute), Ifit1-/- mutant mice were obtained from Dr. M. Diamond (Washington University School of Medicine, St-Louis), Irgm1^{-/-} from Dr. J.D. MacMicking (Yale, New Haven, CT) and Nlrc4^{-/-} from Millenium Pharmaceuticals, Inc. and Dr. R.A. Flavell (Yale, New Haven, CT). All mice were kept under pathogen free conditions and were handled according to the guidelines and regulations of the Canadian Council on Animal Care.

*Parasites and Infection. P. berghei* ANKA was obtained from the Malaria Reference and Research Reagent Resource Centre (MR4), and was stored frozen at -80°C. Prior to experimental infections, *P. berghei* ANKA was passaged in B6 mice until peripheral blood parasitemia levels reached 3-5%, at which point animals were euthanized by CO₂ inhalation, exsanguinated and an infectious stock was prepared. All experimental infections were done via intraperitoneal (i.p.) injection with 10⁶ parasitized red blood cells (pRBC). Blood parasitemia was monitored during infection by microscopic examination of thin-blood smears stained with Diff-Quick™ (Dade Behring, Newark, DE, USA). The appearance of neurological symptoms (shivering, tremors, ruffled fur, seizures) associated with cerebral malaria (CM) was monitored closely, and affected animals were immediately sacrificed. Survival curves were compared using Kaplan-Meier statistics.

*Evans Blue dye extravasation assay.* To monitor the integrity of the blood brain barrier during experimental CM, groups of control and PbA infected C57BL/6 and BXH2 mice were injected i.p. with 0.2ml of 1% Evan's Blue dye (E2129; Sigma-Aldrich, Oakville, ON, Canada) in sterile phosphate-buffered saline (PBS) on d7 and d16 (BXH2 only) post-infection (n=3 mice/condition). The dye was allowed to circulate for 1h, then the mice were sacrificed by CO₂ inhalation, perfused with PBS and the brains were dissected and photographed. To quantify dye accumulation in the brain, tissues were weighed and the dye was extracted 48 hours in 1ml N,N-dimethylformamide, followed by measuring optical density at OD₆₂₀. A standard curve was prepared at the same time and linear regression was used to calculate the concentration of dye in each extracted sample solution. Uninfected mice were also injected with dye, perfused and dissected as controls.

*Serum Cytokines.* Five male mice of each wild type B6, C3H/HeJ, and mutant BXH2 were infected i.p. with 10⁶ *P. berghei* ANKA pRBC, and at 6 days post-infection, they were sacrificed and serum was collected. Levels of circulating cytokines IFNγ, IL-2, IL-10, IL12p40, IL12p70, MCP-1 (CCL2), MIP-1β (CCL4), RANTES (CCL5), TNF-α, and VEGF were measured using an ELISA-based commercial reagent (Milliplex assay™; Millipore).

*Transcript Profiling.* Whole brains were dissected from B6 and BXH2 mice either prior to (d0) or 7 days (d7) post infection (n=3/condition). Total brain RNA was isolated using TRIzol™ reagent (Invitrogen, Burlington, Canada) according to the manufacturer's instructions, followed by further purification with RNeasy™ columns (Qiagen, Toronto, Canada) and hybridized to Illumina MouseWG-6 v2.0 microarrays (Genome Quebec Innovation Centre, Montreal, Canada). Unsupervised principal components analysis was done in R, using the lumi package to transform with vst (variance stabilizing tranformation) and to perform quartile normalization. For other analyses, microarray expression data was log2 transformed, median normalized and analyzed using GeneSifter (Geospiza) software. Groups were compared using either a pairwise (t-test, ≥2-fold cutoff, Benjamini-Hochberg corrected p_{adj}-values <0.05) or using a two factor ANOVA (≥2-fold cutoff, Benjamini-Hochberg corrected p_{adj}-values <0.05) to identify genes whose expression is modulated in a strain-dependent, infection-dependent and/or interactive fashion. Lists of genes that were differentially expressed were clustered according to fold change using Multi Experiment Viewer.

*Chromatin immunoprecipitation (ChIP).* The J774 mouse macrophage cell line was grown to 80% confluence in complete Dulbecco's modified Eagle's medium (DMEM). The cells plated in 150 mm tissue culture-grade Petri dishes (Corning Inc., Corning, NY) were treated with 400U/ml IFNγ (Cell science, Canton, MA) and CpG DNA oligonucleotides (5'-TCCATGACGTTCCTGACGTT-3') (SEQ ID NO: 3) for 3 h. Chromatin immunoprecipitations were performed as previously described with few modifications. Briefly, treated cells were crosslinked for 10 min at 20°C with 1% formaldehyde in culture medium. Crosslink was stopped with ice-cold PBS containing 0.125M glycine for 5 min. Nuclei were prepared and chromatin was sonicated with a Branson Digital Sonifier (Branson Ultrasonics, Danbury, CT) to an average size of 250 bp. Sonicated chromatin was incubated overnight on a rotating platform at 4ºC with a mixture of 20µl Protein A and 20 µl Protein G Dynabeads (Invitrogen, Carlsbad, CA) pre-bound with 6 µg of normal goat IgG (sc-2028) or IRF8 (sc-6058x) antibodies (Santa Cruz Biotechnologies, Santa Cruz, CA). Immune complexes were washed sequentially for 2 min at room temperature with 1ml of the following buffers: Wash B (1% Triton X-100, 0.1% SDS, 150 mM NaCl, 2 mM EDTA, 20 mM Tris-HCI pH 8), Wash C (1% Triton X-100, 0.1% SDS, 500 mM NaCl, 2 mM EDTA, 20 mM Tris-HCI pH 8), Wash D (1% NP-40, 250 mM LiCI, 1 mM EDTA, 10 mM Tris-HCI pH 8), and TEN buffer (50 mM NaCl, 10 mM Tris-HCI pH 8, 1 mM EDTA). After decrosslinking, the DNA was purified with QIAquick™ PCR purification columns following manufacturer's procedure (Qiagen, Mississauga, Ca). IRF8 ChIP efficiency relative to the IgG control was assessed by qPCR using the Perfecta SYBR™ green PCR kit (Quanta Bioscience, Gaithersburg, MD) for known IRF8 binding sites.

*ChIP-seq preparation and analysis.* A total of 8 independent ChIPs were pooled for each condition (IRF8 and IgG). Libraries and flow cells were prepared by the IRCM Molecular Biology Core Facility following Illumina's recommendations (Illumina, San Diego, CA), with a size selection step targeting fragments between 250 and 500 bp.

The ChIP libraries were sequenced on Illumina HiSeq 2000 sequencer. The sequencing yielded 86 and 79 million 50 bp sequence reads for IgG control and IRF8 samples, respectively. The reads were mapped to the mouse mm9 genome assembly using Bowtie with the following parameters: -t --solexa1.3-qual --sam --best mm9. The mapping efficiency was 91.7% for IgG and 91.9% for IRF8 samples. To identify IRF8 binding peaks, we used the MACS 1.4.1 peak finder with the following parameters: -- bw 250 --mfold 7,30 --pvalue 1e-5 -g mm. This analysis yielded 11216 genomic regions bound by IRF8 with p-values under the threshold of 10⁻⁵. The genes identified as affected by PbA infection in the expression profiling experiment were queried for the presence IRF8 binding peaks in a 20 kb interval around the gene transcription start site (TSS). This analysis was also performed for all the genes represented on the Illumina mouse WG-6 v2.0 array used in the microarray experiments, to assess the background association of IRF8 peaks with surrounding genes (Figure 6).

BXH2 is a recombinant inbred mouse strain derived from C57BL/6J (B6) and C3H/HeJ (C3H) parents that carries a severely hypomorphic allele at Irf8 (Irf8^{R294C}) and that causes a myeloid defect expressed as granulocytic hyperplasia, depletion of mononuclear phagocytes, and susceptibility to infections. To assess the contribution of Irf8 to pathological inflammation, BXH2 (n=18) and parental control mice B6 (n=19) and C3H mice (n=10) were infected with the murine agent of cerebral malaria (CM), *P. berghei* ANKA. Parasite replication in the blood, appearance of neurological symptoms of CM and overall survival were recorded in infected mice over 18 days (Figure 3). While all B6 and 80% of C3H mice developed CM and succumbed by day 9, BXH2 mice were completely resistant to the CM phase, succumbing later to hyperanemia caused by uncontrolled blood-stage replication of the parasite (Figures 3A and D). [BXH2 X B6]F1 mice (n=15) showed significant resistance to PbA induced CM when compared to susceptible B6 and C3H parental controls, with approximately 50% of the animals surviving past day 9 (p=0.03 versus C3H, p<0.0001 versus B6), suggesting that the CM-resistance trait of BXH2 is inherited in a co-dominant fashion. Additional phenotyping of a small group of [BXH2 X B6]F2 mice (n=17) identified CM-resistance only in mice either homozygote or heterozygote for the Irf8^{R294C} allele, confirming that the protective effect is due to the Irf8^{R294C} mutation with minimal or no contribution of the mixed B6/C3H genetic background of BXH2. These data show that that partial or complete loss of IRF8 function protects mice against lethality in this CM-associated neuroinflammation model. They also confirm that the CM-protective effect of the Irf8^{R294C} mutation is inherited in a co-dominant fashion.

Previous reports have demonstrated that lethal CM in PbA-infected mice is associated with endothelial dysfunction, including loss of integrity of the blood brain barrier (BBB). Using the Evans Blue dye extravasation test (Figures 3B and C), it was noted that while PbA-infected B6 mice displayed obvious BBB permeability by d7, infected BXH2 mice retained integrity of the BBB, and excluded Evans Blue dye, both early (day 7) and late (day 16) during infection at levels comparable to uninfected mice (Figures 3B and C). Resistance to CM in BXH2 mice was not associated with decreased parasite burden (Figure 3D), as surviving B6 and C3H controls, as well as BXH2 and [BXH2 X B6]F1 showed similar circulating blood parasitemia at days 5, 7 and 9 post-infection (p > 0.1). As the infection progressed, however, some of the BXH2 mice developed extremely high levels of blood parasitemia (between d12-21) in contrast to surviving controls and [BXH2 X B6]F1s. This high parasitemia, rather than cerebral inflammation, was responsible for the observed mortality. These results demonstrate that, although loss of IRF8 activity is protective against cerebral malaria, it is required to control blood stage replication of PbA late in infection, and that partial IRF8 function in [BXH2 X B6]F1 is sufficient to protect against high blood-stage replication.

Although there was substantial variation between individual mice of the same group, serum cytokine analysis showed that 6 days post-infection, resistance to PbA in BXH2 mice was associated with reduced levels of TNF-α (compared to C3H controls; p<0.001), and IFNγ (compared to B6 controls; p=0.07), although these differences did not reach statistical significance for the other parental control. Average levels of IL-2, IL-12p70 and VEGF were below the assay limit of detection and no difference in serum levels were detected amongst the other tested cytokines (IL-10, CCL2 (MCP-1), CCL4 (MIP-1β) and CCL5 (RANTES))(Figure 4).

To gain further insight into the genes, proteins and pathways that play a key pathological role during neuroinflammation, and whose expression is regulated by IRF8, several experiments were conducted. First, transcript profiling was used to identify genes differentially regulated in the brains of BXH2 and B6 mice either prior to or during PbA-infection. Principal components analysis (PCA) clustered the samples along two axes: component 1, which explained 39.4% of the variance and was associated to infection status (infection component), and component 2, analogous to strain (genetic component), which explained 24.4% of the variance (Figure 5A). PCA also indicates that PbA infection had a much stronger impact on transcriptional profiles in B6 mice than in BXH2, with the B6 d7 infected samples forming a remote out-group. In contrast, the BXH2 d7 cluster was only moderately shifted by infection and remained much closer to the BXH2 d0 group (compared to B6 d0 vs. B6 d7) indicating far more modest response by BXH2. Paired t-tests were used to assess the transcriptional response changes due to infection in both B6 and BXH2 mice in order to extract gene lists that include strong changes relevant to pathological neuroinflammation. As suggested by the PCA, B6 response to infection was robust, with 296 unique genes showing statistically significant differences in expression (d0 vs. d7; fold change ≥2, p_{adj}<0.05). On the other hand, response to infection in BXH2 was more modest with 81 genes reaching statistical significance. More than half of the genes (n = 48) regulated by infection in BXH2 were common to the B6 set and may correspond to IRF8-independent regulatory mechanisms. This analysis also identified a subset of 117 genes that show significant differences in expression in B6 vs. BXH2 mice prior to infection. Only ∼10% of these "genetically regulated" genes (n = 16) were further significantly modulated by *P. berghei* infection (Figure 4B). Importantly, this analysis also identified a subset of 231 genes that were uniquely regulated in B6 mice by infection.

To investigate the role of Irf8 in CM-associated neuroinflammation phenotype, a two-factor ANOVA was performed accounting for both differences in basal level of gene expression in the brain (B6 vs. BXH2 at day 0), and infection-induced transcriptional response to PbA (Figure 5C). This analysis identified a total of 107 genes (123 probes; fold change ≥ 2, p_{adj}<0.05) that were strongly regulated by infection in an Irf8 dependent fashion (p_{adj}-interaction<0.05) (Figure 5C, Table 1). Euclidean hierarchical clustering of this gene list identified three major categories of transcripts. Group 1 genes (n=15) were up-regulated by infection in both strains (up-regulation more pronounced in B6 than BXH2), group 2 genes were up-regulated by infection in B6 mice but not significantly induced in BXH2 (n=62) and group 3 genes (n=30) were downregulated by infection (stronger repression in B6 compared to BXH2). Using the online Database for Annotation and Integrated Discovery (DAVID) tool to examine the complete list of genes regulated by strain and by infection indicated substantial enrichment for immune response (4.4-fold enrichment above Illumina WG-6 v2.0 chip background, p_{adj}=3.3x10⁻¹²), antigen processing and presentation (11.0-fold enrichment, p_{adj}=1.4x10⁻⁹), defense response (3.8-fold enrichment, p_{adj}=1.7x10⁻⁸), chemotaxis (5.2-fold enrichment, p_{adj}=3.4x10⁻³) and inflammatory response (3.6-fold enrichment, p_{adj}=3.7x10⁻³). Up-regulated genes on these lists include potent pro-inflammatory chemoattractant chemokines that recruit myeloid and lymphoid cells to the site of infection and/or tissue injury such as Cxcl9, Cxcl10, Ccl4, and Ccl12, proteins in myeloid cells associated with phagocytosis of microbes (Fcgr4; low affinity IgG receptor) and maturation of phagosomes (small GTPases Igtp, Irgm1, Gbp2, Gbp3), IRF8's heterodimerization partner (Irf1), and early type I interferon response (Oasl2, Ifit3). Genes under these immune and inflammatory response categories were found to be expressed at a higher level in B6 than in BXH2 mice, consistent with the notion that resistance to CM-associated neuroinflammation in BXH2 is linked to reduced IRF8-dependent inflammatory and innate immune responses, with a strong involvement of the myeloid compartment.

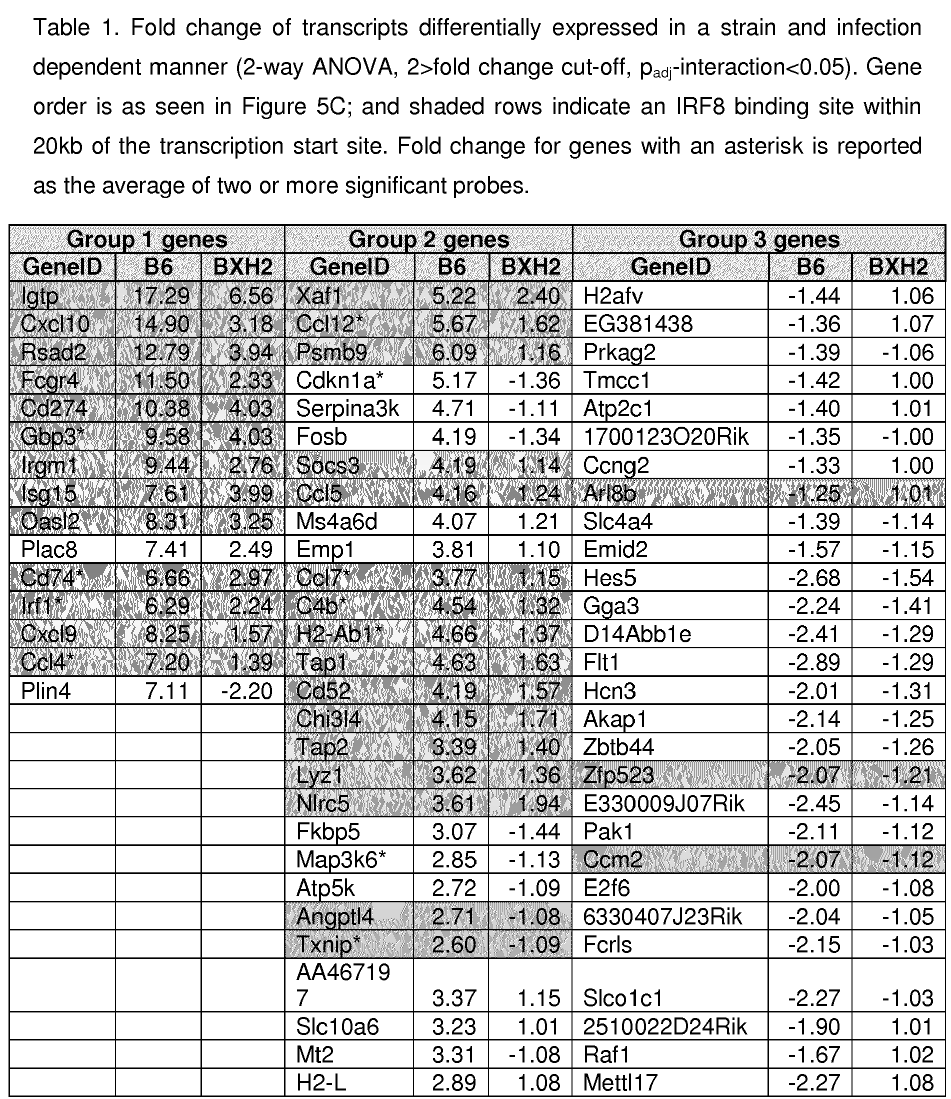

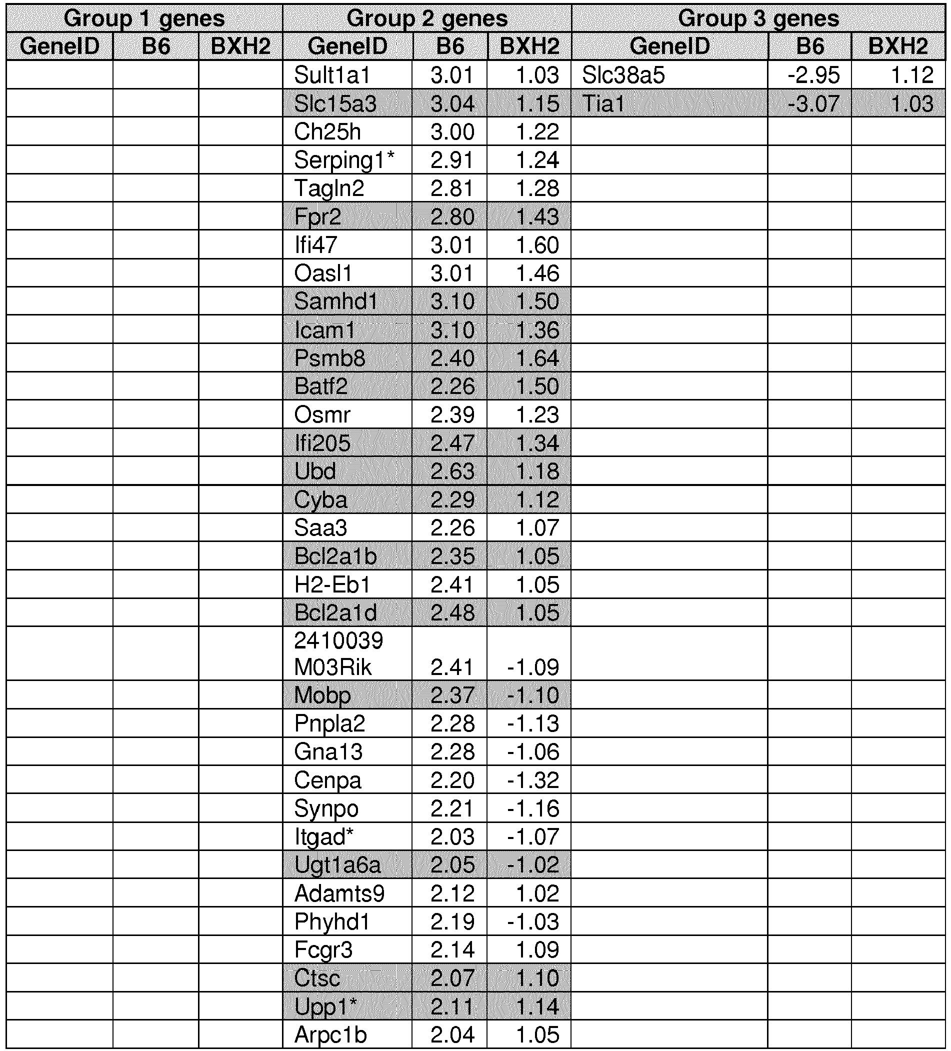

Since total brain RNA was used, genes differentially regulated in response to infection in an Irf8-dependent fashion may represent direct transcriptional targets of IRF8 or may be secondary targets that correspond to markers of cell populations that are differentially recruited to the site of infection in B6 and BXH2 mice. To distinguish between genes that are directly or indirectly regulated by Irf8 in response to infection, we mapped genome-wide IRF8 binding sites. For this, chromatin immunoprecipitation was performed followed by high-throughput sequencing (ChIP-seq) on cultured macrophages treated with CpG and IFNγ. The resulting sequence reads were mapped to the mm mouse reference genome and IRF8 binding peaks were identified using MACS peaks finding algorithm. In order to validate ChIP-seq results, IRF8 recruitment was confirmed on several known target sites by independent ChIP-qPCR experiments (Figure 6A). The list of IRF8-bound genes (identified as containing a IRF8 binding site within a 20kb window from the transcriptional start site) was intersected with the list of genes differentially regulated by PbA in a strain, infection and putatively Irf8-dependent fashion (Figure 5C). This intersection revealed a strong enrichment of IRF8 binding sites in genes up-regulated during infection, with IRF8 binding sites detected in 85% of Group 1 genes (13/15) and 50% of Group 2 genes (31/62) (Table 1). In contrast, differentially down-regulated genes did not show any enrichment with only 13% (4/30) of Group 3 genes associated with IRF8 peaks, lower than background peak association (21% of all genes represented on the Illumina array, Figure 6B). These results strongly suggest that during neuroinflammation, IRF8 functions as a direct transcriptional activator to up-regulate expression of genes that play a key role in this pathological response to PbA infection.

The list of all genes whose expression is regulated in Irf8-competent B6 mice was additionally queried in response to PbA (infection regulated genes; pairwise comparison for B6 d7/d0), for the presence of IRF8 binding sites and in order to identify IRF8-bound genes associated with CM susceptibility and neuropathology . As depicted in Figure 6B, this analysis showed very strong enrichment for IRF8 binding sites (p<0.0001, Fisher's Exact test) in the vicinity of genes up-regulated by infection, with 74% (92/125) of up-regulated genes bearing one or more IRF8 binding sites within 20kb of the TSS (Figure 6B with IRF8-binding profile examples provided in Figure 6C). Genes showing down-regulation in response to infection did not show such any enrichment above background (Figure 6B). The AMIGO gene ontology annotation tool was used to functionally examine the list of genes regulated by infection during neuroinflammation in B6 mice (Figure 5D and Table 2). This list is dominated by genes involved in inflammatory and innate immune response, even more pronounced in the subset (74%) of IRF8-bound genes, which includes inflammatory cytokine and chemokines involved in chemotaxis of myeloid and lymphoid cell types to the sites of infection (Ccl4, Ccl5, Ccl7, Ccl12, Cxcl9, Cxcl10), early innate immune recognition and responses (Nlrc5, Ifi205), response to viral infections (Oasl2, Mx2, Oas1g), type I interferon responsive genes and pathways (Ifit2, Ifit3, Isg15, Rsad2), antigen capture (C1q, C4b, Fcerg1), phagosome maturation (Irgm1, Irgm2, Igtp, Gbp2, Gbp3), antigen processing (Tap1, Tap2) and Class I and Class II MHC-dependent antigen presentation in myeloid cells (B2m, H2-Ab1, H2-D, H2-K, H2-L, H2-Q, H2-T22). Furthermore, other IRF family members implicated in early response to antigenic stimuli or danger signals (Irf1, Irf7, Irf9) were also induced (Table 2). These results support a key role for IRF8 as a transcriptional activator of pro-inflammatory genes and associated pathways that underlie the host-driven pathological neuroinflammation seen in PbA infection. These Irf8-regulated pro-inflammatory pathways appear linked primarily to the myeloid cellular compartment.

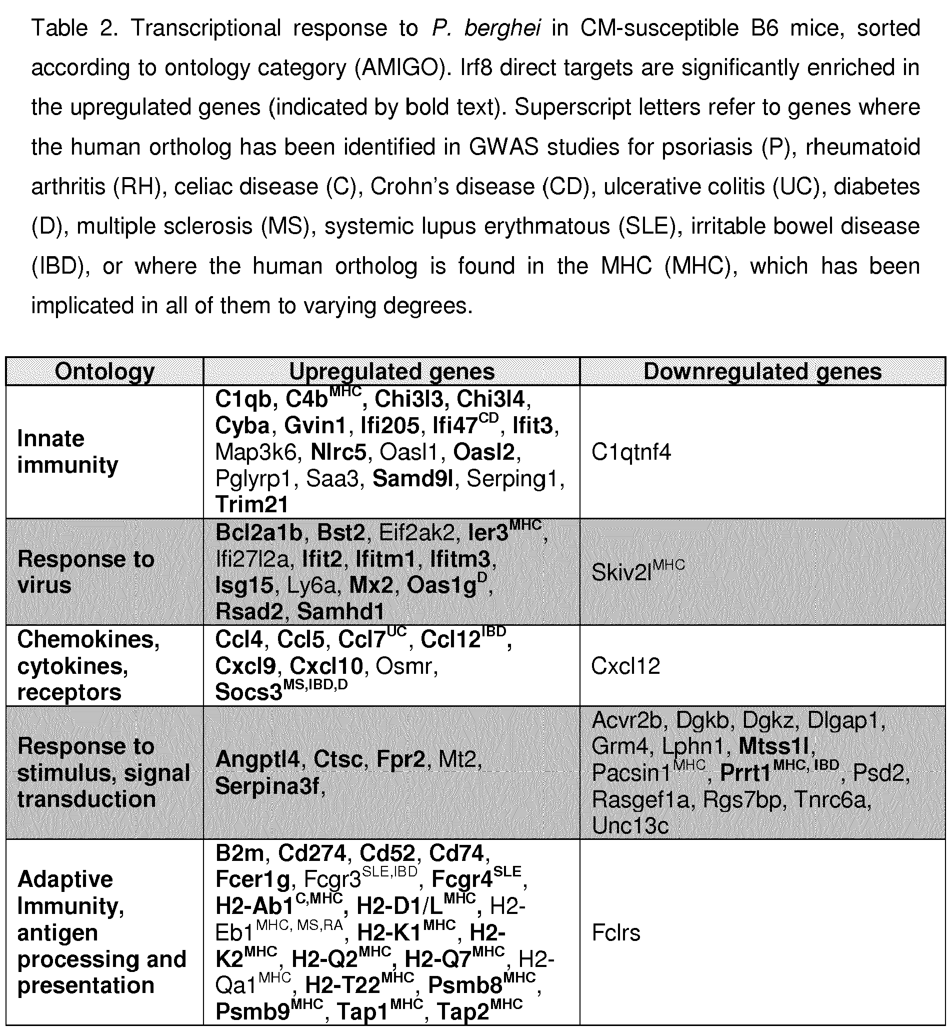

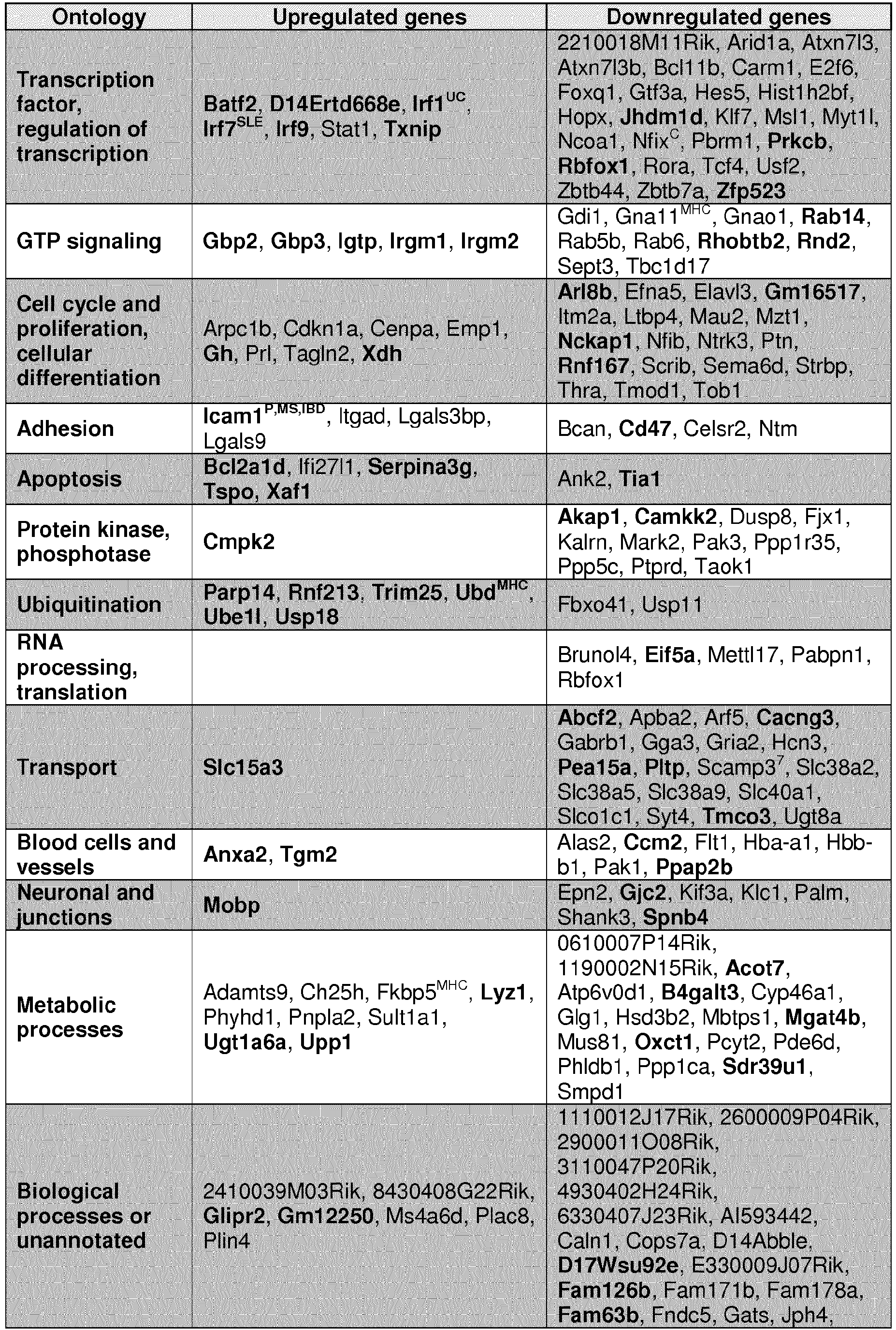

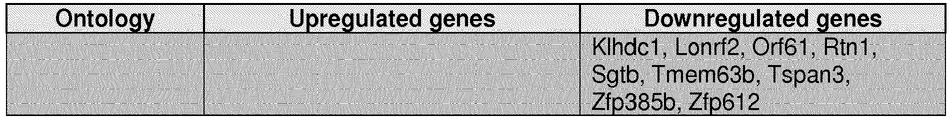

Involvement of the host-response pathways identified in Table 2 is not unique to cerebral malaria, so the list of genes regulated by infection in B6 brains during pathological neuroinflammation (d7/d0) was compared with the list of genes contributing to the protective response in the lungs of *Mycobacterium tuberculosis* infected B6 mice (d30/d0) (Table 3). B6 and BXH2 mice have opposite phenotypes in the two disease models B6 being susceptible to CM, but resistant to *M. tuberculosis,* while BXH2 is resistant to CM but succumbs extremely rapidly to disseminated mycobacterial disease when infected with *M. tuberculosis.* Strikingly, of the 123 genes up-regulated more than 2-fold during CM, 66 were also up-regulated ≥2-fold during M. *tuberculosis* infection (p<0.0001, Fisher's Exact Test). There was minimal overlap in the down-regulated genes (21 *M. tuberculosis*-regulated genes overlapping with the 170 PbA-regulated). Looking at the genes up-regulated by both infections, the overwhelming majority (80%) contained at least one IRF8 binding site, again highlighting IRF8 central role during inflammation and host response to infections (Table 3).

**Table 3. List of genes contributing to the protective response in the lungs of Mycobacterium tuberculosis infected B6 mice (d30/d0).**

| **Gene ID** | **PbAd7/d0** | **Mtb30/0** | **adj. p-value** | **Gene Name** | **#IRF8 binding peaks** |
|---|---|---|---|---|---|
| Gh | 84,82 | | 1,1E-02 | Growth hormone (Gh), mRNA | 1 |
| Gbp2 | 25,38 | 16,53 | 2,0E-03 | Guanylate binding protein 2, mRNA (cDNA clone MGC:41173 IMAGE:1230883) | 1 |
| Prl | 22,77 | | 1,6E-02 | Prolactin (Prl), mRNA | |
| Igtp | 17,29 | 14,99 | 2,3E-03 | Interferon gamma induced GTPase (Igtp), mRNA | 3 |
| CxcI10 | 14,90 | 92,96 | 1,1E-02 | Chemokine (C-X-C motif) ligand 10, mRNA (cDNA clone MGC:41087 IMAGE:1446589) | 3 |
| Ifit3 | 14,88 | 5,28 | 2,4E-03 | Interferon-induced protein with tetratricopeptide repeats 3, mRNA (cDNA clone MGC:6081 IMAGE:3487345) | 3 |
| Rsad2 | 12,79 | 3,93 | 5,3E-03 | Viperin (Vig1) | 4 |
| Fcgr4 | 11,50 | 13,19 | 3,1E-03 | Fc receptor, IgG, low affinity IV (Fcgr4), mRNA | 2 |
| Cd274 | 10,38 | 11,25 | 3,1E-03 | CD274 antigen (Cd274), mRNA | 2 |
| Irgm1 | 9,44 | 10,82 | 5,3E-03 | Immunity-related GTPase family M member 1 (Irgm1), mRNA | 1 |
| Gbp3 | 9,40 | 12,61 | 2,0E-03 | Guanylate binding protein 3, mRNA (cDNA clone MGC:29218 IMAGE:5036920) | 2 |
| CxcI9 | 8,25 | 413,81 | 8,8E-03 | Chemokine (C-X-C motif) ligand 9, mRNA (cDNA clone MGC:6179 IMAGE:3257716) | 1 |
| Ifi27I2a | 7,91 | 2,22 | 5,3E-03 | Interferon stimulated gene 12 (Isg12) | |
| Usp18 | 7,68 | 3,63 | 5,3E-03 | Ubiquitin specific protease UBP43 | 3 |
| Isg15 | 7,61 | | 2,0E-03 | ISG15 ubiquitin-like modifier, mRNA (cDNA clone MGC:18616 IMAGE:3670747) | 1 |
| PIac8 | 7,41 | 4,20 | 7,6E-03 | C15 protein | |
| S3-12 | 7,11 | -4,02 | 1,9E-02 | Plasma membrane associated protein, S3-12 (S3-12), mRNA | |
| Cd74 | 6,51 | 2,25 | 2,5E-03 | CD74 antigen (invariant polypeptide of major histocompatibility complex, class II antigen-associated), mRNA (cDNA clone MGC:6 | 2 |
| Irf1 | 6,49 | 3,53 | 2,5E-03 | Interferon regulatory factor 1, mRNA (cDNA clone MGC:6190 IMAGE:3600525) | 2 |
| Rnf213 | 6,38 | 2,06 | 2,4E-03 | D11 Ertd759e | 2 |
| LgaIs3bp | 6,36 | 3,86 | 6,5E-03 | Lectin, galactoside-binding, soluble, 3 binding protein (LgaIs3bp), mRNA | |
| Psmb9 | 6,09 | 8,46 | 2,0E-03 | Proteasome (prosome, macropain) subunit, beta type 9 (large multifunctional peptidase 2) (Psmb9), mRNA | 5 |
| CcI12 | 6,06 | 10,68 | 1,0E-02 | Chemokine (C-C motif) ligand 12, mRNA (cDNA clone MGC:41146 IMAGE:1548072) | 1 |
| Irgm2 | 5,79 | 7,88 | 2,7E-02 | Immunity-related GTPase family M member 2 (Irgm2), mRNA | 2 |
| Serpina3f | 5,63 | | 5,3E-03 | serine (or cysteine) peptidase inhibitor, clade A, member 3F | 1 |
| OasI2 | 5,58 | 5,97 | 8,7E-03 | 2-5 oligoadenylate synthetase-like 2, mRNA (cDNA clone MGC:6269 IMAGE:2646375) | 1 |
| Ifitm3 | 5,57 | | 5,0E-03 | Interferon induced transmembrane protein 3 (Ifitm3), mRNA | 5 |
| Serpina3g | 5,49 | 24,45 | 2,5E-03 | serine (or cysteine) peptidase inhibitor, clade A, member 3G | 1 |
| Xaf1 | 5,22 | | 5,3E-03 | gene model 881, (NCBI) | 1 |
| Cdkn1a | 5,17 | | 4,5E-02 | Cyclin-dependent kinase inhibitor 1A (P21) (Cdkn1a), transcript variant 1, mRNA | |
| Ifitm1 | 4,84 | | 1,2E-02 | Interferon induced transmembrane protein 1 (Ifitm1), transcript variant 1, mRNA | 1 |
| Parp14 | 4,74 | 2,78 | 8,8E-03 | Poly (ADP-ribose) polymerase family, member 14, mRNA (cDNA clone IMAGE:5065398) | 2 |
| CcI4 | 4,71 | 4,51 | 2,7E-02 | Strain SJUJ small inducible cytokine A4 (ScyA4) | 4 |
| C4b | 4,64 | | 8,8E-03 | Complement component 4B (Childo blood group) (C4b), mRNA | 1 |
| Tap1 | 4,63 | | 6,9E-03 | Transporter 1, ATP-binding cassette, sub-family B (MDR/TAP), mRNA (cDNA clone MGC:6181 IMAGE:3257734) | 5 |
| Cd52 | 4,19 | 7,79 | 2,3E-02 | CD52 antigen, mRNA (cDNA clone MGC:40993 IMAGE:1396480) | 3 |
| Socs3 | 4,19 | 3,06 | 4,5E-02 | Suppressor of cytokine signaling 3 (Socs3), mRNA | 2 |
| H2-K1 | 4,17 | 7,92 | 3,0E-03 | MRNA similar to histocompatibility 2, D region locus 1 (cDNA clone MGC:25703 IMAGE:3675316) | 1 |
| CcI5 | 4,16 | 23,91 | 2,4E-02 | Chemokine (C-C motif) ligand 5, mRNA (cDNA clone MGC:35989 IMAGE:4925413) | 4 |
| Chi3I4 | 4,15 | | 5,6E-03 | Chitinase 3-like 4 (Chi3I4), mRNA | 1 |
| Ms4a6d | 4,07 | 18,49 | 1,5E-02 | Membrane-spanning 4-domains, subfamily A, member 6D, mRNA (cDNA clone MGC:25778 IMAGE:4016611) | |
| Emp1 | 3,81 | | 3,2E-02 | Epithelial membrane protein 1 (Emp1), mRNA | |
| H2-Ab1 | 3,08 | 7,76 | 5,3E-03 | histocompatibility 2, class II antigen A, beta 1 | 1 |
| Mx2 | 3,75 | | 1,9E-02 | Myxovirus (influenza virus) resistance 2, mRNA (cDNA clone MGC:5689 IMAGE:3591798) | 1 |
| H2-D1 | 3,19 | 4,07 | 5,9E-03 | MHC class Ib antigen Qa-1 (H2-T23) | 4 |
| Irf7 | 3,65 | 8,19 | 2,0E-03 | Interferon regulatory factor 7 (Irf7), mRNA | 1 |
| Lyz1 | 3,62 | | 1,0E-02 | Lysozyme 1 (Lyz1), mRNA | 2 |
| NIrc5 | 3,61 | 8,55 | 2,0E-03 | expressed sequence AI451557 (AI451557), mRNA. | 1 |
| Tap2 | 3,39 | 4,21 | 5,6E-03 | Transporter 2, ATP-binding cassette, sub-family B (MDR/TAP), mRNA (cDNA clone MGC:11732 IMAGE:3968225) | 4 |
| Txnip | 3,36 | | 4,1E-02 | Thioredoxin interacting protein, mRNA (cDNA clone MGC:25534 IMAGE:3591421) | 2 |
| Chi3I3 | 3,36 | | 2,6E-03 | Chitinase 3-like 3 (Chi3I3), mRNA | 1 |
| Eif2ak2 | 3,32 | 2,53 | 4,6E-03 | Eukaryotic translation initiation factor 2-alpha kinase 2, mRNA (cDNA clone MGC:11397 IMAGE:3964935) | |
| Mt2 | 3,31 | | 4,5E-02 | Metallothionein 2, mRNA (cDNA clone MGC:19383 IMAGE:2651471) | |
| Samdh9I | 3,30 | | 1,0E-02 | sterile alpha motif domain containing 9-like | 1 |
| Stat1 | 3,25 | 17,30 | 2,1E-02 | Signal transducer and activator of transcription 1, mRNA (cDNA clone MGC:6411 IMAGE:3587831) | |
| Oas1g | 3,22 | 5,59 | 2,5E-03 | 2-5 oligoadenylate synthetase 1G (Oas1g), mRNA | 1 |
| Ifit2 | 3,19 | 8,05 | 6,3E-03 | Interferon-induced protein with tetratricopeptide repeats 2 (Ifit2), mRNA | 1 |
| GIipr2 | 3,13 | 5,28 | 3,5E-02 | GLI pathogenesis-related 2, mRNA (cDNA clone MGC:28417 IMAGE:4037002) | 2 |
| Tgm2 | 3,11 | | 3,0E-02 | Transglutaminase 2, C polypeptide, mRNA (cDNA clone MGC:6152 IMAGE:3256943) | 2 |
| Icam1 | 3,10 | | 8,8E-03 | Intercellular adhesion molecule 1 (Icam1), mRNA | 2 |
| Psmb8 | 3,10 | 7,99 | 6,2E-03 | Proteasome (prosome, macropain) subunit, beta type 8 (large multifunctional peptidase 7), mRNA (cDNA clone MGC:6535 IMAGE:265 | 5 |
| Samhd1 | 3,10 | 3,93 | 5,9E-03 | SAM domain and HD domain, 1, mRNA (cDNA clone MGC:14068 IMAGE:4037046) | 3 |
| Fkbp5 | 3,07 | | 3,0E-02 | FK506 binding protein 5, mRNA (cDNA clone MGC:18417 IMAGE:4237766) | **1** |
| B2m | 3,04 | | 5,3E-03 | Beta-2 microglobulin mRNA, segment 1, clones pBRcB-(1-3). | 3 |
| Slc15a3 | 3,04 | 6,14 | 2,5E-02 | Solute carrier family 15, member 3 (Slc15a3), mRNA | 1 |
| Ifi47 | 3,01 | 8,99 | 6,2E-03 | Interferon gamma inducible protein 47, mRNA (cDNA clone MGC:11403 IMAGE:2651113) | 3 |
| Sult1a1 | 3,01 | | 1,0E-02 | Sulfotransferase family 1A, phenol-preferring, member 1 (Sult1a1), mRNA | |
| Ch25h | 3,00 | 5,86 | 1,2E-02 | Cholesterol 25-hydroxylase (Ch25h), mRNA | |
| 8430408 G22Rik | 2,91 | | 3,5E-02 | RIKEN cDNA 8430408G22 gene (8430408G22Rik), mRNA | |
| Ccl7 | 2,91 | 5,76 | 2,6E-02 | Chemokine (C-C motif) ligand 7 (Ccl7), mRNA | 1 |
| Trim21 | 2,90 | 2,87 | 2,5E-03 | Tripartite motif-containing 21, mRNA (cDNA clone MGC:6059 IMAGE:3584654) | 3 |
| Serping1 | 2,88 | | 1,9E-02 | Serine (or cysteine) peptidase inhibitor, clade G, member 1, mRNA (cDNA clone MGC:5908 IMAGE:3485810) | |
| H2-K2 | 2,86 | | 9,0E-03 | LOC56628 | 1 |
| H2-T22 | 2,85 | 4,82 | 7,6E-03 | Histocompatibility 2, T region locus 10, mRNA (cDNA clone MGC:25390 IMAGE:4165944) | 3 |
| Map3k6 | 2,85 | | 3,3E-02 | Mitogen-activated protein kinase kinase kinase 6 (Map3k6), mRNA | |
| D14Ertd668e | 2,83 | | 6,0E-03 | DNA segment, Chr 14, ERATO Doi 668, expressed, mRNA (cDNA clone MGC:29273 IMAGE:5067268) | 1 |
| - | 2,81 | | 1,6E-02 | RIKEN cDNA 1200016E24 gene | |
| Tagln2 | 2,81 | | 2,0E-02 | Transgelin | |
| Fpr2 | 2,80 | 10,17 | 2,1E-02 | Formyl peptide receptor 2 (Fpr2), mRNA | 2 |
| Irf9 | 2,80 | 2,15 | 1,3E-02 | Interferon regulatory factor 9, mRNA (cDNA clone MGC:13985 IMAGE:3257714) | 1 |
| Pglyrp1 | 2,80 | 3,50 | 1,8E-02 | Peptidoglycan recognition protein 1, mRNA (cDNA clone MGC:11430 IMAGE:3969014) | |
| H2-Q2 | 2,78 | | 1,4E-02 | Histocompatibility 2, Q region locus 2 (H2-Q2), mRNA | 2 |
| AngptI4 | 2,71 | | 2,9E-02 | Angiopoietin-like 4, mRNA (cDNA clone MGC:35885 IMAGE:5137159) | 1 |
| Fcer1g | 2,71 | 5,57 | 2,1E-02 | Fc receptor, IgE, high affinity I, gamma polypeptide, mRNA (cDNA clone MGC:36077 IMAGE:5065647) | 1 |
| Ly6a | 2,68 | | 7,5E-03 | Lymphocyte antigen 6 complex, locus A, mRNA (cDNA clone MGC:6188 IMAGE:3486025) | **1** |
| Upp1 | 2,65 | 4,24 | 2,2E-02 | Uridine phosphorylase 1, mRNA (cDNA clone MGC:41205 IMAGE:5144694) | 1 |
| Ube1l | 2,64 | 2,60 | 1,1E-02 | Ubiquitin-activating enzyme E1-like, mRNA (cDNA clone IMAGE:4013998) | 1 |
| Oasl1 | 2,63 | 3,93 | 2,2E-02 | Oligoadenylate synthetase-like protein-2 | |
| Ubd | 2,63 | 259,08 | 5,3E-03 | Ubiquitin D, mRNA (cDNA clone MGC:41063 IMAGE:1347593) | 1 |
| H2-L | 2,59 | | 2,7E-02 | H2-L | 1 |
| Tspo | 2,54 | | 1,6E-02 | Translocator protein, mRNA (cDNA clone MGC:6086 IMAGE:3493196) | 1 |
| H2-Q7 | 2,52 | | 8,8E-03 | Histocompatibility 2, Q region locus 7 (H2-Q7), mRNA | 2 |
| Trim25 | 2,51 | 2,40 | 2,2E-02 | tripartite motif-containing 25 | 1 |
| Anxa2 | 2,49 | | 3,3E-02 | Annexin A2, mRNA (cDNA clone MGC:6547 IMAGE:2655513) | 1 |
| Bcl2a1d | 2,48 | | 5,0E-03 | B-cell leukemia/lymphoma 2 related protein A1d, mRNA (cDNA clone MGC:41219 IMAGE:1363928) | 1 |
| Ifi205 | 2,47 | 8,65 | 2,5E-03 | (strain C57BI/6) mRNA sequence | 1 |
| 2410039 M03Rik | 2,41 | | 4,6E-02 | 2410039M03Rik | |
| H2-Eb1 | 2,41 | 4,78 | 2,5E-03 | Histocompatibility 2, class II antigen E beta (H2-Eb1), mRNA | |
| Osmr | 2,39 | | 2,7E-02 | Oncostatin M receptor (Osmr), mRNA | |
| Mobp | 2,37 | | 4,0E-02 | Myelin-associated oligodendrocytic basic protein (Mobp), transcript variant 1, mRNA | 1 |
| Bcl2a1b | 2,35 | 2,63 | 1,3E-02 | B-cell leukemia/lymphoma 2 related protein A1a, mRNA (cDNA clone MGC:41220 IMAGE:1226745) | 1 |
| Bst2 | 2,33 | | 2,0E-03 | Bone marrow stromal cell antigen 2, mRNA (cDNA clone MGC:28276 IMAGE:4009434) | 1 |
| Cyba | 2,29 | 4,38 | 3,5E-02 | Cytochrome b-245, alpha polypeptide (Cyba), mRNA | 1 |
| Pnpla2 | 2,28 | | 2,9E-02 | Patatin-like phospholipase domain containing 2, mRNA (cDNA clone IMAGE:4982482) | |
| Batf2 | 2,26 | | 4,6E-03 | Basic leucine zipper transcription factor, ATF-like 2, mRNA (cDNA clone MGC:37488 IMAGE:4984403) | 1 |
| Saa3 | 2,26 | 155,32 | 4,1E-02 | Serum amyloid A 3 (Saa3), mRNA | |
| Cmpk2 | 2,24 | 3,44 | 3,1E-03 | Cytidine monophosphate (UMP-CMP) kinase 2, mitochondrial (Cmpk2), nuclear gene encoding mitochondrial protein, mRNA | 3 |
| Itgad | 2,23 | | 4,1E-02 | integrin, alpha D | |
| Cenpa | 2,20 | 4,28 | 1,1E-02 | Centromere protein A, mRNA (cDNA clone MGC:13888 IMAGE:4018429) | |
| Phyhd1 | 2,19 | | 4,0E-02 | Phytanoyl-CoA dioxygenase domain containing 1, mRNA (cDNA clone MGC:178922 IMAGE:9053914) | |
| H2-T17 | 2,18 | | 1,4E-02 | H2-T17 | 3 |
| Gm12250 | 2,16 | | 3,0E-02 | LOC215405 | 2 |
| Xdh | 2,16 | | 1,3E-02 | Similar to hypothetical protein MGC37588, mRNA (cDNA clone MGC:28125 IMAGE:3980327) | 4 |
| Lgals9 | 2,15 | | 2,4E-03 | Lectin, galactose binding, soluble 9, mRNA (cDNA clone MGC:5882 IMAGE:3601419) | |
| Fcgr3 | 2,14 | 7,42 | 5,4E-03 | Fc gamma receptor III (Fcgr3) mRNA, Fcgr3-b allele | |
| Ier3 | 2,14 | | 4,8E-02 | Immediate early response 3 (Ier3), mRNA | 2 |
| Adamts9 | 2,12 | | 2,2E-02 | Adamts9 | |
| Ifi27l1 | 2,10 | | 6,2E-03 | Interferon, alpha-inducible protein 27 like 1, mRNA (cDNA clone MGC:149996 IMAGE:40091489) | |
| Ctsc | 2,07 | 5,59 | 8,8E-03 | Cathepsin C (Ctsc), mRNA | 1 |
| Gvin1 | 2,06 | | 1,6E-02 | GTPase, very large interferon inducible 1 (Gvin1), transcript variant B, mRNA | 1 |
| Ugt1a6a | 2,05 | | 7,6E-03 | UDP glucuronosyltransferase 1 family, polypeptide A6B, mRNA (cDNA clone MGC:36247 IMAGE:5050488) | 2 |
| Arpc1b | 2,04 | | 3,5E-02 | Actin related protein 2/3 complex, subunit 1B, mRNA (cDNA clone MGC:8155 IMAGE:3589768) | |
| C1qb | 2,02 | 13,98 | 2,5E-02 | Complement component 1, q subcomponent, beta polypeptide (C1qb), mRNA | 2 |
| | | | | | |
| Atxn7I3b | -2,00 | | 2,3E-02 | predicted gene, ENSMUSG00000074747 | |
| E2f6 | -2,00 | | 3,4E-02 | E2F transcription factor 6, mRNA (cDNA clone MGC:46747 IMAGE:5358554) | |
| Nckap1 | -2,00 | | 6,2E-03 | NCK-associated protein 1, mRNA (cDNA clone IMAGE:3488144) | 1 |
| Pcyt2 | -2,00 | | 2,5E-02 | Phosphate cytidylyltransferase 2, ethanolamine, mRNA (cDNA clone MGC:11578 IMAGE:3707732) | |
| Tob1 | -2,00 | | 3,1E-02 | Transducer of ErbB-2.1 (Tob1), mRNA | |
| Abcf2 | -2,01 | | 4,6E-02 | ATP-binding cassette, sub-family F (GCN20), member 2 (Abcf2), nuclear gene encoding mitochondrial protein, mRNA | 1 |
| Hcn3 | -2,01 | | 7,4E-03 | Hyperpolarization-activated, cyclic nucleotide-gated K+ 3 (Hcn3), mRNA | |
| Mus81 | -2,01 | | 1,8E-02 | MUS81 endonuclease homolog (yeast), mRNA (cDNA clone MGC:36246 IMAGE:5038349) | |
| Ncoa1 | -2,01 | | 1,6E-02 | Nuclear receptor coactivator 1 (Ncoa1), mRNA | |
| Ntrk3 | -2,01 | | 3,0E-02 | Neurotrophic tyrosine kinase, receptor, type 3 (Ntrk3), transcript variant 1, mRNA | |
| Palm | -2,01 | | 1,3E-02 | Paralemmin, mRNA (cDNA clone MGC:19169 IMAGE:4223845) | |
| Pbrm1 | -2,01 | | 2,5E-02 | polybromo 1 | |
| Strbp | -2,01 | -2,17 | 3,3E-02 | RIKEN cDNA 6430510M02 gene (6430510M02Rik), mRNA. | |
| Arl8b | -2,02 | 2,23 | 1,1E-02 | ADP-ribosylation factor-like 8B (Arl8b), mRNA | 1 |
| D17Wsu92e | -2,02 | | 1,4E-02 | DNA segment, Chr 17, Wayne State University 92, expressed | 1 |
| Gria2 | -2,02 | | 1,5E-02 | Glutamate receptor, ionotropic, AMPA2 (alpha 2) (Gria2), transcript variant 2, mRNA | |
| Lonrf2 | -2,02 | | 2,9E-02 | LON peptidase N-terminal domain and ring finger 2, mRNA (cDNA clone MGC:170754 IMAGE:8862149) | |
| Pltp | -2,02 | -2,51 | 3,0E-02 | Phospholipid transfer protein, mRNA (cDNA clone MGC:6006 IMAGE:3491360) | 2 |
| Myt1l | -2,03 | | 6,1E-03 | Myelin transcription factor 1-like (Myt1l), transcript variant 2, mRNA | |
| Oxct1 | -2,03 | | 8,7E-03 | Scot mRNA for succinyl CoA transferase | 1 |
| Taok1 | -2,03 | | 2,8E-02 | TAO kinase 1, mRNA (cDNA clone IMAGE:1380120) | |
| Zfp385b | -2,03 | | 4,6E-03 | Zinc finger protein 385B, mRNA (cDNA clone MGC:169863 IMAGE:8861258) | |
| 6330407J 23Rik | -2,04 | | 2,9E-03 | RIKEN cDNA 6330407J23 gene (6330407J23Rik), mRNA | |
| Apba2 | -2,04 | | 2,5E-03 | X11 protein mRNA, 3 end | |
| Fam126b | -2,04 | | 1,1E-02 | Family with sequence similarity 126, member B, mRNA (cDNA clone MGC:76460 IMAGE:30431671) | 1 |
| Sdr39u1 | -2,04 | | 1,6E-02 | Short chain dehydrogenase/reductase family 39U, member 1 (Sdr39u1), mRNA | 1 |
| 1190002N15Rik | -2,05 | | 2,1E-02 | RIKEN cDNA 1190002N15 gene | |
| Atxn7l3 | -2,05 | | 2,3E-02 | ataxin 7-like 3 | |
| Cyp46a1 | -2,05 | | 2,0E-02 | Cytochrome P450, family 46, subfamily a, polypeptide 1, mRNA (cDNA clone MGC:18311 IMAGE:4195579) | |
| Gna11 | -2,05 | | 1,5E-02 | Guanine nucleotide binding protein, alpha 11, mRNA (cDNA clone MGC:18562 IMAGE:4206878) | |
| Ppp5c | -2,05 | | 1,9E-02 | Protein phosphatase 5, catalytic subunit, mRNA (cDNA clone MGC:5847 IMAGE:3590322) | |
| Ptn | -2,05 | | 1,7E-02 | Pleiotrophin (Ptn), mRNA | |
| Sept3 | -2,05 | | 2,6E-02 | Septin 3 (Sept3), mRNA | |
| Thra | -2,05 | -2,12 | 8,8E-03 | Thra | |
| Zbtb44 | -2,05 | | 6,2E-03 | BC038156 | |
| Hist1h2bf | -2,06 | | 1,9E-02 | Histone cluster 1, H2bf (Hist1 h2bf), mRNA | |
| Ppp1r35 | -2,06 | | 3,0E-02 | RIKEN cDNA 2010007H12 gene, mRNA (cDNA clone MGC:62925 IMAGE:1429227) | |
| Ccm2 | -2,07 | | 1,6E-02 | Cerebral cavernous malformation 2 homolog (human) (Ccm2), mRNA | 2 |
| Glg1 | -2,07 | | 1,3E-02 | Golgi apparatus protein 1, mRNA (cDNA clone MGC:29292 IMAGE:4239405) | |
| Tmco3 | -2,07 | | 6,3E-03 | Transmembrane and coiled-coil domains 3, mRNA (cDNA clone IMAGE:3967158) | 1 |
| Zfp523 | -2,07 | | 8,7E-03 | PREDICTED: Mus musculus similar to collagen, type VIII, alpha 2 (LOC100042764), mRNA | 1 |
| - | -2,08 | | 3,0E-02 | LOC385086 | |
| Carm1 | -2,08 | | 1,3E-02 | Coactivator-associated arginine methyltransferase 1, mRNA (cDNA clone MGC:46828 IMAGE:4935077) | |
| Gtrgeo22 | -2,08 | | 3,5E-02 | Gene trap ROSA b-geo 22 (Gtrgeo22), mRNA | 1 |
| Pacsin1 | -2,08 | | 3,8E-03 | Protein kinase C and casein kinase substrate in neurons 1, mRNA (cDNA clone MGC:25285 IMAGE:4527708) | |
| Pde6d | -2,08 | | 7,6E-03 | Phosphodiesterase 6D, cGMP-specific, rod, delta, mRNA (cDNA clone MGC:11435 IMAGE:3964336) | |
| Rnf167 | -2,08 | | 4,0E-02 | Ring finger protein 167, mRNA (cDNA clone MGC:18686 IMAGE:4241357) | 1 |
| Tmem63b | -2,08 | | 1,2E-02 | Transmembrane protein 63b, mRNA (cDNA clone IMAGE:4160190) | |
| Nfix | -2,09 | -2,02 | 1,3E-02 | Nuclear factor I/X, mRNA (cDNA clone MGC:5944 IMAGE:3491917) | |
| Caln1 | -2,09 | | 3,1E-02 | Calneuron 1 (Caln1) | |
| Dgkb | -2,09 | | 8,7E-03 | Diacylglycerol kinase, beta, mRNA (cDNA clone MGC:99855 IMAGE:30649561) | |
| Mbtps1 | -2,09 | | 2,9E-02 | Membrane-bound transcription factor peptidase, site 1 (Mbtps1), mRNA | |
| Rora | -2,09 | | 3,0E-02 | RAR-related orphan receptor alpha, mRNA (cDNA clone MGC:5892 IMAGE:3592667) | |
| Scrib | -2,09 | | 1,7E-02 | Scribbled homolog (Drosophila), mRNA (cDNA clone IMAGE:4459388) | |
| Shank3 | -2,09 | -3,49 | 3,8E-03 | SH3/ankyrin domain gene 3 (Shank3), mRNA | |
| Bcl11b | -2,10 | 5,31 | 8,8E-03 | B-cell leukemia/lymphoma 11B, mRNA (cDNA clone MGC:27524 IMAGE:4457123) | |
| Cops7a | -2,10 | | 1,3E-02 | COP9 (constitutive photomorphogenic) homolog, subunit 7a (Arabidopsis thaliana), mRNA (cDNA clone MGC:5772 IMAGE:3593979) | |
| Fam178a | -2,10 | | 1,4E-02 | family with sequence similarity 178, member A | |
| Foxq1 | -2,10 | | 8,8E-03 | Forkhead box Q1 (Foxq1), mRNA | |
| Rhobtb2 | -2,10 | | 1,6E-02 | Rho-related BTB domain containing 2 (Rhobtb2), mRNA | 1 |
| Zfp612 | -2,10 | | 8,8E-03 | Zinc finger protein 612, mRNA (cDNA clone IMAGE:3586510) | |
| Eif5a | -2,11 | | 1,6E-02 | Eukaryotic translation initiation factor 5A, mRNA (cDNA clone MGC:25474 IMAGE:4482804) | 1 |
| Pak1 | -2,11 | 3,15 | 5,9E-03 | P21 (CDKN1A)-activated kinase 1 (Pak1), mRNA | |
| Tspan3 | -2,11 | | 3,6E-02 | Tspan-3 mRNA for tetraspanin | |
| 1110012J17Rik | -2,12 | -2,39 | 9,3E-03 | RIKEN cDNA 1110012J17 gene (1110012J17Rik), transcript variant 1, mRNA | |
| Dgkz | -2,12 | | 1,8E-02 | Diacylglycerol kinase zeta, mRNA (cDNA clone IMAGE:2650291) | |
| Gnao1 | -2,12 | | 2,8E-02 | Guanine nucleotide binding protein, alpha O (Gnao1), transcript variant A, mRNA | |
| Hsd3b2 | -2,12 | | 2,7E-02 | Hydroxy-delta-5-steroid dehydrogenase, 3 beta- and steroid delta-isomerase 2 (Hsd3b2), mRNA | |
| Ntm | -2,13 | | 1,5E-02 | Neurotrimin, mRNA (cDNA clone MGC:30504 IMAGE:4480983) | |
| Slc38a9 | -2,13 | | 2,3E-02 | Solute carrier family 38, member 9 (Slc38a9), mRNA | 1 |
| Cd47 | -2,14 | | 1,6E-02 | CD47 antigen (Rh-related antigen, integrin-associated signal transducer), mRNA (cDNA clone MGC:13838 IMAGE:4187965) | 1 |
| Gats | -2,14 | | 2,2E-02 | Opposite strand transcription unit to Stag3 (Gats), mRNA | |
| Rab5b | -2,14 | | 1,3E-02 | RAB5B, member RAS oncogene family (Rab5b), transcript variant 2, mRNA | |
| Fam63b | -2,15 | | 6,5E-03 | MKIAA1164 protein | 1 |
| Fcrls | -2,15 | -2,29 | 7,4E-03 | IFGP2 | |
| Pea15a | -2,15 | | 3,9E-02 | Phosphoprotein enriched in astrocytes 15A, mRNA (cDNA clone MGC:47406 IMAGE:4500957) | 1 |
| Skiv2l | -2,15 | | 2,9E-02 | Superkiller viralicidic activity 2-like (S. cerevisiae), mRNA (cDNA clone IMAGE:5007559) | |
| Usf2 | -2,15 | | 4,0E-02 | Upstream transcription factor 2, mRNA (cDNA clone IMAGE:3969222) | 3 |
| Jph4 | -2,16 | | 1,6E-02 | Junctophilin 4 (Jph4), transcript variant b, mRNA | |
| Klhdc1 | -2,16 | | 7,6E-03 | Kelch domain containing 1, mRNA (cDNA clone MGC:141301 IMAGE:40057794) | |
| Ltbp4 | -2,16 | -4,99 | 1,5E-02 | Latent transforming growth factor beta binding protein 4 long splice variant (Ltbp4) mRNA, complete cds, alternatively splice | |
| Zbtb7a | -2,16 | | 2,5E-03 | Zinc finger and BTB domain containing 7a (Zbtb7a), mRNA | |
| Akap1 | -2,17 | | 3,6E-02 | A kinase (PRKA) anchor protein 1 (Akap1), nuclear gene encoding mitochondrial protein, transcript variant 1, mRNA | 1 |
| Gtf3a | -2,17 | | 7,6E-03 | General transcription factor III A, mRNA (cDNA clone MGC:40923 IMAGE:5374268) | |
| Mau2 | -2,17 | | 1,6E-02 | RIKEN cDNA 9130404D08 gene (9130404D08Rik), mRNA | |
| Rasgef1a | -2,17 | -2,06 | 6,6E-03 | RasGEF domain family, member 1A | |
| Slc40a1 | -2,17 | | 1,6E-02 | Solute carrier family 40 (iron-regulated transporter), member 1, mRNA (cDNA clone MGC:6489 IMAGE:2647365) | |
| Klf7 | -2,18 | | 2,3E-02 | Transcribed locus, strongly similar to NP_ 003700.1 Kruppel-like factor 7 (ubiquitous) [Homo sapiens] | |
| Camkk2 | -2,18 | | 2,6E-03 | Calcium/calmodulin-dependent protein kinase kinase 2, beta (Camkk2), mRNA | 1 |
| Fam171b | -2,18 | | 1,1E-02 | Family with sequence similarity 171, member B, mRNA (cDNA clone IMAGE:4501762) | |
| Kalrn | -2,18 | | 6,3E-03 | 2210407G14Rik | |
| Scamp3 | -2,18 | | 7,6E-03 | CDC-like kinase 2, mRNA (cDNA clone MGC:13872 IMAGE:3995512) | |
| Mzt1 | -2,19 | | 3,0E-02 | RIKEN cDNA 2410129H14 gene, mRNA (cDNA clone MGC:151382 IMAGE:40126324) | |
| Phldb1 | -2,19 | | 2,5E-03 | Pleckstrin homology-like domain, family B, member 1 (Phldb1), mRNA | |
| Sgtb | -2,19 | | 2,0E-02 | Small glutamine-rich tetratricopeptide repeat (TPR)-containing, beta (Sgtb), mRNA | |
| Rnd2 | -2,20 | | 2,7E-02 | Rho family GTPase 2 (Rnd2), mRNA | 1 |
| Tcf4 | -2,20 | -2,12 | 7,6E-03 | Transcription factor 4, mRNA (cDNA clone MGC:13998 IMAGE:4014231) | |
| Celsr2 | -2,21 | | 2,0E-02 | Cadherin, EGF LAG seven-pass G-type receptor 2 (flamingo homolog, Drosophila), mRNA (cDNA clone IMAGE:3488511) | |
| Ptprd | -2,21 | -2,54 | 1,4E-02 | protein tyrosine phosphatase, receptor type, D | |
| 3110047P20Rik | -2,22 | | 2,2E-02 | RIKEN cDNA 3110047P20 gene | |
| Dlgap1 | -2,22 | | 8,8E-03 | Discs, large (Drosophila) homolog-associated protein 1 (Dlgap1), transcript variant 2, mRNA | |
| Mgat4b | -2,22 | | 1,6E-02 | Mannoside acetylglucosaminyltransferase 4, isoenzyme B (Mgat4b), mRNA | 3 |
| Usp11 | -2,22 | | 3,1E-02 | Ubiquitin specific peptidase 11 (Usp11), mRNA | |
| Fbxo41 | -2,23 | | 9,8E-03 | F-box protein 41 (Fbxo41), mRNA | |
| B4galt3 | -2,24 | | 1,6E-02 | UDP-Gal:betaGlcNAc beta 1,4-galactosyltransferase, polypeptide 3, mRNA (cDNA clone MGC:11711 IMAGE:3965561) | 1 |
| Fjx1 | -2,24 | | 3,4E-02 | Four jointed box 1 (Drosophila) (Fjx1), mRNA | |
| Gga3 | -2,24 | | 6,1E-03 | Golgi associated, gamma adaptin ear containing, ARF binding protein 3, mRNA (cDNA clone IMAGE:6477214) | |
| Sema6d | -2,24 | -2,23 | 1,4E-02 | Sema domain, transmembrane domain (TM), and cytoplasmic domain, (semaphorin) 6D (Sema6d), transcript variant 5, mRNA | |
| Smpd1 | -2,24 | | 2,9E-02 | Sphingomyelin phosphodiesterase 1, acid lysosomal, mRNA (cDNA clone MGC:25355 IMAGE:4482098) | |
| Rbfox1 | -2,25 | | 1,3E-02 | Hexaribonucleotide binding protein 1 (Hrnbp1) | 1 |
| Arid1a | -2,25 | | 7,6E-03 | AT rich interactive domain 1A (SWI-like) | |
| Bean | -2,25 | | 4,6E-03 | Brevican (Bcan), transcript variant 1, mRNA | |
| Tmod1 | -2,25 | -2,04 | 3,1E-02 | Tropomodulin 1 (Tmod1), mRNA | |
| Rab14 | -2,26 | | 1,0E-02 | RAB14, member RAS oncogene family, mRNA (cDNA clone MGC:36272 IMAGE:3980228) | 2 |
| Brunol4 | -2,27 | | 1,1E-02 | BRUL4 (Brul4) | |
| Mettl17 | -2,27 | | 1,3E-02 | D14Ertd209e | |
| Slco1c1 | -2,27 | | 2,0E-02 | Solute carrier organic anion transporter family, member 1c1 (Slco1c1), mRNA | |
| Tbc1d17 | -2,27 | | 5,3E-03 | TBC1 domain family, member 17 | |
| Acot7 | -2,28 | | 3,7E-02 | BACH mRNA for acyl-CoA hydrolase, complete cds, isoform mBACHb | 1 |
| Efna5 | -2,28 | | 1,5E-02 | Ephrin A5 (Efna5), transcript variant 2, mRNA | |
| Ank2 | -2,29 | | 2,3E-02 | Ankyrin 2, brain (Ank2), transcript variant 3, mRNA | |
| | | | | | |
| C1qtnf4 | -2,29 | | 5,3E-03 | C1q and tumor necrosis factor related protein 4, mRNA (cDNA clone IMAGE:3668760) | |
| Orf61 | -2,29 | | 9,0E-03 | open reading frame 61 | |
| AI593442 | -2,30 | | 1,5E-02 | expressed sequence AI593442 | |
| Jhdm1d | -2,30 | 2,03 | 2,2E-02 | jumonji C domain-containing histone demethylase 1 homolog D (S. cerevisiae) | 1 |
| Spnb4 | -2,30 | | 2,0E-02 | BetaIV-spectrin sigma1 | 2 |
| Acvr2b | -2,32 | | 6,3E-03 | Activin receptor IIB (Acvr2b), mRNA | |
| Elavl3 | -2,39 | | 5,3E-03 | RNA-binding protein mHuC-S | |
| Rab6 | -2,34 | | 1,2E-02 | RAB6, member RAS oncogene family, mRNA (cDNA clone IMAGE:3491845) | |
| Pak3 | -2,35 | | 1,1E-02 | P21-activated kinase 3 (pak3 gene) | |
| Dusp8 | -2,38 | | 1,9E-02 | Dual specificity phosphatase 8 (Dusp8), mRNA | |
| Tnrc6a | -2,38 | | 1,1E-02 | Trinucleotide repeat containing 6a (Tnrc6a), mRNA | |
| Arl2bp | -2,39 | | 5,6E-03 | ADP-ribosylation factor-like 2 binding protein (Arl2bp), transcript variant 1, mRNA | |
| Mtss1l | -2,39 | | 2,9E-02 | Metastasis suppressor 1-like (Mtss1l), mRNA | 1 |
| Cacng3 | -2,40 | | 1,2E-02 | Calcium channel, voltage-dependent, gamma subunit 3 (Cacng3), mRNA | 1 |
| Ppp1ca | -2,40 | | 2,3E-02 | Protein phosphatase 1, catalytic subunit, alpha isoform, mRNA (cDNA clone MGC:25955 IMAGE:4239005) | |
| Rtn1 | -2,40 | | 8,8E-03 | Reticulon 1 (Rtn1), transcript variant 1, mRNA | |
| Gabrb1 | -2,41 | | 1,6E-02 | Gamma-aminobutyric acid (GABA-A) receptor, subunit beta 1 (Gabrb1), mRNA | |
| D14Abb1e | -2,41 | | 9,7E-03 | D14Abb1e | |
| Rgs7bp | -2,42 | | 1,5E-02 | Regulator of G-protein signalling 7 binding protein, mRNA (cDNA clone MGC:143795 IMAGE:40093423) | |
| Syt4 | -2,42 | | 6,6E-03 | Synaptotagmin IV (Syt4), mRNA | |
| Ugt8a | -2,44 | | 5,0E-02 | UDP galactosyltransferase 8A, mRNA (cDNA clone MGC:18397 IMAGE:4223057) | |
| E330009J07Rik | -2,45 | | 3,6E-02 | RIKEN cDNA E330009J07 gene (E330009J07Rik), mRNA | |
| Gjc2 | -2,45 | | 2,5E-03 | Gap junction protein, gamma 2 (Gjc2), transcript variant 1, mRNA | 1 |
| Kif3a | -2,46 | | 7,6E-03 | Kinesin family member 3A (Kif3a), mRNA | |
| Nfib | -2,48 | -5,75 | 6,2E-03 | Strain C57BL/6J nuclear factor I/B (Nfib) | |
| Unc13c | -2,48 | | 3,5E-03 | LOC235480 | |
| Atp6v0d1 | -2,49 | | 2,7E-02 | ATPase, H+ transporting, lysosomal V0 subunit D1, mRNA (cDNA clone MGC:18332 IMAGE:3662404) | |
| Prrt1 | -2,50 | | 1,7E-02 | Proline-rich transmembrane protein 1 (Prrt1), mRNA | 1 |
| 4930402H24Rik | -2,50 | -2,44 | 1,6E-02 | RIKEN cDNA 4930402H24 gene, mRNA (cDNA clone IMAGE:5366525) | |
| Hes5 | -2,53 | | 1,1E-02 | Hairy and enhancer of split 5 (Drosophila) (Hes5), mRNA | |
| Mark2 | -2,59 | | 1,3E-02 | MAP/microtubule affinity-regulating kinase 2 (Mark2), transcript variant 1, mRNA | |
| Msl1 | -2,59 | | 8,8E-03 | Male-specific lethal 1 homolog (Drosophila) (Msl1), mRNA | |
| Gdi1 | -2,60 | | 3,1E-02 | Guanosine diphosphate (GDP) dissociation inhibitor 1, mRNA (cDNA clone MGC:47005 IMAGE:5249271) | |
| Slc38a2 | -2,64 | | 2,1E-02 | Solute carrier family 38, member 2, mRNA (cDNA clone IMAGE:4164084) | 1 |
| Psd2 | -2,68 | | 1,3E-02 | Pleckstrin and Sec7 domain containing 2 (Psd2), mRNA | |
| Ppap2b | -2,69 | -2,65 | 1,5E-02 | Phosphatidic acid phosphatase type 2B (Ppap2b), mRNA | 1 |
| Grm4 | -2,75 | | 2,9E-02 | glutamate receptor, metabotropic 4 | |
| Epn2 | -2,79 | -2,06 | 2,1E-02 | Epsin 2, mRNA (cDNA clone MGC:19376 IMAGE:2647379) | |
| 2210018M11Rik | -2,83 | | 1,0E-02 | 2210018M11Rik | |
| Lphn1 | -2,85 | -2,17 | 1,6E-02 | latrophilin 1 | |
| Flt1 | -2,89 | -2,08 | 5,6E-03 | FMS-like tyrosine kinase 1, mRNA (cDNA clone MGC:36074 IMAGE:5368921) | |
| Slc38a5 | -2,95 | -3,45 | 2,3E-02 | Solute carrier family 38, member 5, mRNA (cDNA clone MGC:173142 IMAGE:40057282) | |
| Alas2 | -3,04 | -2,56 | 2,9E-02 | Aminolevulinic acid synthase 2, erythroid, mRNA (cDNA clone IMAGE:5054102) | |
| Itm2a | -3,05 | | 5,9E-03 | Integral membrane protein 2A, mRNA (cDNA MGC:18323 IMAGE:3668557) | |
| Tia1 | -3,07 | | 1,5E-02 | cytotoxic granule-associated RNA binding protein 1 | 1 |
| Prkcb | -3,21 | 2,70 | 2,0E-02 | Protein kinase C, beta (Prkcb), mRNA | 1 |
| Cxcl12 | -4,18 | 2,58 | 1,4E-02 | Chemokine (C-X-C motif) ligand 12, mRNA (cDNA clone MGC:6119 IMAGE:3483088) | |
| Hbb-b1 | -23,47 | | 2,1E-02 | Hemoglobin, beta adult minor chain, mRNA (cDNA clone MGC:40691 IMAGE:3988455) | |
| Hba-a1 | -24,73 | | 2,9E-02 | Hemoglobin alpha, adult chain 1 (Hba-a1), mRNA | |

Finally, to validate the role of the identified IRF8 targets and associated pathways in innate immunity and pathological neuroinflammation, the susceptibility to PbA infection was phenotyped in mouse strains bearing null mutations at several of these loci. These included infection-regulated genes bearing IRF8 binding sites (Irf1, Ifit1, Isg15, Irgm1 and Nlrc4), and other genes known to play key roles in early innate immune response (Ifng, Jak3, Stat1, Il12p40). Results from these experiments (Figure 7) show that IFNγ^{-l-} , JAK3^{-/-} and STAT1^{-/-} mutant mice were completely resistant to *P. berghei* infection and did not develop CM, highlighting key roles for these molecules in the progression or amplification of the pathological inflammatory response. Loss Irf1 and Irgm1 delayed appearance of neurological symptoms and prolonged survival of PbA infected mice. These results indicate that several of the transcriptional targets of IRF8 activated during PbA infection in lymphoid and myeloid cells play a critical role in neuroinflammation. On the other hand IL12p40^{-/-}, IFIT1^{-/-}, ISG15^{-/-} and NLRC4^{-/-} mutant mice remained susceptible to PbA-induced CM, suggesting that although these proteins may play important roles in neuroinflammation, their deletion is not sufficient to induce protection.

The demonstrated role of IRF8 in the ontogeny of myeloid cells, its known role in defense against infectious pathogens and the growing body of evidence from GWAS studies in humans linking IRF8 variants to chronic autoimmune inflammatory conditions such as multiple sclerosis, systemic lupus erythmatous and Crohn's disease prompted the investigation of a possible role for IRF8 in acute pathological inflammatory reactions. For this, a mouse model of acute encephalitis caused by infection with P. *berghei* (cerebral malaria) was used, which involves lethal neuroinflammation caused by recruitment of inflammatory mononuclear and polynuclear leukocytes, and ensuing loss of integrity of the blood brain barrier (BBB). It was found that the loss of Irf8 in BXH2 mice completely protects against this pathology, preventing the development of neurological symptoms and prolonging survival post infection. Interestingly, the protective effect was inherited in a co-dominant fashion as 50% of Irf8^{R294C/+} F1 heterozygotes survived through the cerebral phase when infected with PbA (Figure 3A). These finding establish that IRF8 is critical to the development of acute lethal neuroinflammation associated with experimental cerebral malaria and further implicate Irf8 as a major regulator of this pathological response. Moreover, results from Irf8^{R294C/+} F1 heterozygotes indicate that Irf8 regulates key pro-inflammatory cells and pathways in a gene dosage dependent fashion.

In addition to its established role in ontogeny and function of myeloid cells, Irf8 is also required for certain aspects of B lymphocytes development and of T lymphocytes function (Th1, Th17 response). To identify the cell types and gene-dosage dependent pathways that are activated by IRF8 during neuroinflammation, brain transcripts profiles from PbA-infected B6 and BXH2 mice were compared to and extracted a list of genes that are induced by infection in an Irf8-dependent and independent fashion (2 X 2 ANOVA and pairwise analysis) (Figure 5, Table 1). In parallel, ChIP-seq experiments we carried out to map genome-wide IRF8 binding sites. We compared these positions to the gene lists generated by transcript profiling and identified both IRF8-bound genes (Table 2), and IRF8 bound genes regulated in an Irf8-allele specific fashion (Table 1). There was substantial overlap between these gene sets, which were similarly dominated by markers and pathways characteristic of antigen-presenting cells (APC), including antigen processing and presentation, production of type I interferon, production of pro-inflammatory cytokines/chemokines and others. These combined analyses confirm that IRF8 plays a prominent role in the unique functions of APCs including antigen capture and microbial phagocytosis (C1q, C4b, Fcgr4, Fcgr1), cytoplasmic inflammasome platforms such as Nlrc5, and Ifi205; phagosome maturation including recruitment of key small GTPases (Irgm1, Irgm2, Igtp, Gbp2, Gbp3), endoplasmic reticulum membrane associated antigen transport (Tap1, Tap2), Class I and Class II MHC-dependent antigen presentation in APCs (B2m, H2-A, D, K, L, Q, T molecules). These gene lists also featured a number of inflammatory cytokine and chemokines involved in chemotaxis of myeloid and lymphoid cell types to the sites of infection (Ccl4, Ccl5, Ccl7, Ccl12, Cxcl9, Cxcl10). These findings are compatible with a simple functional model where myeloid cells (including APCs) are rapidly recruited in large numbers to the site of *P. berghei* infection and associated-tissue injury, namely capillaries of the blood brain barrier. This initiates a robust IRF8-dependent pro-inflammatory cascade. Local amplification of this response by recruited cells leads to excessive production of immunopathological soluble mediators such as IFNγ and TNFα by T lymphocytes and induces other transcription factors including Stat1 and other IRF family members (Irf1, Irf7, Irf9). Absence of IRF8 blunts this pathological response and allows mutant BXH2 mice to avoid developing neuroinflammation during CM, thus surviving the critical acute phase.

Although the severe depletion of dendritic cells and macrophages, along with a concomitant reduction in IL12 production and antigen-specific T-cell priming in BXH2 is likely to account for an important component of CM-resistance, it is proposed that even in the context of normal myeloid cell numbers, reduced IRF8-dependent transcriptional activation of APC-specific pathways is sufficient to significantly blunt inflammatory response and protect against acute pathological inflammation. This is based on three main observations. First, IRF8 behaves primarily as a transcriptional activator, not a repressor, in myeloid cells as can be seen by the enrichment of IRF8 binding sites in up-regulated genes only (Figure 6B). Table 1 also highlights that for each gene regulated in a strain and infection specific way, IRF8 competent mice invariably show a higher magnitude fold change than BXH2, and the majority of these genes are up-regulated (Group 1 & 2) rather than downregulated (Group 3). Second, Irf8^{R294C/+} F1 heterozygotes show normal numbers of myeloid cells (DCs, macrophages) and lymphoid cells (data not shown), but still display significant resistance to *P. berghei* induced CM (Figure 3A). Thirdly, the inactivation of several direct transcriptional targets of IRF8 (identified as bound and regulated by IRF8 during PbA infection) including the phagosome associated small GTPase Irgm1, the pro-inflammatory cytokines Cxcl9, and Cxcl10, the Cd11b receptor Icam1 and the transcriptional activator and IRF8 dimerization partner Irf1 have been shown to cause resistance to CM in corresponding deletion mouse mutants (Figure 7). Finally, the inactivation of additional IRF8 targets, detected herein by ChlP-Seq, has been shown to protect against *P. berghei* induced CM, including Ifng, Jak3, Cd8, Cd14, Cd40, Hc, Fcgr2, Lta and Ltbr8. Together, these results highlight the role of IRF8 in regulating pro-inflammatory pathways in myeloid cells during CM-associated neuroinflammation.

Although IRF8-dependent activation of pro-inflammatory pathways in myeloid cells has detrimental and pathological consequences in PbA infection, it clearly plays a protective role in other types of infections including pulmonary tuberculosis. Indeed, using the same analysis and very stringent statistical parameters, a strong overlap was noted between the list of genes up-regulated in brains of B6 mice in response to PbA infection and up-regulated in lungs 30 days following aerosol infection with Mycobacterium tuberculosis (Table 3). Of the 123 genes up-regulated in *P. berghei-*infected brains, more than half (n = 66) were also up-regulated in *M. tuberculosis-*infected lungs, and nearly 3/4 (90/123) of the *P. berghei* regulated genes harbored an IRF8 binding site. Amongst the 66 genes up-regulated during both *M. tuberculosis* infection and during CM, a striking 80% (n=53) display one or more IRF8 binding sites. Furthermore, inactivating mutations in several of these genes including Irf8, Irf1,and Irgm1 cause susceptibility to pulmonary TB, while conveying some degree of protection against CM. Mutations in Tap1 and B2m also cause susceptibility to TB, while their effects on CM susceptibility have yet to be tested. It is proposed that this set of 53 genes represents the core Irf8-dependent pro-inflammatory response pathways that play key roles in protection against TB, and pathological inflammation associated with CM.

Overall, the *P. berghei* infection model indicate that Irf8 and IRF8-regulated targets play a major role in pathological inflammation. Inactivating mutations or absence of Irf8 (or certain transcriptional targets) leads to complete protection against CM, and reduced Irf8 expression causes partial protection (in Irf8^{R294C/+} F1 heterozygotes). Such gene-dosage dependent effects also raise the possibility that even small changes in expression or activity of IRF8 may have phenotypic consequences, with increased Irf8 expression possibly associated with enhanced and/or chronic inflammation.

In agreement with this hypothesis, results from recent genome wide association studies (GWAS) have pointed to IRF8 as one of the genetic factors implicated in the complex genetic etiology of several human autoimmune and chronic inflammatory conditions. For example, a SNP near IRF8 was found associated with systemic lupus erythematosus, a disease where production of type I interferon is central to pathogenesis. In addition, SNPs in IRF8 are found within a risk haplotype detected using GWA studies and meta-analysis for inflammatory bowel disease (IBD) patients63, and in linkage disequilibrium mapping in certain groups of Crohn's disease patients (rs16940202). Finally, IRF8 is key risk factor in multiple sclerosis (MS), and its association with this disease has been validated in multiple GWA studies and meta-analyses. In MS, disease risk is associated with an expression SNP (rs17445836) which maps 61 kb downstream IRF8 and regulates gene expression with higher mRNA levels associated with disease. These results not only support a role for in human chronic inflammatory conditions but further suggest that, in agreement with our results in mice, even modest changes in expression or activity of Irf8 in the context of persistent microbial or autoimmune stimulus, may lead to chronic or pathological inflammation. In agreement with this proposal, it is noted that several targets regulated during neuroinflammation in PbA-infected mice have also been detected as genetic risk factors in GWA studies of human chronic inflammatory conditions, including the MHC (type 1 diabetes, rheumatoid arthritis, lupus, MS, psoriasis), CCL7 (in IBD), IRF1 (IBD), IRF7 (Lupus) and ICAM1 (IBD) (Table 2). This highlights the role of regulated pathways in pathological inflammation in humans.

The mouse model of acute neuroinflammation induced by *P. berghei* infection has proven valuable to identify novel genes, proteins and pathways involved in pathological inflammatory conditions. This model can help prioritize genes identified in human GWA studies for therapeutic development, including assessing activity of novel antiinflammatory drug candidates for use in common human inflammatory conditions.

### Reference

Bongfen SE, Rodrigue-Gervais IG, Berghout J, Torre S, Cingolani P, Wiltshire SA, Leiva-Torres GA, Letourneau L, Sladek R, Blanchette M, Lathrop M, Behr MA, Gruenheid S, Vidal SM, Saleh M, Gros P. An N-ethyl-N-nitrosourea (ENU)-induced dominant negative mutation in the JAK3 kinase protects against cerebral malaria. PLoS One. 2012;7(2):e31012. Epub 2012 Feb 21.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. An in vitro method for assessing the ability of an agent to prevent, treat and/or alleviate the symptoms associated with an inflammatory condition in an individual, said method comprising :
(a) combining in vitro the agent with a reagent to obtain a parameter associated with at least one biological activity of USP15, wherein the at least one biological activity is a de-ubiquinating activity, wherein the reagent is
• a nucleic acid molecule, wherein the nucleic acid molecule (i) comprises a promoter of a gene encoding USP15 and the parameter is a measure of the expression driven by the promoter, (ii) is a transcript encoding USP15 and the parameter is a measure of the amount of the transcript or (iii) is a transcript of at least one gene whose expression can be modified by USP15 and the parameter is the amount of the transcript;
• a USP15 polypeptide; or
• a binding partner of the USP15 polypeptide that can be de-ubiquinated by the USP 15 polypeptide;
(b) measuring in vitro the parameter of step (a) to obtain a test level;
(c) comparing the test level to a control level, wherein the control level is associated with the at least one biological activity of USP15 in the presence of a control agent known not to limit inflammation or prevent, treat, alleviate the symptoms of inflammation; and
(d) characterizing the agent as (i) useful for the prevention, treatment and/or alleviation of the symptoms associated with the inflammatory condition when the at least one biological activity associated with the test level is lower than the at least one biological activity associated with the control level or (ii) lacking utility for the prevention, treatment and/or alleviation of the symptoms associated with the inflammatory condition when the at least one biological activity associated with the test level is equal to or higher than the at least one biological activity associated with the control level.

2. The method of claim 1, wherein the parameter is the amount of USP15.

3. The method of claim 1, wherein the parameter is the amount of ubiquitinated or de-ubiquitinated binding partner of USP15.

## Patentansprüche

1. In-Vitro-Verfahren
zur Bewertung der Fähigkeit eines Agens, mit einem Entzündungszustand einer Person verbundene Symptome zu verhindern, zu behandeln und/oder zu lindern, wobei das Verfahren umfasst:
a) In-Vitro-Kombination des Agens mit einem Reagens, um einen zumindest einer biologischen Aktivität von USP15 entsprechenden Parameter zu erhalten, wobei die zumindest eine biologische Aktivität eine de-ubiquitinierende Aktivität ist, wobei das Reagens
- ein Nukleinsäuremolekül ist, wobei das Nukleinsäuremolekül
(i) einen Promoter eines USP15 kodierenden Gens aufweist und der Parameter ein Maß für die durch den Promoter gesteuerte Expression darstellt,
(ii) ein USP 15 kodierendes Transkript ist und der Parameter ein Maß für die Menge des Transkripts darstellt, oder
(iii) ein Transkript zumindest eines Gens ist, dessen Expression durch USP15 modifizierbar ist, und der Parameter die Menge des Transkripts darstellt;
- ein USP15-Polypeptid ist; oder
- ein Bindungspartner des USP15-Polypeptids ist, welcher durch das USP15-Polypeptid de-ubiquitinierbar ist;
b) In-Vitro-Messung des Parameters aus Schritt (a), um ein Testniveau zu erhalten;
c) Vergleich des Testniveaus mit einem Referenzniveau, wobei das Referenzniveau der zumindest einen biologischen Aktivität von USP15 bei Vorhandensein eines Referenzagens, von welchem bekannt ist, dass es weder entzündungshemmend wirkt noch Entzündungssymptome verhindert, behandelt oder lindert, entspricht; und
d) Kennzeichnung des Agens als
(i) nützlich zur Verhinderung, Behandlung und/oder Linderung der mit dem Entzündungszustand verbundenen Symptome, wenn die zumindest eine dem Testniveau entsprechende biologische Aktivität geringer ist als die zumindest eine dem Referenzniveau entsprechende biologische Aktivität, oder
(ii) ohne Nutzen zur Verhinderung, Behandlung oder Linderung der mit dem Entzündungszustand verbundenen Symptome, wenn die zumindest eine dem Testniveau entsprechende biologische Aktivität gleich groß ist wie oder größer ist als die zumindest eine dem Referenzniveau entsprechende biologische Aktivität.

2. Das Verfahren nach Anspruch 1,
wobei der Parameter die Menge an USP15 ist.

3. Das Verfahren nach Anspruch 1,
wobei der Parameter die Menge des ubiquitinierten oder de-ubiquitinierten Bindungspartners von USP15 ist.

## Revendications

1. Un procédé in vitro
pour évaluer la capabilité d'un agent d'empêcher, traiter et/ou réduire les symptômes associés avec une condition inflammatoire d'un individu, ledit procédé comprenant :
a) combiner in vitro l'agent avec un réactif pour obtenir un paramètre associé avec au moins une activité biologique d'USP15, où l'au moins une activité biologique est une activité désubiquitinante, où le réactif est
- une molécule d'acide nucléique, la molécule d'acide nucléique
(i) comprenant un promoteur d'un gène encodant USP15 et le paramètre étant une mesure de l'expression commandée par le promoteur,
(ii) étant une transcription encodant USP15 et le paramètre étant une mesure de la quantité de la transcription, ou
(iii) étant une transcription d'au moins un gène, dont l'expression est modifiable par USP15, et le paramètre étant la quantité de la transcription ;
- un polypeptide USP15 ; ou
- un partenaire de liaison du polypeptide USP15, qui peut être désubiquitiné par le polypeptide USP15 ;
b) mesurer in vitro le paramètre de l'étape (a) pour obtenir un niveau de test ;
c) comparer le niveau de test à un niveau de référence, le niveau de référence étant associé à l'au moins une activité biologique de l'USP15 en la présence d'un agent de référence connu pour ne pas limiter une inflammation ou pour empêcher, traiter, réduire les symptômes d'inflammation ; et
d) caractériser l'agent d'être
(i) utile pour l'empêchement, le traitement et/ou la réduction des symptômes associés avec la condition inflammatoire si l'au moins une activité biologique associée avec le niveau de test est inférieure à l'au moins une activité biologique associée avec le niveau de référence, ou
(ii) n'ayant pas d'utilité pour l'empêchement, le traitement et/ou la réduction des symptômes associés avec la condition inflammatoire si l'au moins une activité biologique associée avec le niveau de test et égale ou supérieure à l'au moins une activité biologique associée avec le niveau de référence.

2. Le procédé selon la revendication 1,
où le paramètre est la quantité d'USP15.

3. Le procédé selon la revendication 1,
où le paramètre est la quantité du partenaire de liaison d'USP15 ubiquitiné ou désubiquitiné.


### SEQUENCE LISTING

<110> THE ROYAL INSTITUTION FOR THE ADVANCEMENT OF LEARNING / MCGILL UNIVERSITY
<120> INFLAMMATION-ENABLING POLYPEPTIDES AND USES THEREOF
<130> 05001770-480PCT
<150> US 61/652,271
   <151> 2012-05-28
<160> 10
<170> PatentIn version 3.5
<210> 1
   <211> 10
   <212> DNA
   <213> Artificial Sequence
<220>
   <223> ISRE canonical motif
<220>
   <221> misc_feature
   <222> (5)..(6)
   <223> n is a, c, g, or t
<400> 1
   gaaanngaaa 10
<210> 2
   <211> 10
   <212> DNA
   <213> Artificial Sequence
<220>
   <223> EICE-type canonical motif
<220>
   <221> misc_feature
   <222> (5)..(6)
   <223> n is a, c, g, or t
<400> 2
   ggaanngaaa 10
<210> 3
   <211> 20
   <212> DNA
   <213> Artificial Sequence
<220>
   <223> CpG DNA oligonucleotide
<400> 3
   tccatgacgt tcctgacgtt 20
<210> 4
   <211> 1284
   <212> PRT
   <213> Mus musculus
<400> 4
   Met Glu Gly Ala Lys Gly Pro Arg Leu Arg Gly Phe Leu Ser Gly Ser
   1               5                   10                  15
   Leu Ala Thr Trp Ala Leu Gly Leu Ala Gly Leu Val Gly Glu Ala Glu
               20                  25                  30
   Glu Ser Ala Gly Gly Thr Glu Glu Glu Glu Glu Glu Glu Glu Glu Glu
           35                  40                  45
   Gly Ala Leu Cys Thr Glu Lys Arg Phe Leu Arg Leu Ile Asp Gly Ala
       50                  55                  60
   Leu Leu Leu Arg Val Leu Gly Ile Ile Ala Pro Ser Ser Arg Gly Gly
   65                  70                  75                  80
   Leu Arg Met Val Arg Gly His Asp Gly Pro Ala Ala Cys Arg Met Trp
                   85                  90                  95
   Asn Leu Cys His Leu Trp Gly Arg Leu Arg Asp Phe Tyr Gln Glu Glu
               100                 105                 110
   Leu Gln Leu Leu Ile Leu Ser Pro Pro Pro Asp Leu Gln Thr Met Gly
           115                 120                 125
   Cys Asp Pro Phe Ser Glu Glu Ala Val Asp Glu Leu Glu Ser Ile Leu
       130                 135                 140
   Arg Leu Leu Leu Gly Ala Ser Val Gln Cys Glu His Arg Glu Leu Phe
   145                 150                 155                 160
   Ile Arg His Ile Arg Gly Leu Ser Leu Asp Val Gln Ser Glu Leu Ala
                   165                 170                 175
   Gly Ala Ile Gln Glu Val Thr Gln Pro Gly Ala Gly Val Val Leu Ala
               180                 185                 190
   Leu Ala Gly Pro Glu Ser Gly Glu Leu Val Ala Glu Glu Leu Glu Met
           195                 200                 205
   Gln Leu Arg Ser Leu Thr Gly Met Met Ser Arg Leu Ala Arg Glu Arg
       210                 215                 220
   Asp Leu Gly Ala Gln Arg Leu Ala Glu Leu Leu Leu Glu Arg Glu Pro
   225 230 235 240
   Ala His Leu Leu Leu Pro Glu Ala Pro Ala Asn Ala Ser Ala Glu Gly
   245 250 255
   Val Ser His His Leu Ala Leu Gln Leu Thr Asn Ala Lys Ala Gln Leu
   260 265 270
   Arg Arg Leu Arg Gln Glu Val Glu Glu Lys Ala Glu Gln Leu Leu Asp
   275 280 285
   Ser Gln Ala Glu Val Gln Gly Leu Glu Ala Glu Ile Arg Arg Leu Arg
   290 295 300
   Gln Glu Thr Gln Ala Leu Ser Ala Gln Ala Lys Arg Ala Glu Leu Tyr
   305 310 315 320
   Arg Glu Glu Ala Glu Ala Leu Arg Glu Arg Ala Gly Arg Leu Pro Arg
   325 330 335
   Leu Gln Glu Glu Leu Arg Arg Cys Arg Glu Lys Leu Gln Ala Ala Glu
   340 345 350
   Val Phe Lys Gly Gln Leu Glu Glu Glu Arg Val Leu Ser Glu Ala Leu
   355 360 365
   Glu Ala Ser Lys Val Leu Leu Glu Glu Gln Leu Glu Val Ala Arg Glu
   370 375 380
   Arg Ser Ala Arg Leu His Glu Thr Gln Arg Glu Asn Leu Leu Leu Arg
   385 390 395 400
   Thr Arg Leu Gly Glu Ala His Ala Asp Leu Asp Ser Leu Arg His Gln
   405 410 415
   Leu Glu Gln Leu Val Glu Glu Asn Val Glu Leu Glu Leu Glu Leu Gln
   420 425 430
   Arg Ser Leu Glu Pro Pro Pro Gly Ser Pro Gly Glu Ala Ser Leu Pro
   435 440 445
   Gly Ala Ala Pro Ser Leu Gln Asp Glu Val Arg Glu Ala Glu Ala Gly
   450 455 460
   Arg Leu Arg Ala Val Glu Arg Glu Asn Arg Glu Leu Arg Gly Gln Leu
   465 470 475 480
   Gln Met Leu Gln Ala Gln Leu Gly Ser Gln His Pro Leu Leu Glu Glu
   485 490 495
   Gln Arg Glu Asn Ser Arg Gln Pro Pro Val Pro Asn Arg Asp Pro Ala
   500 505 510
   Thr Pro Ser Ala Leu His His Ser Pro Gln Ser Pro Ala Cys Gln Ile
   515 520 525
   Gly Gly Glu Gly Ser Glu Ser Leu Asp Leu Pro Ser Pro Ala Ser Tyr
   530 535 540
   Ser Asp Ile Thr Arg Ser Pro Lys Cys Ser Gln Ala Pro Asp Ser His
   545 550 555 560
   Pro Glu Leu Glu Ser Pro Leu Gln Met Val Ser Gln Asp Pro Gln Thr
   565 570 575
   Ser Asp Gln Ala Leu Gln Glu Ser Asp Pro Thr Val Glu Thr His Gln
   580 585 590
   Cys Leu Glu Lys Ser Gly His Arg Val Pro Leu Gln Ser Pro Ile Val
   595 600 605
   Trp Asp Pro Pro Gln Gly Pro Glu Val Arg Ile Glu Val Gln Glu Leu
   610 615 620
   Leu Gly Glu Thr Gly Ser Arg Glu Ala Pro Gln Gly Glu Leu Val His
   625 630 635 640
   Lys Ala Gln Val Leu Lys Gln Glu Ser Pro Lys Cys Arg Pro Arg Ser
   645 650 655
   Ala Glu Leu Thr Leu Arg Glu Pro Leu Lys Asp Gln Lys Ala Leu Asp
   660 665 670
   Arg Glu Leu Glu Leu Ser Lys Gln Gln Lys Glu Thr Gly Arg His Glu
   675 680 685
   Gln Arg Pro Lys Gly Leu Glu Ser Lys Leu Gly Pro Gln Lys Pro Gln
   690 695 700
   Gln Thr Ser Glu Gly Val Pro Asp Ala Trp Ser Arg Glu Glu Pro Thr
   705 710 715 720
   Pro Gly Glu Thr Leu Val Ser Ala Ile Pro Glu Glu Gln Ala Leu Arg
   725 730 735
   Asp Glu Val Ala Gln Leu Arg Arg Glu Val Ala Gly Leu Glu Val Lys
   740 745 750
   Leu Gln Ala Gln Ala Gln Arg Leu Glu Ala Arg Ser Ala Glu Ala Leu
   755 760 765
   Cys Leu Ser Glu Glu Leu Ala Gln Ala Arg Arg Thr Glu Ala Glu Ala
   770 775 780
   His Gln Glu Ala Glu Ala Gln Ala Arg Glu Gln Ala Arg Leu Arg Glu
   785 790 795 800
   Ala Val Asp Thr Ala Ser Leu Glu Leu Glu Ala Ala Ser Arg Glu Arg
   805 810 815
   Glu Ala Leu Ala Glu Ala Leu Ala Ala Ala Gly Arg Glu Arg Arg Gln
   820 825 830
   Trp Glu Arg Asp Gly Pro Arg Leu Arg Ala Gln Val Glu Ala Ala Glu
   835 840 845
   Gln Gln Val Gln Ala Leu Glu Ser Gln Val Arg Cys His Leu Glu Glu
   850 855 860
   Ala Glu Arg Glu His Ala Glu Lys Gln Ala Leu Arg Glu Glu Leu Glu
   865 870 875 880
   Lys Ala Val Leu Arg Gly Gln Glu Leu Gly Asp Arg Leu Glu His Leu
   885 890 895
   Gln Glu Glu Leu Glu Gln Ala Ala Leu Glu Arg Gln Lys Phe Leu Gln
   900 905 910
   Glu Gln Glu Asn Gln His Gln Arg Tyr Arg His Leu Glu Gln Arg Leu
   915 920 925
   Glu Ala Glu Leu Gln Ala Ala Ser Thr Ser Lys Glu Glu Ala Leu Met
   930 935 940
   Glu Leu Lys Ala Arg Ala Leu Gln Leu Glu Glu Glu Leu Ile Gln Leu
   945 950 955 960
   Arg Gln Tyr Pro Val Asp Leu Ala Thr Gly Ala Arg Ala Gly Pro Arg
   965 970 975
   Thr Val Glu Thr Gln Asn Gly Arg Leu Ile Glu Val Glu Arg Asn Asn
   980 985 990
   Ala Thr Leu Val Ala Glu Lys Ala Ala Leu Gln Gly Gln Leu Gln His
   995 1000 1005
   Leu Glu Gly Gln Leu Gly Ser Leu Gln Gly Arg Ala Gln Glu Leu
   1010 1015 1020
   Leu Leu Gln Ser Gln Arg Ala Gln Glu His Ser Ser Arg Leu Gln
   1025 1030 1035
   Ala Glu Lys Ser Met Met Glu Met Gln Gly Gln Glu Leu His Arg
   1040 1045 1050
   Lys Leu Gly Val Leu Glu Glu Glu Val Arg Ala Ala Arg Arg Ala
   1055 1060 1065
   Gln Glu Glu Thr Arg Gly Gln Gln Gln Ala Leu Leu Arg Asp His
   1070 1075 1080
   Glu Ala Leu Val Gln Leu Gln Arg Arg Gln Glu Thr Glu Leu Glu
   1085 1090 1095
   Gly Leu Leu Val Arg His Arg Asp Leu Lys Ala Asn Met Arg Ala
   1100 1105 1110
   Leu Glu Leu Ala His Arg Glu Leu Gln Gly Arg His Glu Gln Leu
   1115 1120 1125
   Gln Ala Gln Arg Ala Asn Val Glu Ala Gln Glu Val Ala Leu Leu
   1130 1135 1140
   Ala Glu Arg Glu Arg Leu Met Gln Asp Gly His Arg Gln Arg Gly
   1145 1150 1155
   Leu Glu Glu Glu Leu Arg Arg Leu Gln Asn Glu His Glu Arg Ala
   1160 1165 1170
   Gln Met Leu Leu Ala Glu Val Ser Arg Glu Arg Gly Glu Leu Gln
   1175 1180 1185
   Gly Glu Arg Gly Glu Leu Arg Ser Arg Leu Ala Arg Leu Glu Leu
   1190 1195 1200
   Glu Arg Ala Gln Leu Glu Ile Gln Ser Gln Gln Leu Arg Glu Ser
   1205 1210 1215
   Asn Gln  Gln Leu Asp Leu Ser  Ala Cys Arg Leu Thr  Thr Gln Ser
       1220                 1225                 1230
   Ser Asp  Pro Ala Ser Gln Arg  Ala Gly Gly Gly Glu  Gln Ala Ala
       1235                 1240                 1245
   Val Ser  Arg Gly Pro Gly Pro  Glu Pro Gly Glu Gln  Gly Ala Ala
       1250                 1255                 1260
   Gly Ala  Gln Pro Gly Glu Pro  Arg Pro Ser Ala Ser  Arg Ala Ala
       1265                 1270                 1275
   Arg Val  Pro Gly Pro Ala
       1280
<210> 5
   <211> 53
   <212> DNA
   <213> Artificial Sequence
<220>
   <223> Sense oligonucloetide for ISH
<400> 5
   gcgctataat acgactcact atagggagat ccgaatcttt ggacctgcct tct 53
<210> 6
   <211> 53
   <212> DNA
   <213> Artificial Sequence
<220>
   <223> Antisense oligonucleotide for ISH
<400> 6
   gcattaattt aggtgacact atagaagcga agctagccgt atccactgct tca 53
<210> 7
   <211> 21
   <212> DNA
   <213> Artificial Sequence
<220>
   <223> Oligonucleotide
<400> 7
   gatctggggg cacagcggtt g 21
<210> 8
   <211> 21
   <212> DNA
   <213> Artificial Sequence
<220>
   <223> Oligonucleotide
<400> 8
   gcgtctcagc tgggccttgg c 21
<210> 9
   <211> 21
   <212> DNA
   <213> Artificial Sequence
<220>
   <223> Oligonucleotide
<400> 9
   tccagcaggt cagcaaagaa c 21
<210> 10
   <211> 22
   <212> DNA
   <213> Artificial Sequence
<220>
   <223> Oligonucleotide
<400> 10
   ggactgatta tggacaggac tg 22